# EUROPEAN PATENT APPLICATION

(11) **EP 4 494 643 A1**
(43) Date of publication of application: **22.01.2025**
(21) Application number: 23769821.2
(22) Date of filing: 15.03.2023
(51) Int. Cl.: A61K 31/675, A61K 31/664, A61K 31/06, A61K 31/04, A61K 31/495, A61K 31/4747, A61P 35/00

(54) **METHOD FOR TREATING PATIENT WITH BRCA-MUTATED CANCER**

(30) Priority: 15.03.2022 CN 202210253284
(71) Applicant: Ascentawits Pharmaceuticals, Ltd., Shenzhen, Guangdong 518118 (CN)
(72) Inventor: DUAN, Jianxin, Shenzhen, Guangdong 518118 (CN); MENG, Fanying, San Francisco California 94121 (US); QI, Tianyang, Shenzhen, Guangdong 518118 (CN)
(74) Representative: Studio Torta S.p.A.
(86) International application number: PCT/CN2023/081542
(87) International publication number: WO 2023/174319

(57) **Abstract**

A method for treating BRCA gene-mutated cancer. In the method, a drug containing hypoxia-activated prodrug compounds of formulas (1), (2) and (3) or AKR1C3 enzyme-activated prodrug compounds of formulas (4), (5), (6), (7), (8), (9), (10), (11) and (12) and salts, esters, solvates and isotopic isomers thereof, is used alone or in combination with other drugs to treat patients with BRCA gene-mutated cancer and tumors.

## Description

### TECHNICAL FIELD

The present invention relates to a treatment method of cancer, and in particular to a treatment method of cancer with BRCA pathogenic mutations.

### BACKGROUND

AST-3424 is an AKR1C3 enzyme-activated DNA alkylating agent prodrug which undergoing phase II clinical trials (China registration for clinical are CTR20201915, CTR20201908, CTR20191399, CTR20191371, the United States registration for clinical are NCT04315324, NCT03592264) in both China and the United States, which are specifically activated by AKR1C3 enzyme highly expressed in a variety of tumors to release DNA alkylating agent, thereby exerting anti-tumor effects.

Preclinical studies show that the efficacy of AST-3424 is highly correlated with the expression of AKR1C3 enzyme, and small differences in the enzyme expression will greatly affect the final therapeutic efficacy of the drug (AACR 2016, Abstract# 1369: In vitro and in vivo antitumor activity of TH3424: Preclinical rationale for a highly selective AKR1C3 prodrug for treating hepatocellular carcinomas; AACR-NCI-EORTC Annual Meeting, 2017, Abstract: LB-B16: The AKR1C3-Activated Prodrug OBI-3424 Exerts Profound In Vivo Efficacy Against Preclinical Models of T-Cell Acute Lymphoblastic Leukemia (T-ALL); a Pediatric Preclinical Testing Consortium Study; Clin Cancer Res 2019;25:4493-503:OBI-3424, a Novel AKR1C3-Activated Prodrug, Exhibits Potent Efficacy against Preclinical Models of T-ALL; Am J Cancer Res 2021;11(7):3645-3659, A novel selective AKR1C3-activated prodrug AST-3424/OBI-3424 exhibits broad anti-tumor activity).

The above Phase II clinical trials in China and the United States will investigate the relationship between AKR1C3 expression levels and therapeutic efficacy in cancer patients, and determine quantitative values of AKR1C3 expression levels for screening patients who would benefit from the experimental drug.

TH-302 (Evofosfamide, Cas No. 918633-87-1) is an hypoxia-activated DNA alkylating agent prodrug, which is activated in the hypoxia or anoxic environment of tumors (such as far away blood vessels) to release DNA alkylating agents, thereby exerting anti-tumor effects.

### SUMMARY OF THE INVENTION

In the further preclinical efficacy studies, the applicant have found that the AKR1C3 enzyme-activated DNA alkylating agent prodrug compound with similar structure of AST-3424 and the hypoxia-activated DNA alkylating agent prodrug compound with similar structure of TH-302 have excellent therapeutic efficacy on BRCA-mutated tumors, that is, relatively speaking, above AKR1C3 enzyme-activated DNA alkylating agent prodrug compounds with similar structure of AST-3424 and hypoxia-activated DNA alkylating agent prodrug compound with similar structure of TH-302 have better therapeutic efficacy in tumor models carrying BRCA pathogenic mutations than in wild-type (not having BRCA mutations or having non-pathogenic BRCA mutations) tumor models, and the difference is very significant.

Based on the experimental results, the present application provides the following treatment method of cancer.

A method for treating BRCA gene-mutated cancer, which uses a drug monotherapy containing hypoxia-activated prodrug compounds of formulas (1), (2), (3) or AKR1C3 enzyme-activated prodrug compounds of formulas (4), (5), (6), (7), (8), (9), (10), (11), (12) and salts, esters, solvates, and isotopic isomers thereof or in combination with other drugs to treat patients with BRCA gene-mutated cancer and tumors: 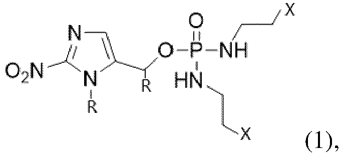 wherein R is each independently selected from H, -CH₃, -CH₂CH₃, -CF₃, X is each independently selected from Cl, Br, MsO, TsO and other leaving functional groups.

Preparations related to TH-302 or analogous compound thereof 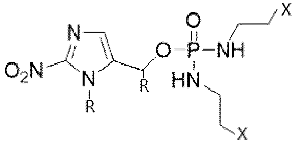 comprise orally preparation, lyophilized preparation and concentrated injection, and the relevant prescription, preparation method and clinical compatibility and administration method have been described in detail and disclosed by the relevant patents of Threshold Company: WO2010048330A1, WO2012142520A2, WO2008083101A1, which are incorporated herein by reference in its entirety.

TH-302 or analogous compound thereof 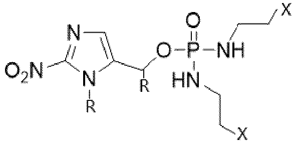 is a DNA alkylating agent class of anticancer drugs with a wide range of cancer treating potential, and these relevant cancer indications trials, clinical trials are disclosed in the relevant patent application text of Threshold Company and other pharmaceutical companies (such as WO2016011195A2, WO2004087075A1, WO2007002931A1, WO2008151253A2, WO2009018163A1, WO2009033165A2, WO2010048330A2, WO2012142520A1, WO2008083101A2, WO2020007106A1, WO2020118251A1, WO2014169035A1, WO2013116385A1, WO2019173799A2, WO2016081547A1, WO2014062856A1, WO2015069489A1, WO2012006032A2, WO2018026606A2, WO2010048330A2, WO2015171647A1, WO2013096687A1, WO2013126539A2, WO2013096684A2, WO2012009288A2, WO2012145684A2, WO2016014390A2, WO2019055786A2, WO2012135757A2, WO2015013448A2, WO2016011328A2, WO2013177633A2, WO2016011195A2, WO2015051921A2) and in clinical trials registered with FDA (NCT02402062, NCT02020226, NCT02076230, NCT01381822, NCT02093962, NCT01440088, NCT02255110, NCT02342379, NCT01864538, NCT01149915, NCT02433639, NCT00743379, NCT01485042, NCT01721941, NCT02047500, NCT00742963, NCT01497444, NCT00495144, NCT01746979, NCT01144455, NCT01403610, NCT01522872, NCT01833546, NCT02598687, NCT03098160, NCT02496832, NCT02712567), which are incorporated herein by reference in its entirety. 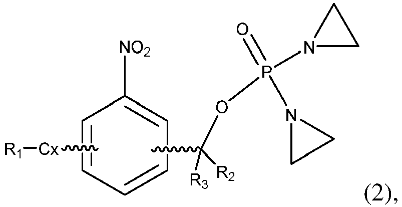 wherein the definitions of R₁, R₂, R₃, Cx are described in the claims of Patent Application PCT/CN2020/114519 with Publication No. WO2021120717A1, and the synthesis preparation method of specific compounds is also described in above application. Specifically, the groups are defined as follows:
Cx is 5-10 membered aromatic ring or aromatic heterocycle, aliphatic heterocycle or cycloalkane, which shares two carbon atoms with nitrobenzene ring to form a fused ring structure;
R₁ is attached to any skeleton atom of Cx ring and selected from hydrogen, halogen atom, cyano or isocyano, hydroxyl, sulfhydryl, amine, OTs, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15 membered heterocycle or Z-substituted heterocycle, 5-15 membered heteroaryl or Z-substituted heteroaryl, alkoxy with 1-6 carbon atoms or Z-substituted alkoxy with 1-6 carbon atoms, -CONR⁶R⁷, -SO₂NR⁶R⁷, -SO₂R⁶, -OCOO-R⁶, -COOR⁶, -NR⁶COR⁷, -OCOR⁶, -NR⁶SO₂R⁷, -NR⁶SO₂NR⁶R⁷,
R₂ and R₃ are each independently hydrogen, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15 membered heterocycle or Z-substituted heterocycle, 5-15 membered heteroaryl or Z-substituted heteroaryl, or R₂ and R₃ together with the benzyl carbon atom to which they are bonded form 3-6 membered ring; 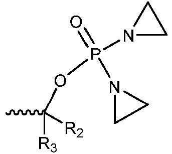 group can substitute the hydrogen atom at any position on the carbon atom of the fused ring, and the number of substitution is 1;
the substituent Z is a halogen atom, cyano or isocyano, hydroxy, sulfhydryl, amino, C₁-C₃ alkyl or substituted alkyl, C₁-C₃ alkoxy or substituted alkoxy, C₂-C₃ alkenyl or substituted alkenyl, C₂-C₃ alkynyl or substituted alkynyl, C₃-C₈ cycloalkyl or substituted cycloalkyl;
R₆ and R₇ are each independently hydrogen, C₁-C₆ alkyl or Z-substituted C₁-C₆ alkyl, C₂-C₆ alkenyl or Z-substituted C₂-C₆ alkenyl, C₂-C₆ alkynyl or Z-substituted C₂-C₆ alkynyl, C₃-C₈ cycloalkyl or Z-substituted C₃-C₈ cycloalkyl, C₆-C₁₀ aryl or Z-substituted C₆-C₁₀ aryl, 4-15 membered heterocycle or Z-substituted 4-15 membered heterocycle, 5-15 membered heteroaryl or Z-substituted 5-15 membered heteroaryl, or R⁶ and R⁷ together with the atom to which they are bonded form 5-7 membered heterocyclyl or Z-substituted 5-7 membered heterocyclyl. 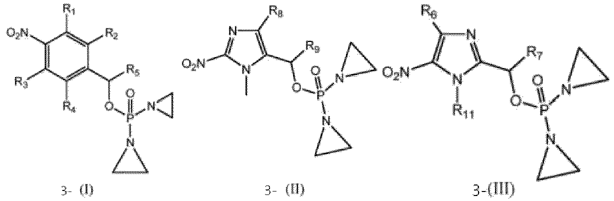 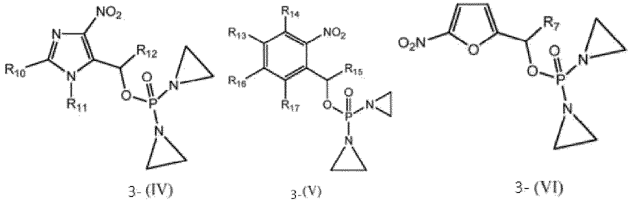
wherein the definitions of R₁, R₂, R₃, R₄, R₅, R₆, R₇, R₈, R₉, R₁₀, R₁₁, R₁₂, R₁₃, R₁₄, R₁₅, R₁₆, R₁₇ are described in the claims of Patent Application PCT/US2016/039092 with Publication No. WO2016210175A1 (corresponding to the Chinese Application No. 2016800368985 with Publication No. CN108024974A), and the synthesis preparation method of specific compounds is also described in above application. Specifically, the groups are defined as follows:
   R₁ is hydrogen, -N₃, CN, halogen, NR²¹R²², -OR²³, -SO₂ (C₁-C₆ alkyl), C₁-C₆ alkyl, C₂-C₆ alkenyl, C₂-C₆ alkynyl, C₃-C₈ cycloalkyl, C₆-C₁₀ aryl, 4-15 membered heterocycle, 5-15 membered heteroaryl or ether;
   R²¹ and R²² are each independently hydrogen, hydroxyl, C₁-C₆ alkyl, C₂-C₆ alkenyl, C₂-C₆ alkynyl, C₃-C₈ cycloalkyl, C₆-C₁₀ aryl, 4-15 membered heterocycle, 5-15 membered heteroaryl or -SO₂ (C₁-C₆ alkyl); or R²¹ and R²² together with the nitrogen atom to which they are bonded form 4-15 membered heterocycle or 5-15 membered heteroaryl;
   R²³ is hydrogen, C₁-C₆ alkyl or C₆-C₁₀ aryl; and R₃ are each independently hydrogen or halogeno;
   R₄ is hydrogen, halogeno, C₁-C₆ alkoxy, C₁-C₆ alkyl, or C₆-C₁₀ aryl;
   R₅, R₇, R₉, R₁₂ and R₁₅ are each independently hydrogen, C₁-C₆ alkyl, C₂-C₆ alkenyl, C₂-C₆ alkynyl, C₃-C₈ cycloalkyl, C₆-C₁₀ aryl, 4-15 membered heterocycle, 5-15 membered heteroaryl or R₄ and R₅ together with the intervening carbon atom between them form C₅-C₆ cycloalkyl ring;
   R₆ and R₁₀ are each independently hydrogen or halogeno;
   R₈ is hydrogen, C₁-C₆ alkyl, C₂-C₆ alkenyl, C₂-C₆ alkynyl or 5-15 membered heteroaryl;
   R₁₁ is each independently C₁-C₆ alkyl, C₂-C₆ alkenyl, C₂-C₆ alkynyl, C₃-C₈ cycloalkyl or C₆-C₁₀ aryl;
   R₁₃, R₁₄, R₁₆, and R₁₇ are independently hydrogen, halogeno, C₁-C₆ alkyl, C₂-C₆ alkenyl, C₂-C₆ alkynyl or C₁-C₆ alkoxy group;
   wherein the alkyl, alkenyl, alkynyl, cycloalkyl, aryl, heterocycle, heteroaryl, alkoxy and ether groups are optionally substituted. 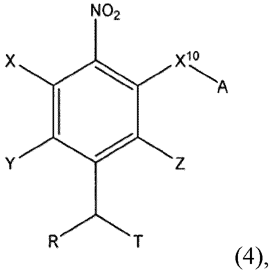
   wherein the definitions of X, Y, Z, R, T, A and X¹⁰ are described in the claims of Patent Application PCT/US2016/062114 with Publication No.WO2017087428A1 (corresponding to the Chinese Application No. 2016800200132 with Publication No. CN108136214A), and the synthesis preparation method of specific compounds is also described in above application. Specifically, the groups are defined as follows:
      X¹⁰ is O, S, SO, or SO₂;
      A is C₆-C₁₀ aryl, 5-15 membered heteroaryl or -N=CR¹R²;
      R¹ and R² are each independently hydrogen, C₁-C₆ alkyl, C₃-C₈ cycloalkyl, C₆-C₁₀ aryl, 4-15 membered heterocycle, ether, -CONR¹³R¹⁴ or -NR¹³COR¹⁴;
      X, Y and Z are independently hydrogen, CN, halogeno, C₁-C₆ alkyl, C₂-C₆ alkenyl, C₂-C₆ alkynyl, C₃-C₈ cycloalkyl, C₆-C₁₀ aryl, 4-15 membered heterocycle, ether, -CONR¹³R¹⁴ or -NR¹³COR¹⁴;
      R is hydrogen, C₁-C₆ alkyl, C₂-C₆ alkenyl, C₂-C₆ alkynyl, C₃-C₈ cycloalkyl, C₆-C₁₀ aryl, 4-15 membered heterocycle, ether, -CONR¹³R¹⁴ or -NR¹³COR¹⁴;
      R¹³ and R¹⁴ are each independently hydrogen, C₁-C₆ alkyl, C₃-C₈ cycloalkyl, C₆-C₁₀ aryl, 4-15 membered heterocycle or ether;
      T comprises a phosphoramidate alkylating agent comprising one or more Z⁵-X⁵-Y⁵ moieties bonded to an -O-P(Z¹) moiety, where Z⁵ is a heteroatom such as nitrogen, sulfur or oxygen, X⁵ is substituted or unsubstituted ethylene, Y⁵ is halogeno or another leaving group, or Z⁵-X⁵-Y⁵ together form an aziridinyl (NCH₂CH₂) moiety, and Z¹ is O or S; and
      wherein the alkyl, alkenyl, alkynyl, cycloalkyl, aryl, heterocycle, heteroaryl, ether groups are substituted or unsubstituted. 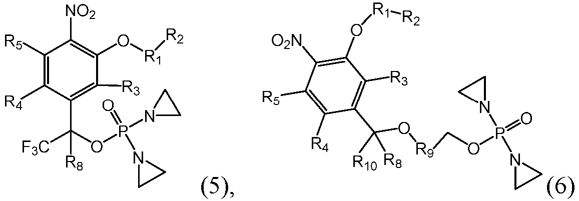
      wherein the definitions of R₁, R₂, R₃, R₄, R₅, R₈, R₉, and R₁₀ are described in the claims of Patent Application PCT/CN2020/089692 with Publication No. WO2020228685A1, and the synthesis preparation method of specific compounds is also described in above application. Specifically, the groups are defined as follows:
         R₁ is C₆-C₁₀ aryl or Z-substituted aryl, 4-15 membered heterocycle or Z-substituted heterocycle, 5-15 membered heteroaryl or Z-substituted heteroaryl, a 7-15 membered fused ring or Z-substituted fused ring;
         R₂ is hydrogen, a halogen atom, cyano or isocyano, hydroxy, sulfhydryl, amino, OTs, OMS, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15 membered heterocycle or Z-substituted heterocycle, 5-15 membered heteroaryl or Z-substituted heteroaryl, ether having from 1 to 6 carbon atoms or Z-substituted alkoxy having from 1 to 6 carbon atoms, -CONR⁶R⁷, -SO₂NR⁶R⁷, -SO₂R⁶, -OCOO-R⁶, -COOR⁶, -NR⁶COR⁷, -OCOR⁶, -NR⁶SO₂R⁷, -NR⁶SO₂NR⁶R⁷, or together with the atom in the group R¹ to which it is bonded form a 7-15 membered fused ring or Z-substituted fused ring;
         R₃ is hydrogen, halogen, cyano or isocyano, hydroxy, sulfhydryl, amino, OTs, OLCMS, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15 membered heterocycle or Z-substituted heterocycle, 5-15 membered heteroaryl or Z-substituted heteroaryl, C₁-C₆ alkoxy or Z-substituted C₁-C₆ alkoxy, -CONR⁶R⁷, -SO₂NR⁶R⁷, -SO₂R⁶, -OCO-R⁶, -OCOO-R⁶, -COOR⁶, -NR⁶COR⁷, -OCOR⁶, or -NR⁶SO₂R⁷;
         R₄ and R₅ are each independently hydrogen, a halogen atom, cyano or isocyano, hydroxy, sulfhydryl, amino, OTs, OLCMS, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15 membered heterocycle or Z-substituted heterocycle, 5-15 membered heteroaryl or Z-substituted heteroaryl, C₁-C₆ alkoxy or Z-substituted C₁-C₆ alkoxy, -CONR⁶R⁷, -SO₂NR⁶R⁷, -SO₂R⁶, -OCOO-R⁶, -COOR⁶, -NR⁶COR⁶, -OCOR⁶ or -NR⁶SO₂R⁷, or R⁴ and R⁵ together with the atom in the benzene ring to which they are bonded form a 7-15 membered fused ring or Z-substituted fused ring;
         R₆ and R₇ are each independently hydrogen, cyano or isocyano, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, 4-15 membered heterocycle or Z-substituted heterocycle, 5-15 membered heteroaryl or Z-substituted heteroaryl, C₁-C₆ alkoxy or Z-substituted C₁-C₆ alkoxy, or R⁶ and R⁷ together with the atom to which they are bonded form 5-7 membered heterocyclyl or Z-substituted 5-7 membered heterocyclyl;
         R₈ and R₁₀ are each independently hydrogen, deuterium, aryl or Z-substituted aryl, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, and at least one of R⁸ and R¹⁰ must be hydrogen or deuterium;
         R₉ is substituted C₆-C₁₀ aryl which is substituted with at least one fluorine atom or nitro group, substituted 4-15 membered heterocycle which is substituted with at least one fluorine atom or nitro group, or substituted 5-15 membered heteroaryl which is substituted with at least one fluorine atom or nitro group;
         the substituent Z is a halogen atom, cyano or isocyano, hydroxy, sulfhydryl, amino, OTs, OMS, C₁-C₃ alkyl or substituted alkyl, C₁-C₃ alkoxy or substituted alkoxy, C₂-C₃ alkenyl or substituted alkenyl, C₂-C₃ alkynyl or substituted alkynyl, C₃-C₈ cycloalkyl or substituted cycloalkyl, an aromatic ring, heterocycle, a heteroaromatic ring and fused ring or a substituted aromatic ring, heterocycle, a heteroaromatic ring and fused ring, the pattern of substitution being mono- or di-substitution;
         the substitution in the substituted C₆-C₁₀ aryl, substituted 4-15 membered heterocycle or substituted 5-15 membered heteroaryl in R₉ is a halogen atom, nitro, cyano or isocyano, hydroxy, amino, C₁-C₃ alkyl or alkoxy, alkenyl, alkynyl, cycloalkyl or benzene ring, substituted benzene ring, C₁-C₃ alkoxy or halogen atom-substituted alkoxy. 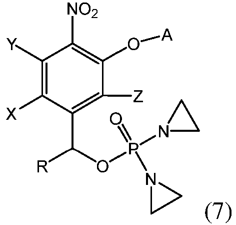
         wherein:
            A is substituted or unsubstituted C₆-C₁₀ aryl, biaryl or substituted biaryl, 5-15 membered heteroaryl, or -N=CR¹R²; wherein the substituents are selected from the group consisting of halogeno, -CN, -NO₂, -O-(CH₂)-O-, -CO₂H and salt thereof, -OR¹⁰⁰, -CO₂R¹⁰⁰, -CONR¹⁰¹R¹⁰², -NR¹⁰¹R¹⁰², -NR¹⁰⁰SO₂R¹⁰⁰, -SO₂R¹⁰⁰, -SO₂NR¹⁰¹R¹⁰², C₁-C₆ alkyl, and C₃-C₁₀ heterocyclyl;
            wherein R¹⁰⁰, R¹⁰¹ and R¹⁰² are each independently hydrogen, C₁-C₈ alkyl, or C₆-C₁₂ aryl; or R¹⁰¹ and R¹⁰² together with the nitrogen atom to which they are attached form a 5-7 membered heterocycle;
            wherein the alkyl group and the aryl group are each substituted by 1-3 halogeno groups or 1-3 C₁-C₆ alkyl groups;
            R¹ and R² are each independently phenyl or methyl;
            X, Y and Z are each independently hydrogen or halogeno; and
            R is hydrogen or C₁-C₆ alkyl or halogen-substituted alkyl. 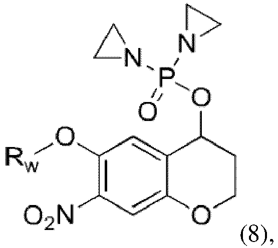
            wherein the definition of Rw is described in the claims of Patent Application PCT/CN2020/120281 with Publication No. WO2021068952A1, and the synthesis preparation method of specific compounds is also described in above application. Specifically, the groups are defined as follows:
            Rw is 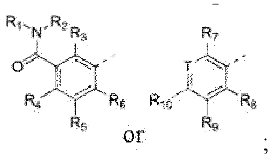
            R₁ is H, C₁₋₆ alkyl, C₃₋₆ cycloalkyl, 4-6 membered heterocycloalkyl, 5-6 membered heteroaryl or phenyl, wherein the C₁₋₆ alkyl, C₃₋₆ cycloalkyl, 4-6 membered heterocycloalkyl, 5-6 membered heteroaryl and phenyl are optionally substituted with 1, 2 or 3 R^{a};
            each R^{a} is independently H, F, Cl, Br, I, -CN, -OH, C₁₋₃ alkoxy or C₁₋₃ alkyl;
            R₂ is H or C₁₋₆ alkyl;
            or R₁ and R₂, together with the N atom to which they are attached form a 4-6 membered heterocycloalkyl, wherein the 4-6 membered heterocycloalkyl is optionally substituted with 1, 2 or 3 R^{b};
            each R^{b} is independently H, F, Cl, Br, I, -CN, -OH, -NH₂, -OCH₃, -OCH₂CH₃, -CH₃ or -CH₂CH₃;
            R₃ is H, F, Cl, Br, I, -OH, -NH₂, C₁₋₃ alkoxy or C₁₋₃ alkyl;
            or and R₃ are attached together to make the structural unit 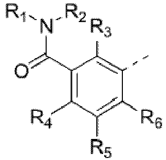 to be 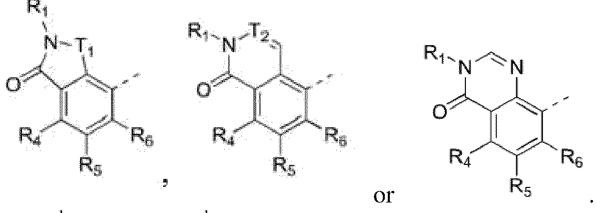
            T₁ is -(CR^{c}R^{d})ₘ- or -(CR^{c}R^{d})ₙ-O-;
            m is 1, 2 or 3;
            n is 1 or 2;
            T₂ is N or CH;
            R^{c} and R^{d} are each independently H, F, C₁₋₃ alkyl or C₁₋₃ alkoxy;
            R₄, R₅ and R₆ are each independently H, F, Cl, Br, I, C₁₋₃ alkyl or C₁₋₃ alkoxy;
            T is N or CH;
            R₇ and R₈ are each independently H, F, Cl, Br or I;
            R₉ and R₁₀ are each independently H, F, Cl, Br, I, -CN or
            the 4-6 membered heterocycloalkyl and 5-6 membered heteroaryl each contain 1, 2, 3 or 4 heteroatoms independently selected from N, -O- and -S-. 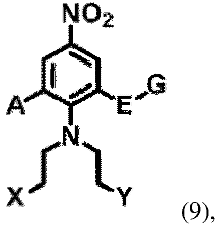
            wherein the definitions of A, E, G, X and Y are described in the claims of Patent Application PCT/NZ2019/050030 with Publication No. WO2019190331A1 (corresponding to Chinese Patent Application No. 2019800234236 with Publication No. CN111918864A), and the synthesis preparation method of specific compounds is also described in above application. Specifically, the groups are defined as follows:
               A is H, C₁-C₆ alkyl, C₁-C₆ alkenyl, C₁-C₆ alkynyl, CFH₂, CF₂H, CF₃, F, Cl, Br, I, OCF₃, COR or CON(R)₂;
               E is SO or SO₂;
               X is Cl, Br, I or OSO₂R;
               Y is Cl, Br, I or OSO₂R;
               Each R is independently H or C₁-C₆ alkyl;
               G is a radical group selected from the group consisting of Formulae (B)-(AA): 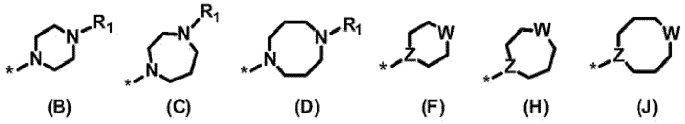 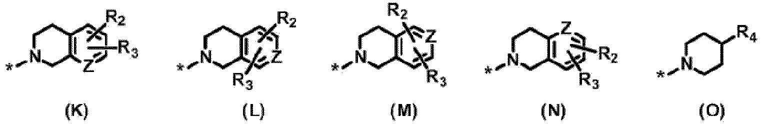 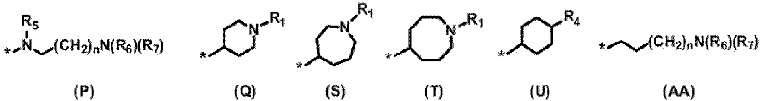
               wherein:
                  R₁ is H, C₁-C₆ alkyl, CH₂(CH₂)ₙOH, CH₂CH(OH)CH₂OH, phenyl, pyridyl, benzyl, or pyridylmethyl, provided that when R₁ is phenyl, pyridyl, benzyl or pyridylmethyl, R₁ is optionally substituted at any available position with C₁-C₆ alkyl, C₁-C₆ alkenyl, C₁-C₆ alkynyl, OR₆, N(R₆)(R₇), CFH₂, CF₂H, CF₃, F, Cl, Br, I, OCF₃, COR₆, CON(R₆)(R₇), SOR₆, SON(R₆)(R₇), SO₂R₆, SO₂N(R₆)(R₇), CN, or NO₂;
                  and R₃ are each independently H, C₁-C₆ alkyl, C₁-C₆ alkenyl, C₁-C₆ alkynyl, OR₆, N(R₆)(R₇), CFH₂, CF₂H, CF₃, F, Cl, Br, I, OCF₃, COR₆, CON(R₆)(R₇), SOR₆, SON(R₆)(R₇), SO₂R₆, SO₂N(R₆)( R₇), CN or NO₂;
                  R₄ is N(R₆)(R₇), OH, OCH₂(CH₂)ₙN(R₆)(R₇) or CH₂(CH₂)ₙN(R₆)(R₇);
                  R₅ is H or a C₁-C₆ alkyl group;
                  R₆ and R₇ are each independently H or C₁₋₆ alkyl, or R₆ and R₇ together form substituted or unsubstituted 5-membered or 6-membered heterocycle;
                  Z is CH orN;
                  W is CH₂, O, S, SO or SO₂;
                  n is 0 to 6;
                  * represents a point of attachment to Formula (I).

The drug described in the present application refers to medicine or preparation, the prepared drug comprises a specific dose range of hypoxia-activated compound of formula (1) or salt or solvate thereof as active ingredient, and/or the prepared drug is administered in specific dosage form, and specific administration method. 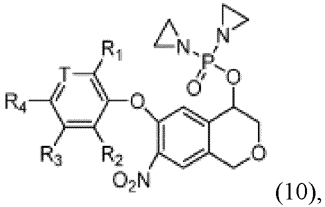 wherein the definitions of R₁, R₂, R₃, R₄ and T are described in the claims of Patent Application PCT/CN2021/118597 with Publication No. WO2022057838A1, and the synthesis preparation method of specific compounds is also described in above application. Specifically, the groups are defined as follows:
T is N or CH;
R₁ and are each independently H, F, Cl, Br, I or C₁₋₃ alkyl, wherein the C₁₋₃ alkyl is optionally substituted by 1, 2 or 3 Rₐ;
Each Rₐ is independently F, Cl, Br, I, -CN, -OH or -NH₂;
R₃ and R₄ are independently H, F, Cl, Br, I, CN, C₁₋₃ alkyl, C₁₋₃ alkoxy, 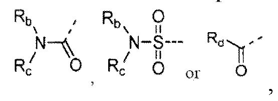 wherein the C₁₋₃ alkyl is optionally substituted by 1, 2 or 3 Rₑ; R_{b} and R_{c} are each independently H, -CH₃, -CH₂CH₃, -(CH₂)₂CH₃, -CH(CH₃)₂;
R_{d} is -CH₃, -CH₂CH₃, -(CH₂)₂CH₃, -CH(CH₃)₂;
Each Rₑ is independently F, Cl, Br, I, -CN, -OH, or -NH₂. 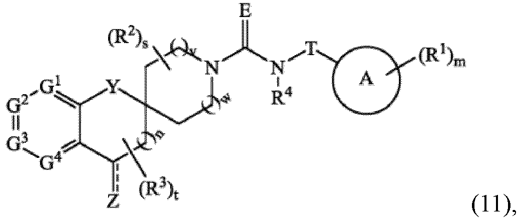
wherein the definitions of R₁, R₂, R₃, R₄, G₁, G₂, G₃, G₄, E, T, Y, Z, m, n, s, t, v, w, and ring A are described in the claims of Patent Application CN202210585771.6 with Publication No. CN115403579A, and the synthesis preparation method of specific compounds is also described in above application. Specifically, the groups are defined as follows:
   G1, G2, G3, or G4 is identical or different, and is each independently CR⁵ or N atom;
   Each R⁵ is identical or different, and is each independently selected from hydrogen, halogen, alkyl, alkenyl, alkynyl, alkoxy, hydroxyl, cyano, amino, nitro, -NR^{a}R^{b}, -C(O)NR^{a}R^{b}, cycloalkyl, heterocycle, aryl, and heteroaryl, wherein the alkyl, alkenyl, alkynyl, alkoxy, cycloalkyl, heterocycle, aryl and heteroaryl are each independently optionally substituted by one or more substituents selected from the group consisting of halogen, alkyl, haloalkyl, alkoxy, haloalkoxy, hydroxyalkyl, hydroxyl, oxy, cyano, amino, nitro, cycloalkyl, heterocycle, aryl and heteroaryl;
   Y is selected from -(C(R^{y2}R^{y3}))_{f}-NR^{y1}-, -(C(R^{y2}R^{y3}))_{g}-O-, -(C(R^{y2}R^{y3}))ₕ-S-, -(C(R^{y2}R^{y3}))ₕ-S(O)-, -(C(R^{y2}R^{y3}))ₕ-S(O)₂-, -C(R^{y2}R^{y3})-, -NR^{y1}-(C(R^{y2}R^{y3}))_{f}-, -O-(C(R^{y2}R^{y3}))_{g}-, -S-(C(R^{y2}R^{y3}))ₕ-, -S(O)-(C(R^{y2}R^{y3}))ₕ- and -S(O)₂-(C(R^{y2} R^{y3}))ₕ-;
   R^{y1} is selected from hydrogen atom, alkyl, haloalkyl, hydroxyalkyl, cycloalkyl and heterocycle;
   R^{y2} and R^{y3} are identical or different, and are independently selected from hydrogen atom, halogen, alkyl, haloalkyl, hydroxyalkyl, cycloalkyl and heterocycle;
   Or R^{y2} and R^{y3} together form =O;
   Z is O or OH;
   -̅ -̅ -̅ -̅ -̅ is single bond or double bond, Z is OH when -̅ -̅ -̅ -̅ -̅ is single bond and is O when -̅ -̅ -̅ -̅ -̅ is double bond;
   E is selected from NH, O and S atom;
   T is selected from -C(R^{T1}R^{T2})-, -NR^{T3}- or -O-;
   R^{T1} and R^{T2} are identical or different, and are each independently selected from hydrogen atom, deuterium atom, halogen, alkyl, haloalkyl, hydroxyalkyl, cycloalkyl, and heterocycle;
   Or R^{T1} and R^{T2} together with the carbon atom to which they are attached form cycloalkyl or heterocycle, wherein the cycloalkyl or heterocycle is each independently optionally substituted by one or more substituents selected from the group consisting of halogen, alkyl and hydroxyl;
   R^{T3} is selected from hydrogen, alkyl, haloalkyl, hydroxyalkyl, cycloalkyl, and heterocycle;
   Ring A is 6 to 10 membered aryl or 5 to 10 membered heteroaryl;
   Each R¹ is identical or different, and is each independently selected from hydrogen atom, deuterium atom, halogen, alkyl, alkenyl, alkynyl, alkoxy, hydroxyl, cyano, -NR^{a}R^{b}, -C(O)NR^{a}R^{b}, -S(O)NR^{a}R^{b}, -S(O)₂NR^{a}R^{b}, -S(O)R^{c}, -S(O)₂R^{c}, -B(OR^{d})₂, nitro, cycloalkyl, heterocycle, aryl and heteroaryl, wherein the alkyl, alkenyl, alkynyl, alkoxy, cycloalkyl, heterocycle, aryl and heteroaryl are each independently optionally substituted by one or more substituents selected from the group consisting of halogen, alkyl, haloalkyl, alkoxy, haloalkoxy, hydroxyalkyl, hydroxyl, oxy, cyano, -NR^{a}R^{b}, -C(O)NR^{a}R^{b}, -S(O)NR^{a}R^{b}, -S(O)₂NR^{a}R^{b}, -S(O)R^{c}, -S(O)₂R^{c}, -B(OR^{d})₂, nitro, cycloalkyl, heterocycle, aryl and heteroaryl;
   R^{a} and R^{b} are identical or different, and are each independently selected from hydrogen atom, alkyl, haloalkyl, hydroxyl, hydroxyalkyl, -C(O)R^{e}, cycloalkyl and heterocycle; or R^{a} and R^{b} together with the nitrogen atom to which they are attached form cycloalkyl or heterocycle, wherein the cycloalkyl or heterocycle is optionally substituted by one or more substituents selected from the group consisting of halogen, alkyl, haloalkyl, alkoxy, haloalkoxy, hydroxyalkyl, hydroxyl, cyano, amino, nitro, cycloalkyl, heterocycle, aryl and heteroaryl;
   R^{c} is selected from alkyl, cycloalkyl, heterocycle, aryl and heteroaryl, and the alkyl, cycloalkyl, heterocycle, aryl and heteroaryl are optionally substituted by one or more substituents selected from the group consisting of halogen, alkyl, haloalkyl, alkoxy, haloalkoxy, hydroxyalkyl, hydroxyl, cyano, amino, nitro, cycloalkyl, heterocycle, aryl and heteroaryl;
   R^{d} is hydrogen atom or C₁₋₆ alkyl;
   R^{e} is selected from alkyl, alkoxy, cycloalkyl, heterocycle, aryl and heteroaryl, and the alkyl, alkoxy, cycloalkyl, heterocycle, aryl and heteroaryl are optionally substituted by one or more substituents selected from the group consisting of halogen, alkyl, haloalkyl, alkoxy, haloalkoxy, hydroxyalkyl, hydroxyl, cyano, amino, nitro, cycloalkyl, heterocycle, aryl and heteroaryl;
   Each is identical or different, and is each independently selected from hydrogen atom, deuterium atom, halogen, alkyl, haloalkyl, alkoxy, haloalkoxy, hydroxyalkyl, oxy, hydroxyl, cyano, amino, nitro, cycloalkyl, heterocycle, aryl and heteroaryl;
   Each R³ is identical or different, and is each independently selected from hydrogen atom, deuterium atom, halogen, alkyl, haloalkyl, alkoxy, haloalkoxy, hydroxyalkyl, oxy, hydroxyl, cyano, amino, nitro, cycloalkyl, heterocycle, aryl and heteroaryl;
   R⁴ is selected from hydrogen atom, alkyl, haloalkyl, hydroxyl and hydroxyalkyl;
   n is 0, 1, 2 or 3;
   v is 0, 1 or 2;
   w is 0, 1 or 2;
   f is 0, 1 or 2;
   g is 0, 1 or 2;
   h is 0, 1 or 2;
   m is 0, 1, 2, 3, 4 or 5;
   s is 0, 1, 2, 3, 4, 5, 6, 7 or 8;
   t is 0, 1, 2, 3, 4, 5 or 6;
   provided that,
   when Y is -O- atom and E is O atom, ring A is phenyl or 5 to 6 membered heteroaryl, and G³ is CR⁵ or N atom, R⁵ is not hydrogen atom;
   when Y is -O- atom and E is S atom, ring A is phenyl or 5 to 6 membered heteroaryl;
   when G¹, G², G³, and G⁴ are all CR⁵, Y is NR^{y1}, n, v, and w are all 1, and E is O atom, 1) T is not CH₂ or CD₂, 2) at least one of or R³ is deuterium atom, 3) R⁴ is selected from alkyl, haloalkyl, hydroxyl, and hydroxyalkyl, 4) one of R¹ is 3 to 8 membered cycloalkyl or 5 to 8 membered heterocycle, and the 3 to 8 membered cycloalkyl or 5 to 8 membered heterocycle is substituted by one or more substituents selected from the group consisting of halogen, alkyl, haloalkyl, alkoxy haloalkoxy, hydroxyalkyl, hydroxyl, oxy, cyano, amino, nitro, cycloalkyl, heterocycle, aryl and heteroaryl, 5) ring A is R^{d}, which is hydrogen atom or C₁₋₆ alkyl. 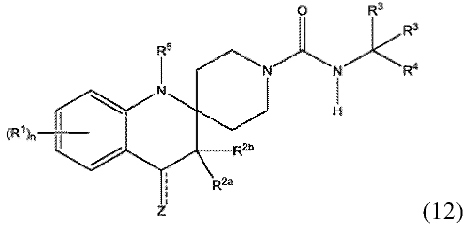 or pharmaceutically acceptable salt thereof,
   wherein the definitions of R¹, R^{2a}, R^{2b}, R³, R⁴, R⁵, n, and Z are described in the claims of Patent Application PCT/IB2020/057285 with Publication No. WO2021005586A1 (corresponding to the Chinese Application No. CN202080053804.1 with Publication No. CN114206870A), and the synthesis preparation method of specific compounds is also described in above application.

Specifically, the groups are defined as follows:
-̅ -̅ -̅ -̅ -̅ is single bond or double bond;
Z is OH when = is single bond and is O when -̅ -̅ -̅ -̅ -̅ is double bond;
Each R¹ is independently selected from the group consisting of: (C₁-C₆) alkyl, (C₁-C₆) alkoxy, (C₀-C₄) alkyl N(R⁸)₂ and halogeno;
R^{2a} and R^{2b} are each independently selected from the group consisting of: H, (C₁-C₆) alkyl and halogeno;
Each R³ is independently selected from the group consisting of: H and halogeno;
R⁴ is independently selected from the group consisting of: aryl, 5 to 6 membered heteroaryl containing 1, 2, 3, or 4 heteroatoms independently selected from N, O and S; and 9 to 10 membered fused bicyclic heteroaryl containing 1, 2, 3, or 4 heteroatoms independently selected from N, O, and S; wherein any of the foregoing is optionally substituted by one or more R⁶;
R⁵ is independently selected from the group consisting of: H, (C₁-C₆) alkyl, (C₂-C₆) alkenyl, (C₀-C₄) alkyl OR⁸, (C₁-C₄) alkyl (C₃-C₁₀) cycloalkyl, halo (C₁-C₆) alkyl, (C₂-C₃) alkynyl, (C₁-C₄) alkyl N(R¹⁰)₂;
R⁶ is independently selected from the group consisting of: halogeno, (C₁-C₆) alkyl, (C₁-C₆) alkoxy, halo (C₁-C₆) alkyl, OH, aryl, 3 to 6 membered heterocycle, 5 to 6 membered heteroaryl, (C₀-C₄) alkyl S(O)ₘ(C₁-C₆) alkyl, halo (C₁-C₆) alkoxy, (C₀-C₄) alkyl S(O)ₘN(R⁸)₂, (C₀-C₄) alkyl N(R⁸)₂, (C₀-C₄) alkyl (CO)OR⁷, N(R⁸)S(O)ₘ(C₁-C₆) alkyl, N(R⁸)S(O)ₘ(C₃-C₆) cycloalkyl, OP(O)(OH)₂, (C₀-C₃) alkyl (CO)NHR¹¹, (C₀-C₃) alkyl OR⁷, and (C₃-C₁₀) cycloalkyl; wherein each R⁶ is optionally substituted by one to three R⁹ when each R⁶ is not halogeno, OH, or OP(O)(OH)₂; or two adjacent R⁶ together with the atom to which they are attached form 5 to 7 membered heterocycle or (C₅-C₈) cycloalkyl;
Each R⁷ and R⁸ is independently selected from the group consisting of: H or (C₁-C₆) alkyl optionally substituted by one to three R⁹;
R⁹ is independently selected from the group consisting of: halogeno, -OH, amino, (C₁-C₄) alkyl amino, di(C₁-C₄) alkylamino, OP(O)(OH)₂, (C₁-C₆) alkyl, (C₁-C₃) alkynyl, (C₁-C₆) alkoxy, halo (C₁-C₆) alkyl, (C₀-C₄) alkyl S(O)ₘ(C₁-C₆) alkyl, halo (C₁-C₆) alkoxy, 3 to 6 membered heterocycle optionally substituted by oxy (=O), (C₀-C₄) alkyl S(O)ₘN(R¹⁰)₂, (C₀-C₄) alkyl (CO)R¹⁰, (C₀-C₄) alkyl (CO)OR¹⁰, (C₀-C₄) alkyl NR¹⁰S(O)ₘ(C₁-C₆) alkyl, (C₀-C₄) alkyl OR¹⁰, (C₀-C₄) alkyl N(R¹⁰)₂, (C₀-C₄) alkyl CN, (C₀-C₄) alkyl N(R¹⁰)₂, and (C₀-C₄) alkyl (CO)N(R¹⁰)₂;
Each R¹⁰ is independently selected from the group consisting of: H, (C₁-C₆) alkyl; or 3 to 6 membered heterocycle, wherein the 3 to 6 membered heterocycle is optionally substituted by one or more of the following: (C₁-C₆) alkyl; and oxy (=O);
Each R¹¹ is selected from the group consisting of: H, 4 to 6 membered heterocycle optionally substituted by one to four (C₃-C₆) cycloalkyl optionally substituted by one to four (C₀-C₃) alkyl (C₃-C₆) cycloalkyl (C₁-C₃) alkyl optionally substituted by halogeno, CH₂-aryl optionally substituted by one to three (C₁-C₆) alkyl, (C₂-C₆) alkenyl, or (C₂-C₆) alkynyl, wherein each of the (C₁-C₆) alkyl, (C₂-C₆) alkenyl and (C₂-C₆) alkynyl is optionally substituted by one or more R¹³;
Each R¹² is independently selected from the group consisting of: OH, (C₁-C₃) alkoxy, NH₂, or (C₁-C₃) alkyl optionally substituted by one or more OH;
Each R¹³ is independently selected from the group consisting of: halogeno, OH, amino, (C₁-C₄) alkylamino, di(C₁-C₄) alkylamino, (C₁-C₃) alkoxy, and C(O)-(C₃-C₈) cycloalkyl;
m is 0, 1 or 2; and
n is 0, 1 or 2.

In addition to containing DNA alkylating agent prodrug compounds of formulas (1)-(9), (10) and AKR1C3-activated KARS inhibitor prodrug compounds of formulas (11), (12), the above-mentioned drugs should also be supplemented with pharmaceutically acceptable auxiliaries or excipients based on the specific characteristics of the drug, medicament or formulation. The medicament can be any dosage form for clinical administration, such as tablets, suppositories, dispersible tablets, enteric-coated tablets, chewable tablets, orally disintegrating tablets, capsules, sugar coated agents, granules, dry powders, oral solutions, a small needle for injection, lyophilized powder for injection, or infusion solutions. According to the specific dosage form and the mode of administration, the pharmaceutically acceptable auxiliaries or excipients in the medicament may include one or more of the following: diluent, solubilizer, disintegrant, suspension, lubricant, adhesive, filler, flavoring agent, sweetener, antioxidant, surfactant, preservative, wrapping agent and pigment, etc.

"Cancer" refers to leukemias, lymphomas, carcinomas, and other malignant tumors (including solid tumors) with potentially unrestrained growth that can expand locally by invasion and systemically by metastasis.

Examples of cancers that can be treated by TH-302 or such hypoxia-activated DNA alkylating agent prodrug, AST-3424 or such AKR1C3-activated DNA alkylating agents include, but are not limited to, cancer of the adrenal gland, bone, brain, breast, bronchi, colon and/or rectum, gallbladder, head and neck, kidney, larynx, liver, lung, nervous tissue, pancreas, prostate, parathyroid, skin, stomach and thyroid. Certain other examples of cancers include acute and chronic lymphocytic and granulocytic neoplasms, adenocarcinoma, adenoma, basal cell carcinoma, cervical dysplasia carcinoma and in situ carcinoma, Ewing's sarcoma, epidermoid carcinoma, giant cell carcinoma, glioblastoma multiforme, hairy-cell tumor, intestinal ganglioneuroma, hyperplastic corneal nerve tumor, islet cell carcinoma, Kaposi's sarcoma, leiomyoma, leukemia, lymphoma, malignant carcinoid, malignant melanoma, malignant hypercalcemia, marfanoid habitus tumor, medullary epithelial carcinoma, metastatic skin cancer, mucosal neuroma, myeloma, mycosis fungoides, neuroblastoma, osteosarcomas, osteogenic and other sarcomas, ovarian tumors, pheochromocytoma, polycythemia vera, primary brain tumor, small cell lung cancer, squamous cell carcinoma of both ulcerative and papillary types, hyperplasia, seminoma, soft tissue sarcoma, retinoblastoma, rhabdomyosarcoma, renal cell tumors, topical skin lesion, reticulum cell sarcoma and Wilm's tumor.

The recommended dosage, preparation administration form of TH-302 or such hypoxia-activated DNA alkylating agent prodrug for treating cancer can refer to the dosage of the patent application text of Threshold Company and other pharmaceutical companies (such as WO2016011195A2, WO2004087075A1, WO2007002931A1, WO2008151253A2, WO2009018163A1, WO2009033165A2, WO2010048330A2, WO2012142520A1, WO2008083101A2, WO2020007106A1, WO2020118251A1, WO2014169035A1, WO2013116385A1, WO2019173799A2, WO2016081547A1, WO2014062856A1, WO2015069489A1, WO2012006032A2, WO2018026606A2, WO2010048330A2, WO2015171647A1, WO2013096687A1, WO2013126539A2, WO2013096684A2, WO2012009288A2, WO2012145684A2, WO2016014390A2, WO2019055786A2, WO2012135757A2, WO2015013448A2, WO2016011328A2, WO2013177633A2, WO2016011195A2, WO2015051921A2) and the dosage in clinical trials registered with FDA (NCT02402062, NCT02020226, NCT02076230, NCT01381822, NCT02093962, NCT01440088, NCT02255110, NCT02342379, NCT01864538, NCT01149915, NCT02433639, NCT00743379, NCT01485042, NCT01721941, NCT02047500, NCT00742963, NCT01497444, NCT00495144, NCT01746979, NCT01144455, NCT01403610, NCT01522872, NCT01833546, NCT02598687, NCT03098160, NCT02496832, NCT02712567):
120 mg/m² to 460 mg/m² daily dose for intravenous injection administration;
480 mg/m² to about 670 mg/m² or for example 575 mg/m² weekly dose for intravenous injection administration.

TH-302 (concentrate for application solution) used in clinical trials is sterile liquid preparation of TH-302. TH-302 was prepared with 70% anhydrous ethanol, 25% dimethylacetamide and 5% polysorbate 80. It is provided by the originators in a 10 mL glass vial with rubber stopper and flip-off closure. TH-302 drug product is a clear, colorless to yellowish solution that is essentially free of visible particles. For a nominal total amount of 650 mg TH-302, each single-use vial contains a nominal fill volume of 6.5 mL of TH-302 drug product (equivalent to 100 mg/mL) and clearly labeled to disclose the lot number, route of administration, required storage conditions, the name of the originators and the appropriate warning marking required by applicable regulations. It is needed to perform dilution according to the pharmacy manual before application.

The commercially available 5% glucose aqueous solution is used to dilute into a total volume of 500 mL (1000 mL for total doses ≥ 1000 mg) prior to administration, to obtain the desired final concentration. Each dose of TH-302 is prepared with 5% glucose aqueous solution without di(2-ethylhexyl)phthalate (without DEHP) and the intravenous drip is performed using intravenous infusion administration device without DEHP.

Lyophilized preparations developed by Threshold Company can also be used:
A solution (20 mL) of TH-302 (100 mg) and sucrose (1 g) is added to a lyophilized vial and lyophilized to produce a lyophilized unit dosage form of TH-302 with a drug load of less than 5 mg/cm³. For the purpose of human administration, the unit dosage form was dissolved in 5% glucose injection and an appropriate amount of the solution is administered to a patient;
The subsequent phase I clinical trial administration regimen of TH-302 in human patients used lyophilized preparations. TH-302 lyophilized preparation for injection is prepared in 100mL glass vial with a drug load of 100 mg/100mL, stored under controlled conditions at 2-8 °C, and 250 mL of 5% glucose injection is injected into the lyophilized preparation vial during use, and the intravenous drip is performed through an infusion pump within 30 minutes.

The recommended dosage, preparation administration form of AST-3424 or such AKR1C3 enzyme-activated DNA alkylating agent prodrug for treating cancer can refer to the patent application text filed by Threshold Company and Ascentawits, OBI, etc. (such as WO2017087428A1, WO2017087428A1, WO2019062919A1, WO2021008520A1) and in clinical trials registered with FDA, NMPA (CTR20201915, CTR20201908, CTR20191399, CTR20191371 and NCT04315324, NCT03592264).

Monotherapy refers to a single drug therapy. Combination refers to a combined drug therapy. Single drug therapy refers to the use of only one anticancer drug in a course of treatment. Combination therapy refers to the simultaneous or sequential use of two or more anticancer drugs in a course of treatment.

Generally speaking, combination therapy needs to explore different administration dosages and administration cycles according to the characteristics of the disease and the types of drugs to be used in combination, and only according to the above situation, the combination therapy plan obtained from the exploration may achieve better therapeutic effect as compared with single drug therapy.

The administration dosages and administration cycles of drug of both monotherapy and combination therapy regimens need to be explored through clinical trials with reference to the dosage and administration regimens of TH-302 and analogues thereof, AST-3424 and analogues thereof and combination drugs as described above.

Furthermore, tumors or cancer tissues of the patient are detected to have mutations in either or both of the genes corresponding to BRCA1 and BRCA2; or the patient is detected to have mutations in either or both of the genes corresponding to BRCA1 or BRCA2.

Mutations in either or both of the genes corresponding to BRCA1 or BRCA2 can be detected with commercially available (companion) diagnostic kits:
Olaparib partner test kit BRACAnalysisCDx, the genetic test is used to detected BRCA gene mutations in blood samples of ovarian cancer patients;
BRCA1/2 gene mutation detection kit (combined probe anchoring polymerization sequencing), which is used to qualitatively detect germline variation in the exon region and adjacent intron region of BRCA1/2 genes of clinically diagnosed ovarian and breast cancer patients;
Human BRCA1 gene and BRCA2 gene mutation detection kit (reversible terminal termination sequencing), which can be used for the relevant medication guidance of PARP inhibitor Olaparil tablets, and it can also be used for the detection of BRCA1 gene and BRCA2 gene mutation of the present invention.

BRCA1 and BRCA2 mutations include the BRCA1 and BRCA2 mutations of germline mutations (gBRCAm) and somatic mutations (sBRCAm).

Wherein tumors or cancer tissues of the patient are detected to have mutations in either or both of the genes corresponding to BRCA1 and BRCA2; or the patient is detected to have mutations in either or both of the genes corresponding to BRCA1 and BRCA2.

The above BRCA1 and BRCA2 are preferably pathogenic mutations.

Treatment method, including the following steps:
detecting BRCA1 and BRCA2 gene mutations of the tumor patient;
If the patient has BRCA1 or BRCA2 gene mutations, using a drug monotherapy containing hypoxia-activated compounds of formulas (1)-(3) or AKR1C3 enzyme-activated compounds of formulas (4)-(12) or in combination with other drugs for treating.

The present invention also provides a method for treating gene-mutated cancer, which uses a drug monotherapy containing hypoxia-activated prodrug compounds of formulas (1), (2), (3) or AKR1C3 enzyme-activated prodrug compounds of formulas (4), (5), (6), (7), (8), (9), (10), (11), (12) and salts, esters, solvates, and isotopic isomers thereof or in combination with other drugs to treat patients with gene-mutated cancer and tumors, wherein the gene mutation is any mutation in the homologous DNA double-strand damage repair genes.

Homologous DNA double-strand damage repair genes are selected from the corresponding genes of FANCA, FANCD1, FANCD2, ATM, ATR, CHEK1, CHEK2, CTP, BARD1, BRIP1, PALB2, RAD51D, RAD51C, RAD52, RAD54, RAD55, RAD57, FAM175, NBN, Rad50, MRE11, p53, NBS1, XRS2, XRCC2, XRCC3, XRCC4/XPF, ERCC1, ERCC2/XPD, ERCC3/XPB, ERCC4/XPF, XRCC1, Ku80, MHS6, MGMT, PARP, ERCC5/XPG, CCNH, CDK7, CETN2, DDB1, DDB2, ERCC5/XPG, ERCC6/CSB, ERCC8/CSA, LIG1/DNA ligase I, MMS19, MNAT1, RAD23A, RAD23B, RPA1, RPA2, TFIIH, XAB2, XPA, XPC, MBD4, NEIL1, BAP1, CDK12, EXO1, FAAP20, FAN1, FANCE, FANCM, MDC1, NONO, POLQ, RAD51B, RBBP8, SMC5, USP11, WRN and AP endonuclease, end processing enzyme, DNA polymerase, Flap endonuclease, DNA ligase.
BRCA1, BRCA2, namely Breast Cancer 1, 2 genes.
FANCA, namely Fanconi anemia complememtation group A gene, FANCD1, FANCD2, namely Fanconi anemia group D1, 2 protein genes.
ATM, Ataxia Telangiectasia-Mutated gene.
ATR, ATM-Rad3-Related gene.
CHEK1, CHEK2, Checkpoint kinase 1, 2 gene.
CTP, Cytidine Triphosphate gene.
BARD1, BRCA1-associated RING domain protein 1 gene.
BRIP1, BRCA1-interacting protein 1 gene.
RAD51D, RAD51C, RAD52, RAD54, RAD55, RAD57, namely Restriction-site associated DNA, that is restriction endonuclease site 51D, 51C, 52, 54, 55, 57 associated DNA genes.
FAM175, family with sequence similarity 175 member gene.
NBN, Nijmegen breakage syndrome 1 gene.
Rad50, RAD23A, RAD23B, RAD51B, DNA repair protein RAD50, RAD23A, RAD23B, RAD51B gene.
MRE11, Mismatch Repair Endonuclease 11 gene.
p53, Tumor protein p53 gene.
NBS1, Nijmegen breakage syndrome 1 gene.
XRS2, a homologous gene of human Nbs1.
XRCC1, XRCC2, XRCC3, DNA repair protein X-Ray Repair Cross Complementing1, 2, 3 gene.
ERCC1, ERCC2, ERCC3, ERCC4, ERCC5/XPG, ERCC6/CSB, ERCC8/CSA, Excision Repair Cross-Complementation group 1, 2, 3, 4, 5, 6, 8 gene.
Ku80, the gene encoding Ku80 protein.
MHS6, MutS homolog 6 gene.
MGMT, Methylguanine Methyltransferase gene.
PARP, poly ADP-ribose polymerase gene.
CCNH, Cyclin H gene.
CDK7, 12, Cyclin Dependent Kinase 7, 12 gene.
CETN2, Centrin 2 gene.
DDB1, DDB2, Damage Specific DNA Binding Protein 1/2 gene.
LIG1/DNA Ligase I, DNA ligase 1 gene.
MMS19, MMS19 Homolog, Cytosolic Iron-Sulfur Assembly Component.
MNAT1, NAT1, CDK Activating Kinase Assembly Factor gene.
RPA1, RPA2, Replication Protein A1, A2 gene.
TFIIH, Transcription Factor IIH gene.
XAB2, XPA Binding Protein 2 gene.
XPA, DNA Damage Recognition And Repair Factor gene.
XPC, PC Complex Subunit, DNA Damage Recognition And Repair Factor gene.
MBD4, Methyl-CpG Binding Domain 4, DNA Glycosylase gene.
NEIL1, Nei Like DNA Glycosylase 1 gene.
BAP1, BRCA1 Associated Protein 1.
EXO1, Exonuclease 1.
FAAP20, Fanconi Anemia Core Complex Associated Protein 20.
FAN1, FANCD2 And FANCI Associated Nuclease 1.
FANCE, Fanconi Anemia Complementation Group E.
FANCM, Fanconi Anemia Complementation Group M.
MDC1, Mediator Of DNA Damage Checkpoint 1.
NONO, Non-POU Domain Containing Octamer Binding.
POLQ, DNA Polymerase Theta.
RBBP8, RB Binding Protein 8, Endonuclease.
SMC5, Structural Maintenance Of Chromosomes 5.
USP11, Ubiquitin Specific Peptidase 11.
WRN, Werner Syndrome RecQ Like Helicase.
AP endonucleases.
End processing enzymes.
DNA polymerases.
Flap endonuclease.

The mutations of above **homologous DNA double-strand damage repair genes** of tumor patient are detected.

If the patient has the mutations of **homologous DNA double-strand damage repair genes,** a drug monotherapy containing hypoxia-activated compounds of formulas (1)-(3) or AKR1C3 enzyme-activated compounds of formulas (4)-(12) or in combination with other drugs was used for treating.

The combination of other drugs comprises immunotherapy drugs, preferably, the immunotherapy drugs are selected from immune checkpoint inhibitors, more preferably, the immunotherapy drugs are selected from PD-1 monoclonal antibodies and PD-L1 monoclonal antibodies.

The combination of other drugs also comprises PARP inhibitor, preferably, the PARP inhibitor is Olaparib.

Immune checkpoint molecules are regulatory molecules in the immune system that play an inhibitory role and are essential for maintaining self-tolerance, preventing autoimmune reactions, and minimizing tissue damage by controlling the timing and intensity of the immune response. Immune checkpoint molecules are expressed on immune cells, and will inhibit the function of immune cell, thereby preventing the body from generating an effective anti-tumor immune response, and causing immune escape of tumor. Tumor-related immune checkpoint molecules include PD1, PD-L1, CTLA4, Tim3, and LAG3, etc. Currently, PD1, PD-L1, and CTLA4 are more frequently studied. Immune checkpoint inhibitors are some monoclonal antibody drugs developed for corresponding immune checkpoints. Their main function is to block the interaction between tumor cells expressing immune checkpoints and immune cells, thereby blocking the inhibitory effect of tumor cells on immune cells. Immunotherapy drugs are selected from the group consisting of PD-1 monoclonal antibodies and PD-L1 monoclonal antibodies.

The TMB (Tumor Mutation Load (burden)) level of the described gene mutation is medium.

Since the TMB (Tumor Mutation Load (burden)) is differently high or low between different tumor types: it is generally considered that: a TMB of more than 20 mutations/Mb (Mb represents bases per million) is high; a TMB of less than 10 mutations/Mb is low, and a TMB in the middle is medium. At the World Conference on Lung Cancer in 2017, Squibb Inc. had announced the results of a clinical trial named CheckMate-032. This was a phase II clinical trial that enrolled 401 patients with advanced lung cancer who had failed first-line therapy and were treated with a PD-1 inhibitor alone or in combination with Ipilimumab. The patients were divided into three categories of patients with high TMB, medium TMB, and low TMB according to their TMB levels, then among those who received the combination therapy, the efficiency of the three groups was 62%, 20%, and 23%, respectively, and the efficiency of the group with high TMB was three times higher than the remaining two groups; and the median overall survival of the three groups was: 22.0 months, 3.6 months, and 3.4 months, respectively, with 22.0 months versus 3.4 months, a 6-fold difference! This trial demonstrated that for different cancer treatment drugs, different TMB levels have a significant impact on the efficacy of the drugs.

As a preference, cancers or tumors are selected from adenocarcinoma, gastric cancer, ovarian cancer, breast cancer, cervical cancer, prostate cancer, liver cancer, non-small cell lung cancer, colon cancer, rectal cancer.

The hypoxia-activated prodrug compound of formula (1) is selected from the compounds of following structures:

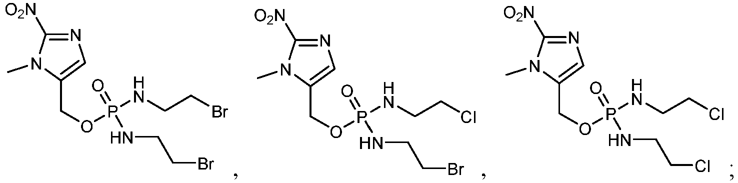

The specific synthesis method of the compound of formula I and the corresponding spectral data are disclosed in WO2007002931 (corresponding to the Chinese publication text CN101501054A), which is incorporated herein by reference in its entirety.

The hypoxia-activated prodrug compound of formula (2) is selected from the compounds of following structures:

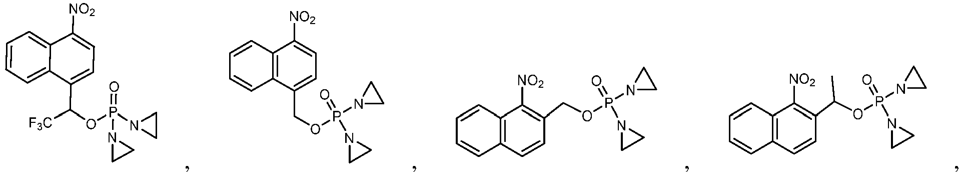

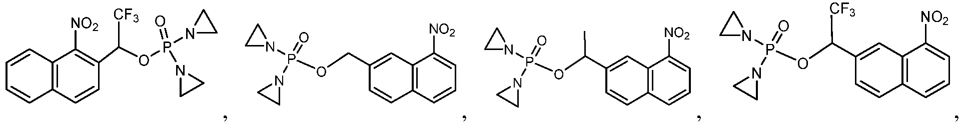

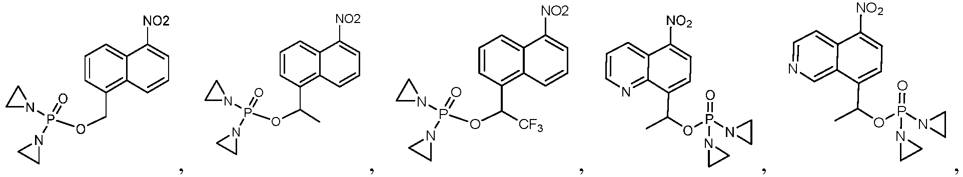

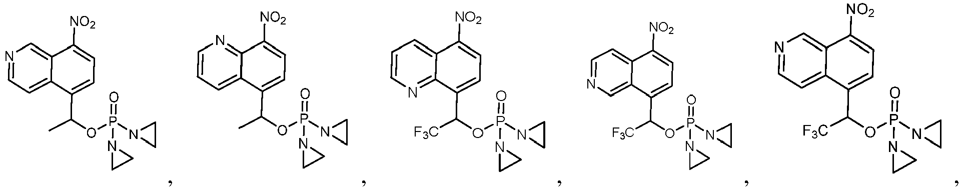

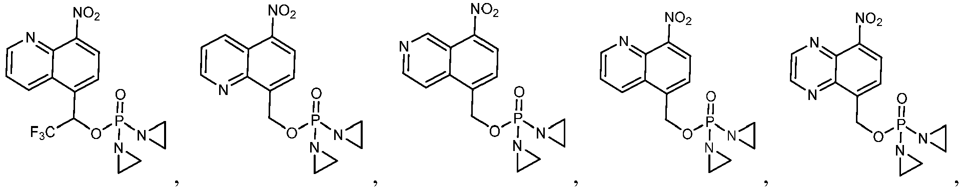

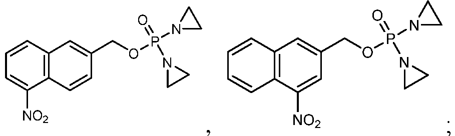

The hypoxia-activated prodrug compound of formula (3) is selected from the compounds of following structures:

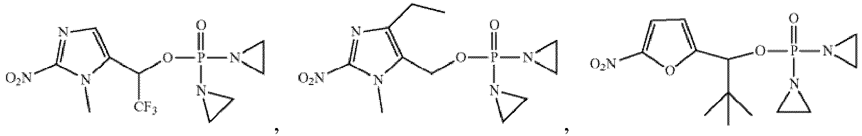

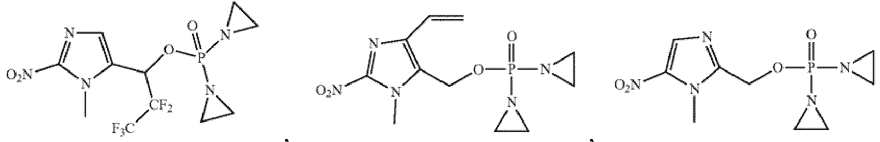

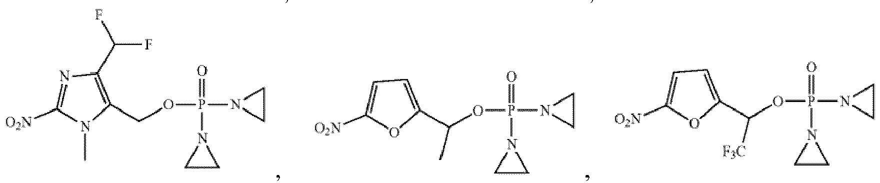

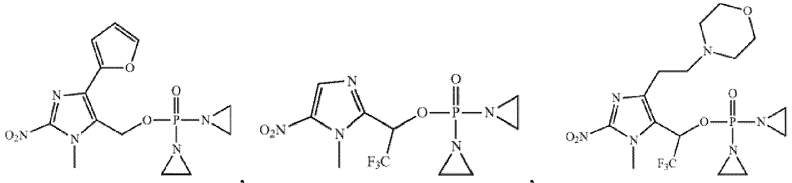

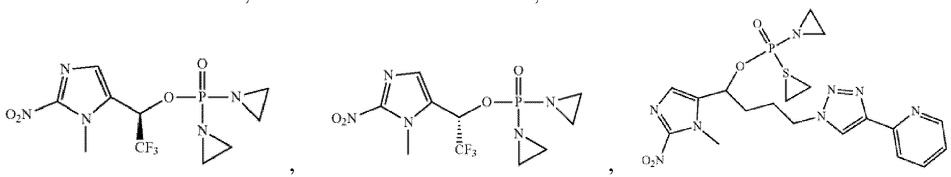

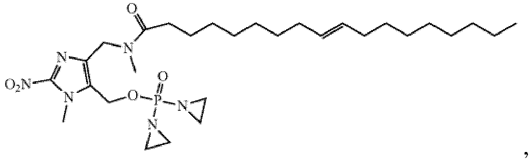

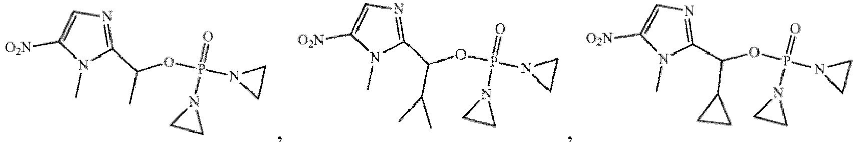

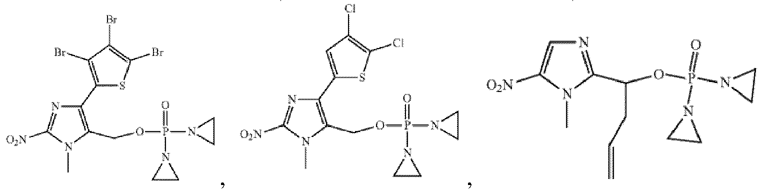

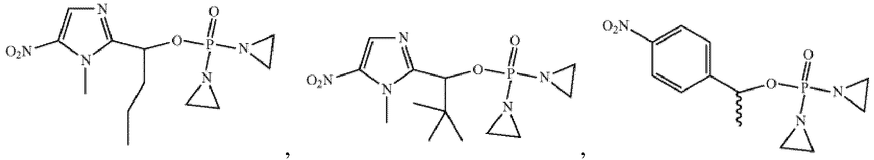

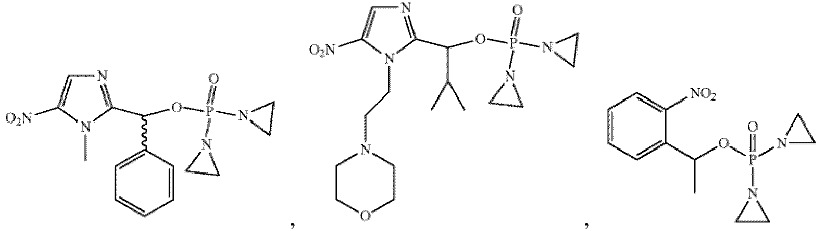

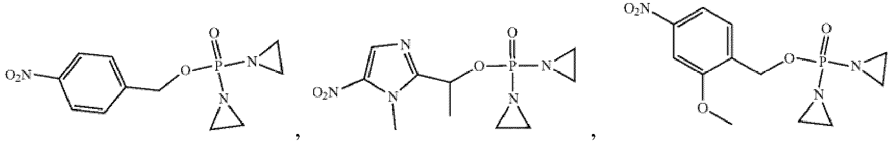

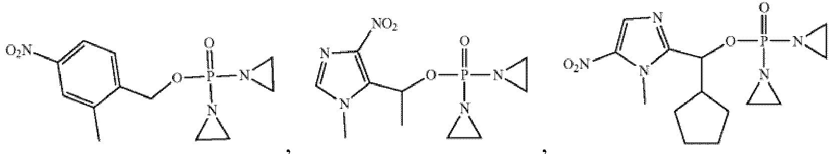

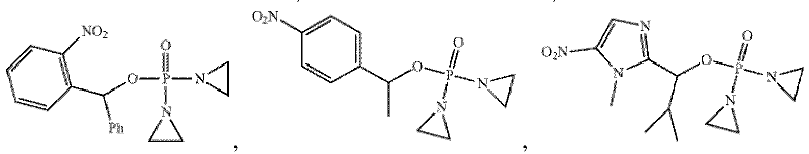

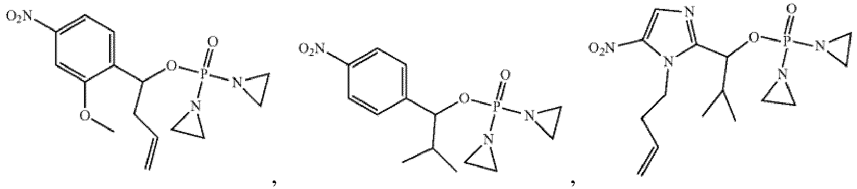

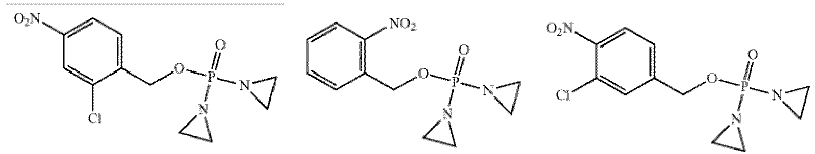

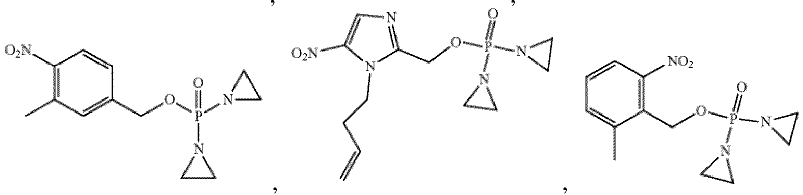

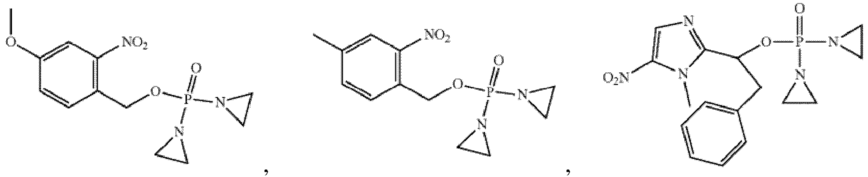

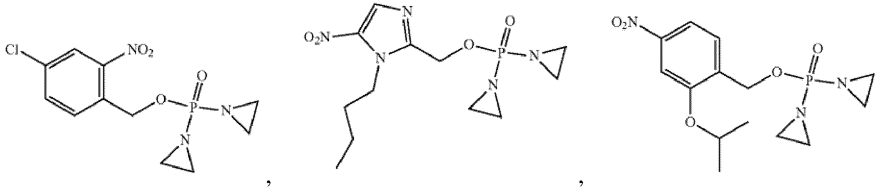

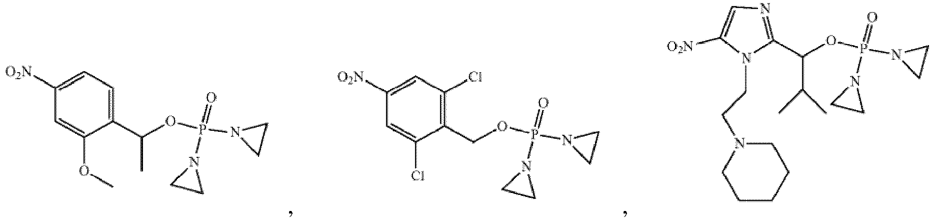

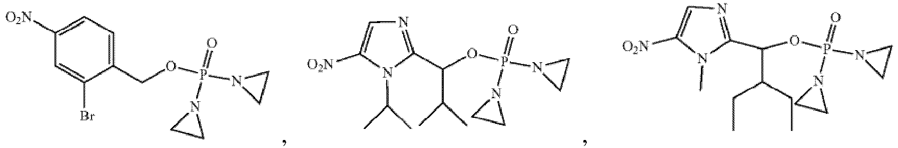

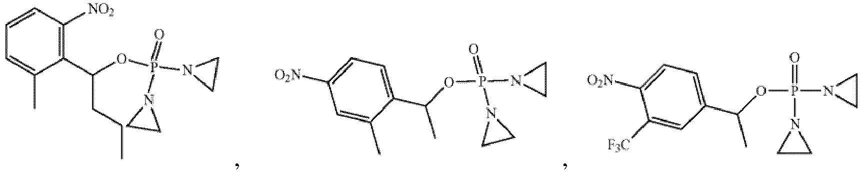

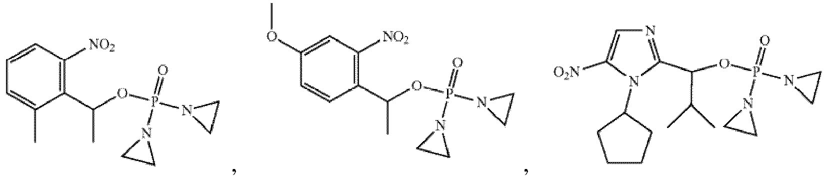

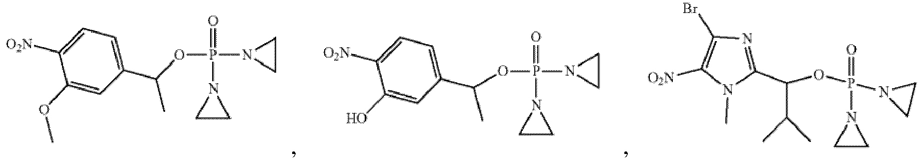

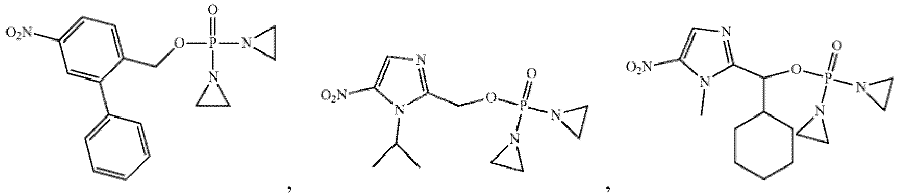

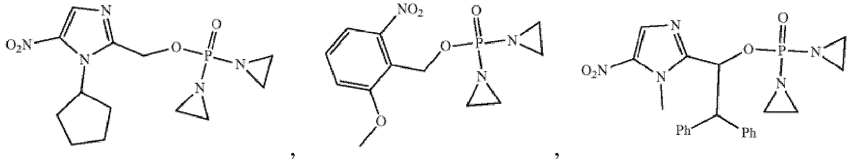

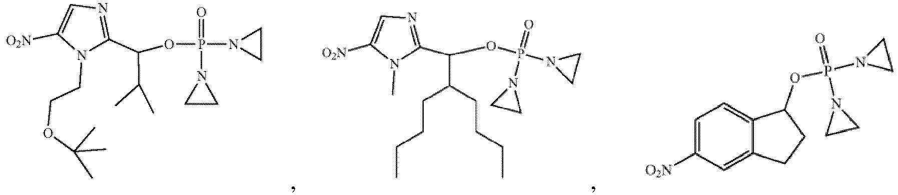

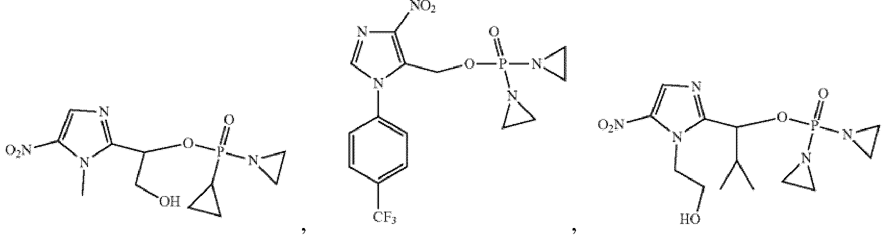

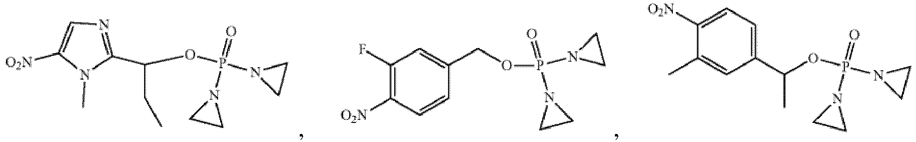

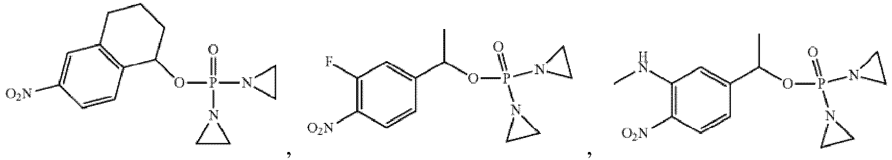

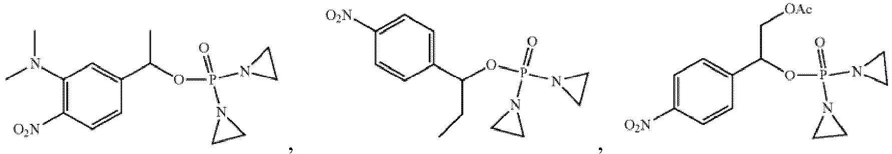

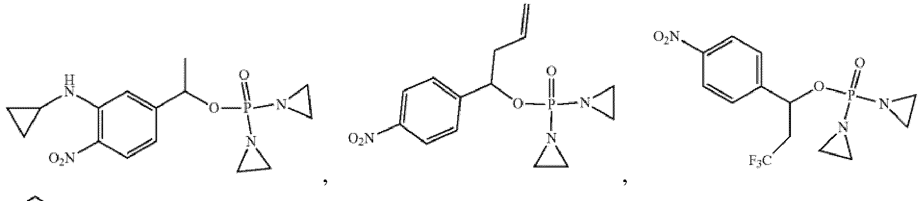

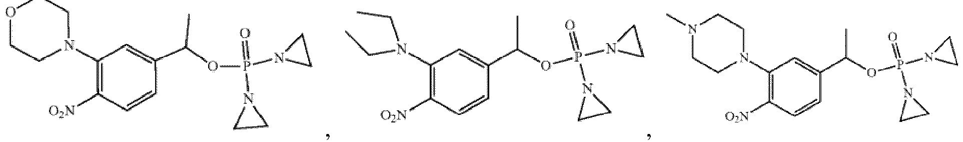

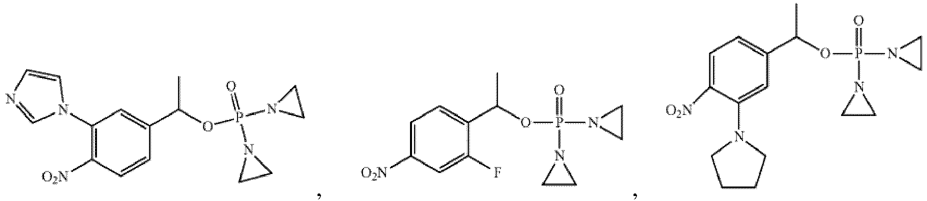

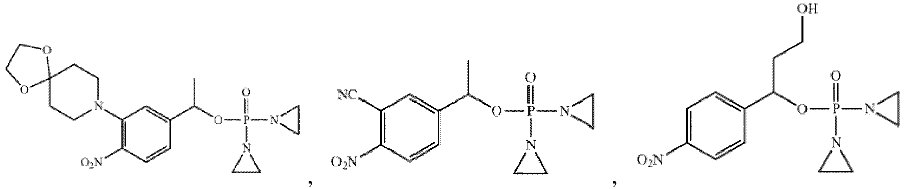

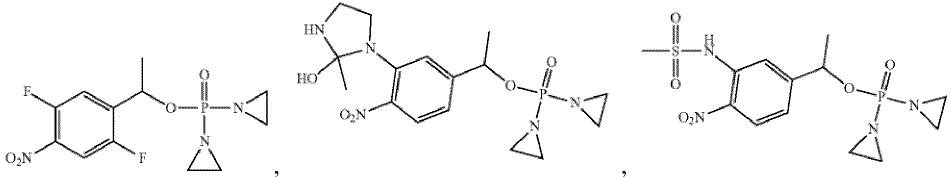

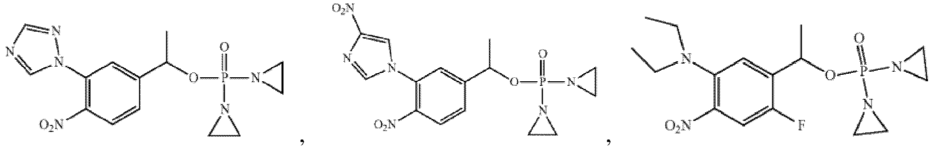

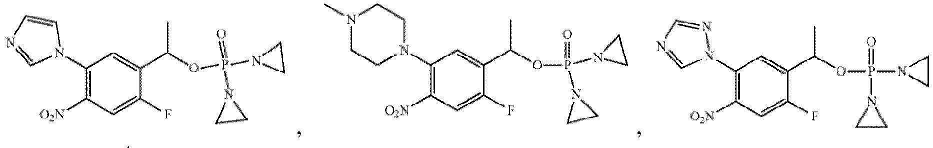

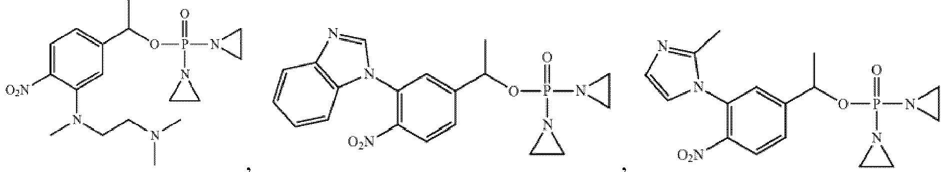

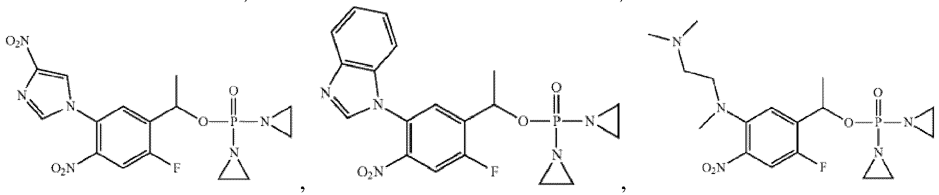

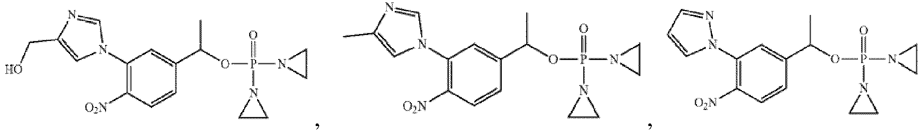

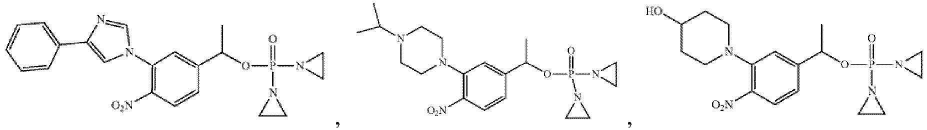

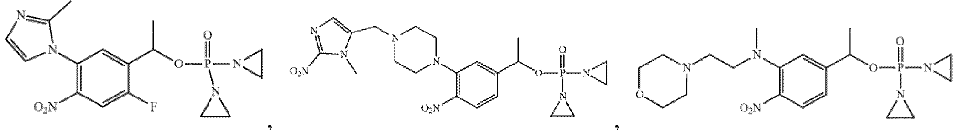

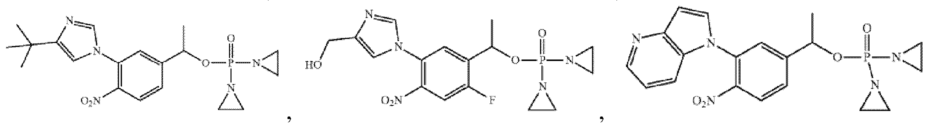

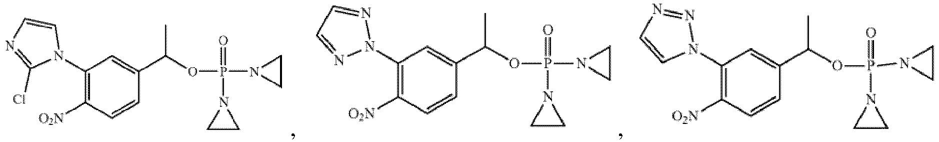

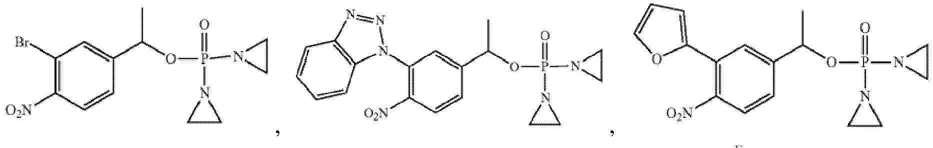

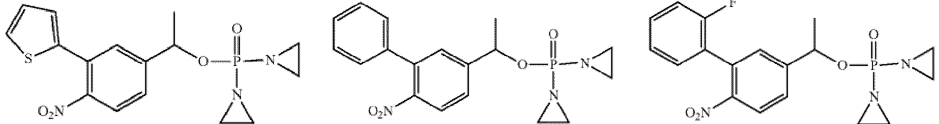

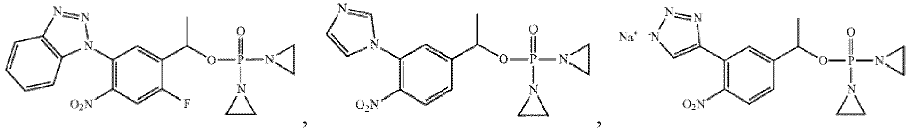

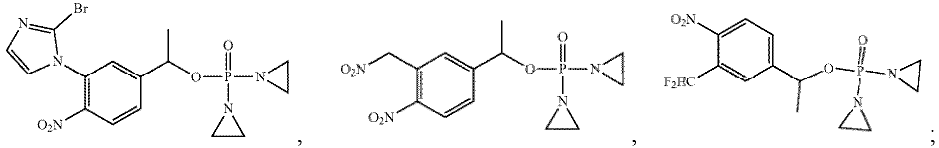

The AKR1C3 enzyme-activated prodrug compound of formula (4) is selected from the compounds of following structures:

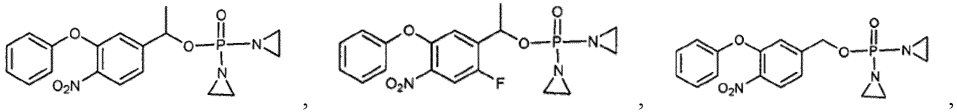

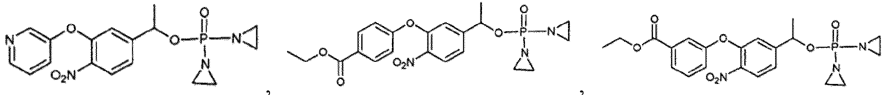

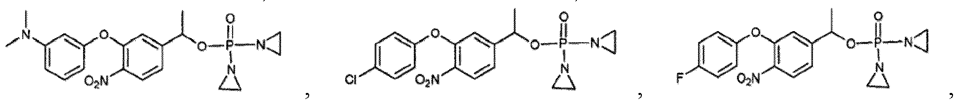

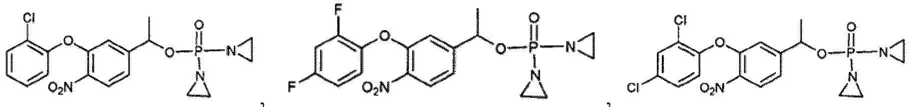

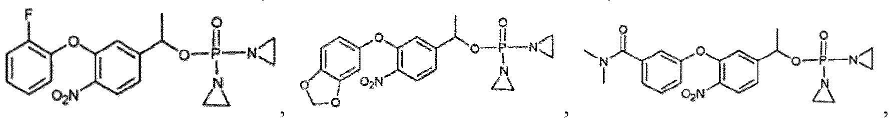

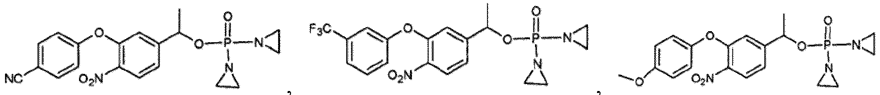

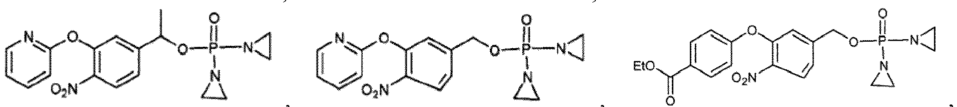

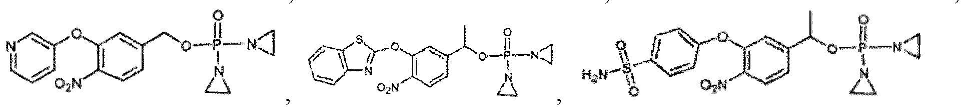

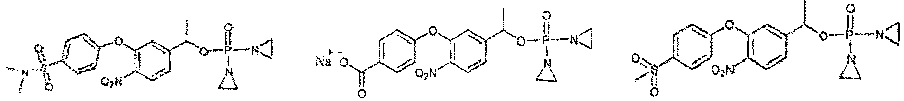

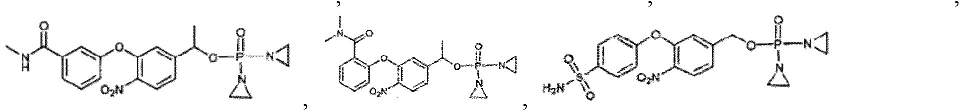

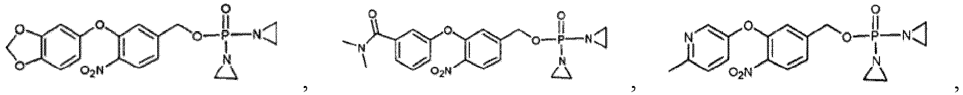

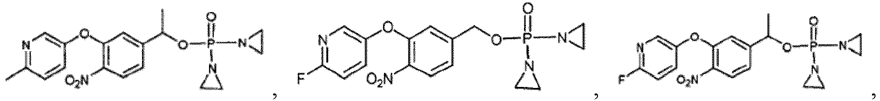

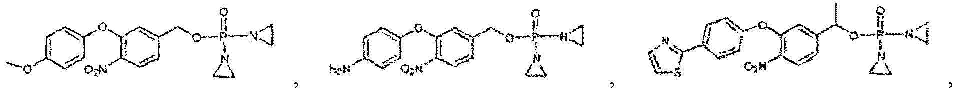

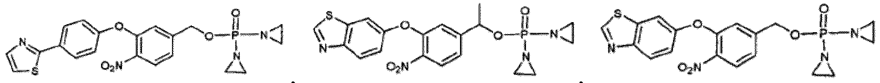

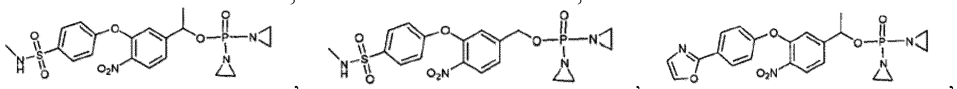

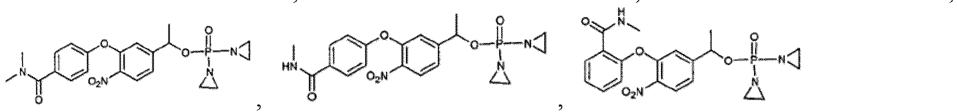

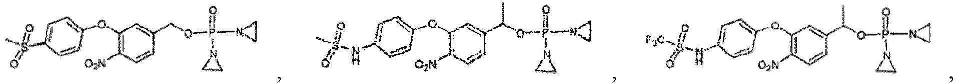

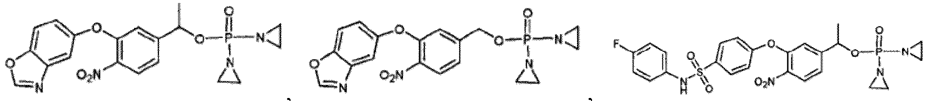

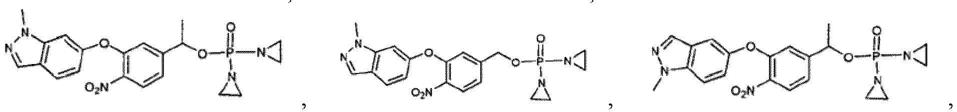

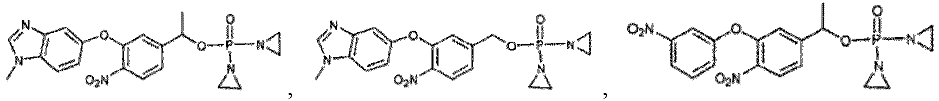

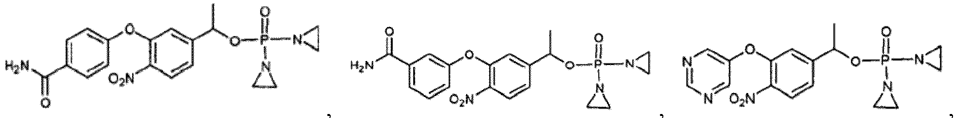

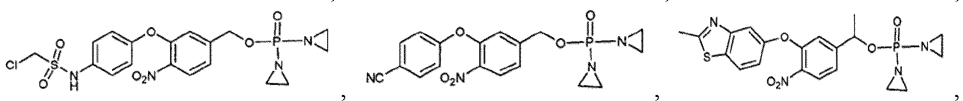

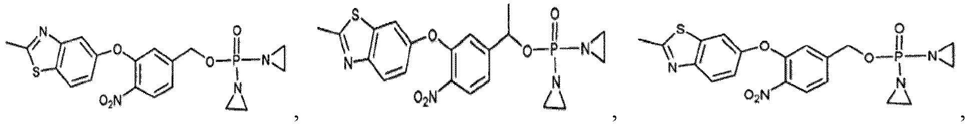

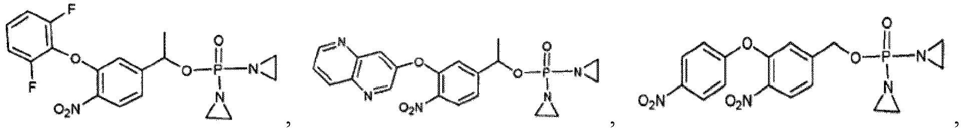

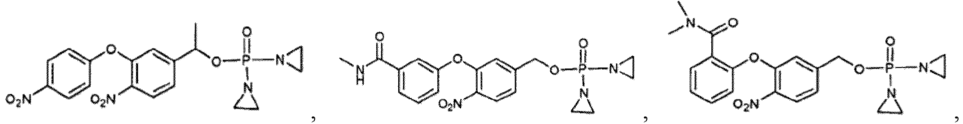

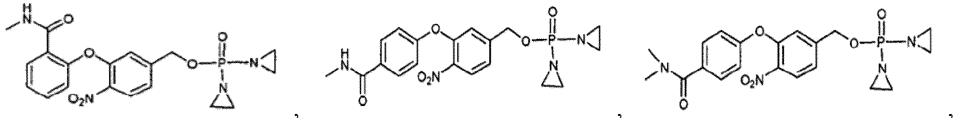

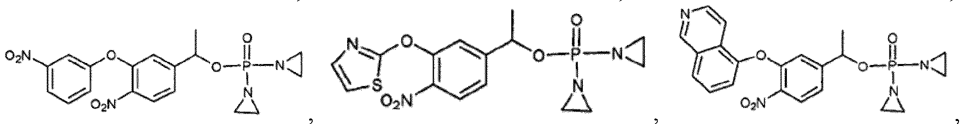

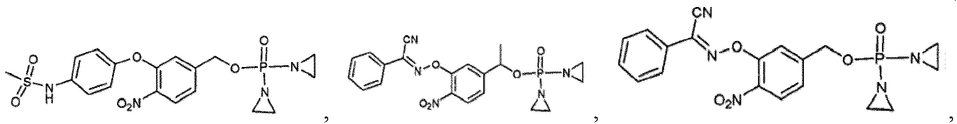

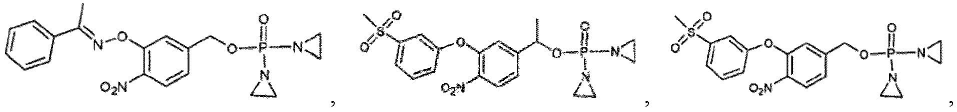

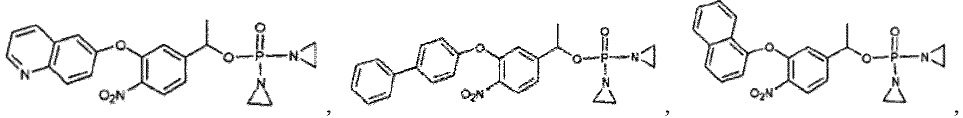

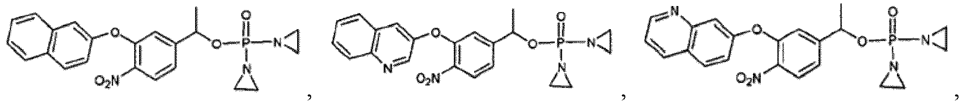

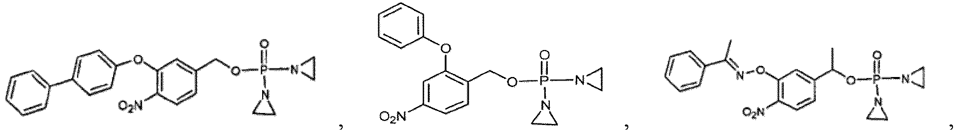

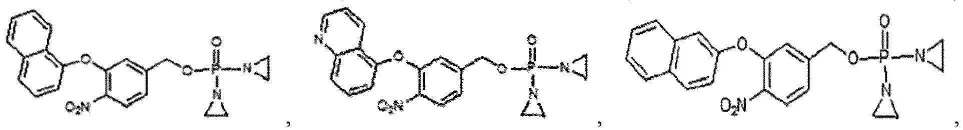

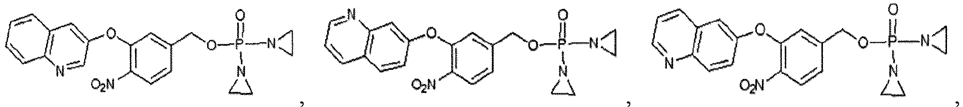

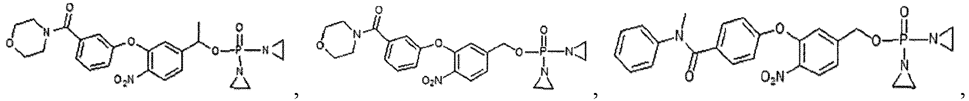

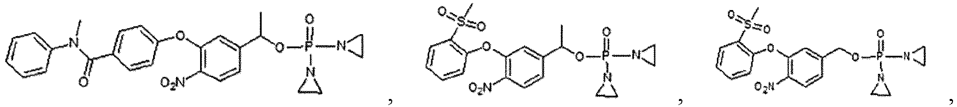

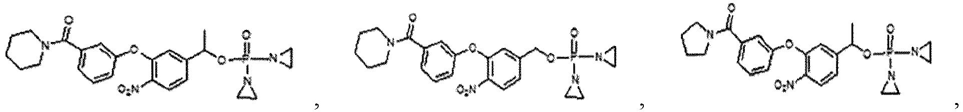

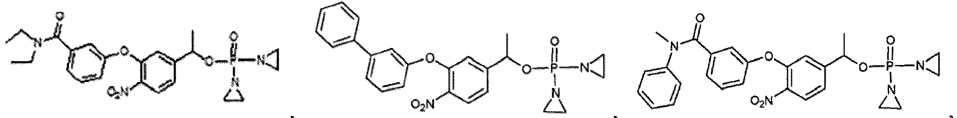

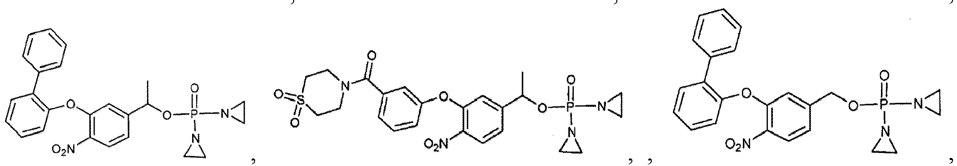

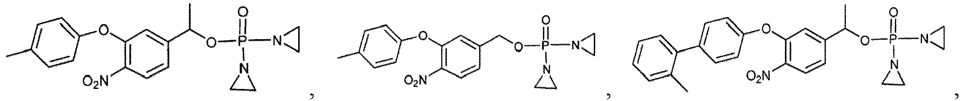

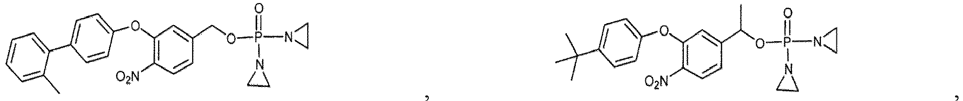

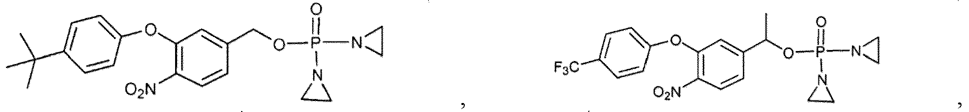

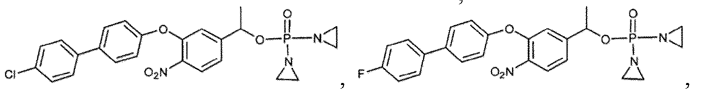

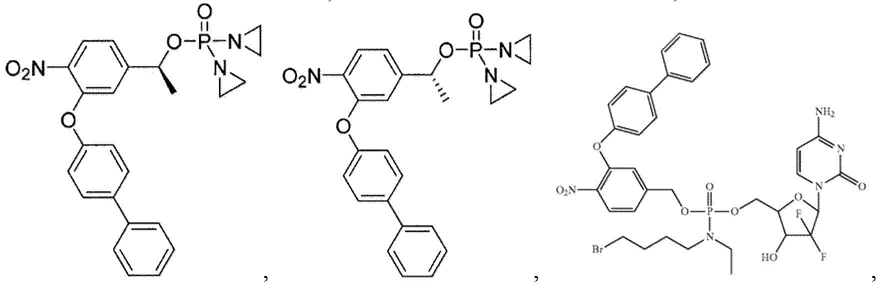

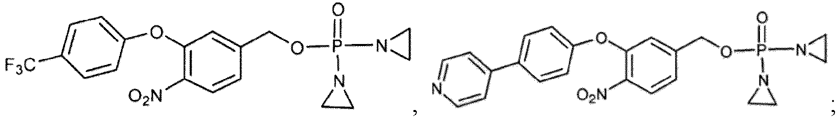

The AKR1C3 enzyme-activated prodrug compound of formula (6) is selected from the compounds of following structures:

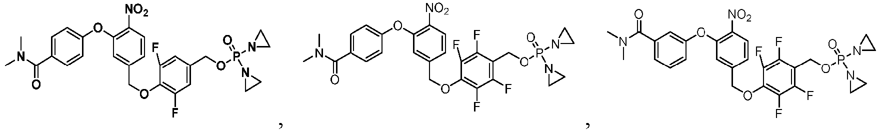

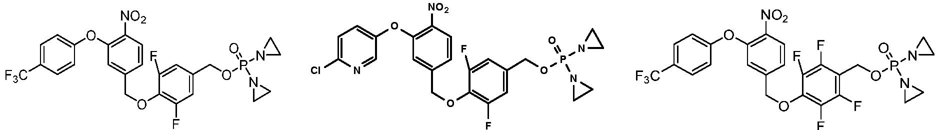

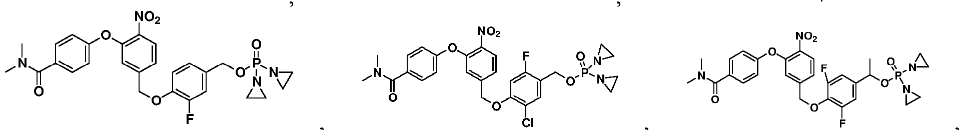

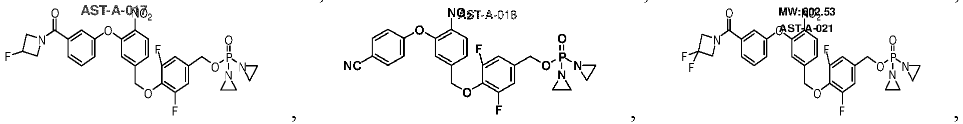

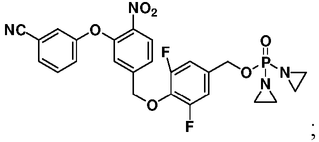

The AKR1C3 enzyme-activated prodrug compound of formula (5) is selected from the compounds of following structures:

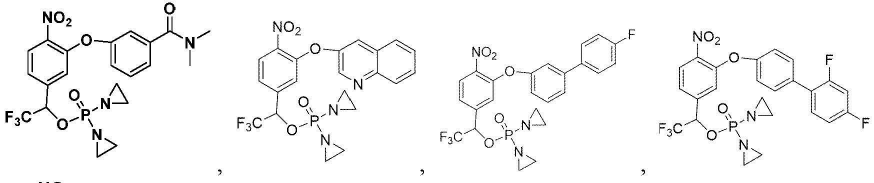

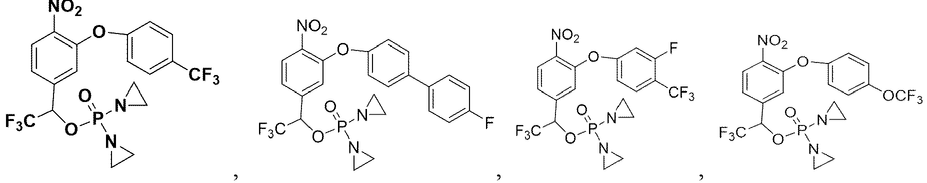

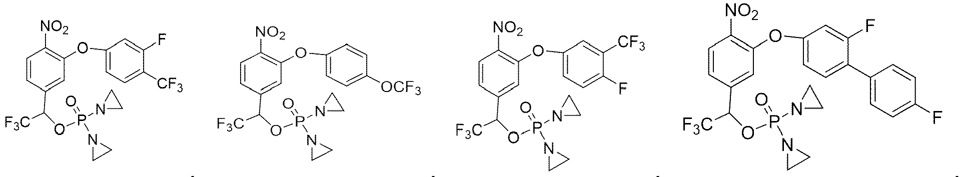

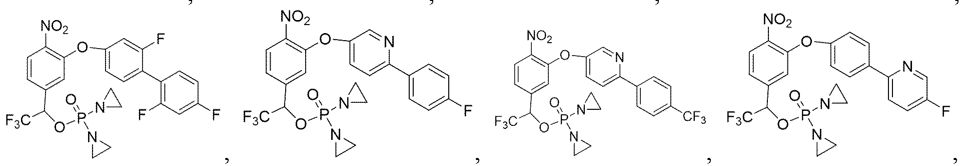

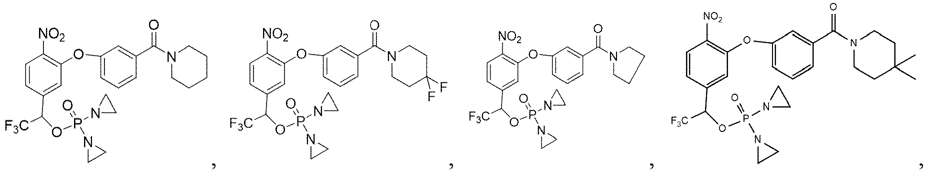

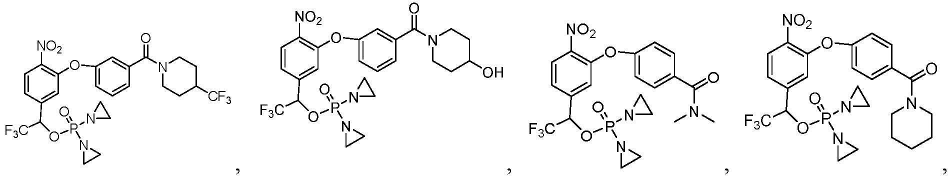

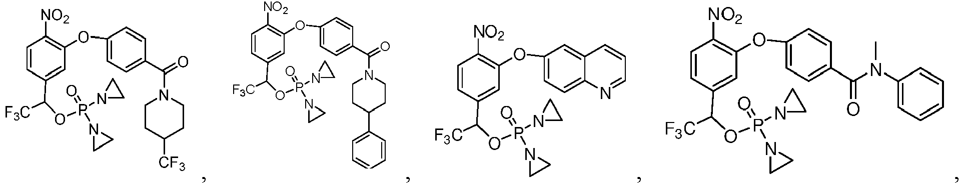

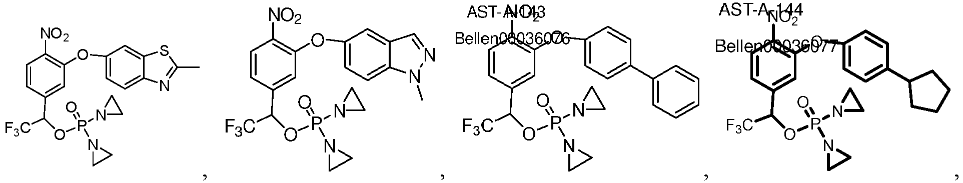

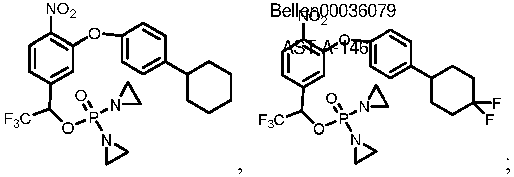

The AKR1C3 enzyme-activated prodrug compound of formula (7) is selected from the compounds of following structures: 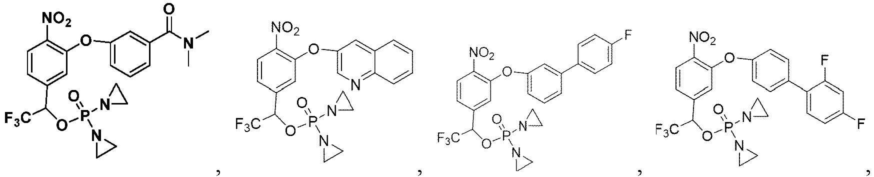 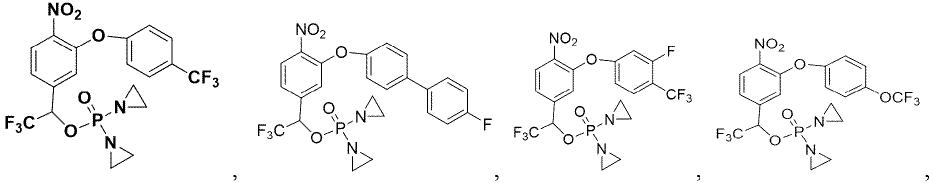 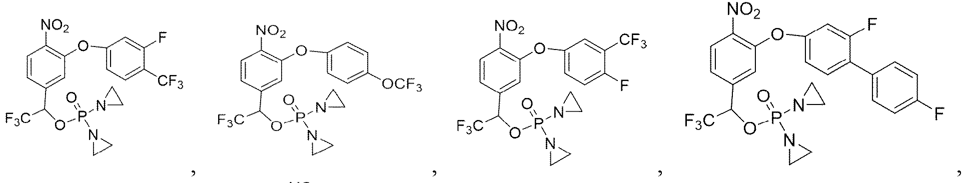 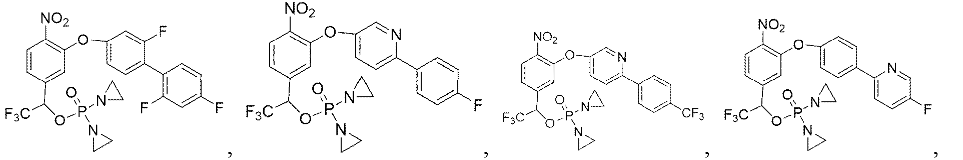 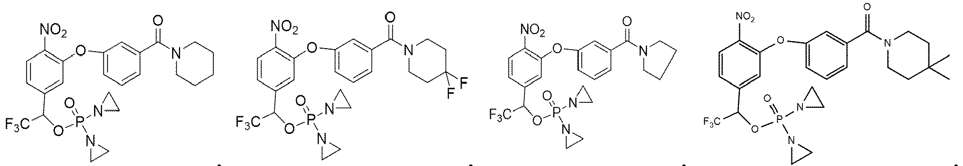 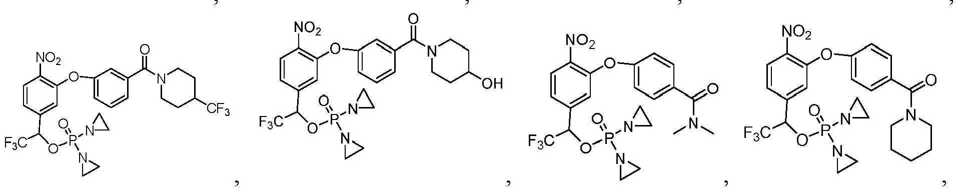 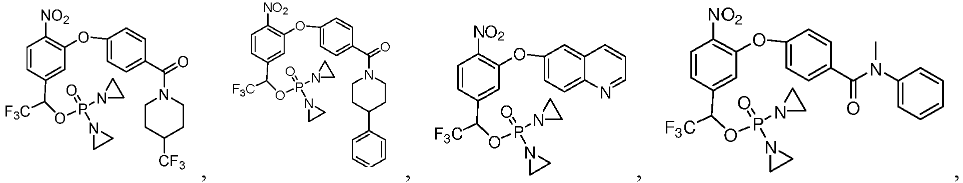 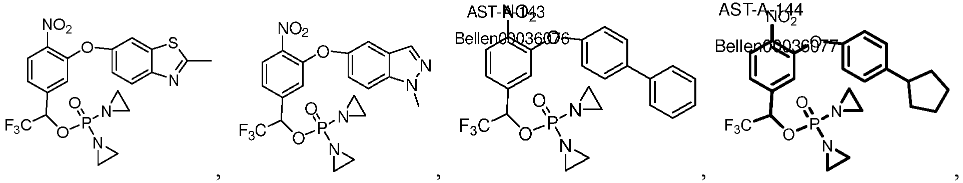 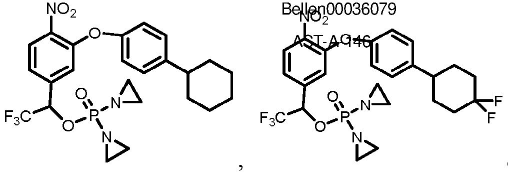 and

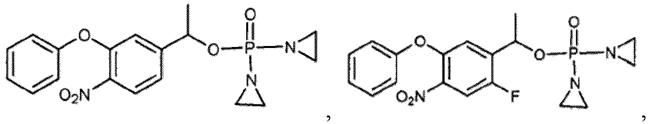

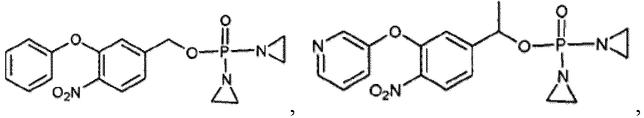

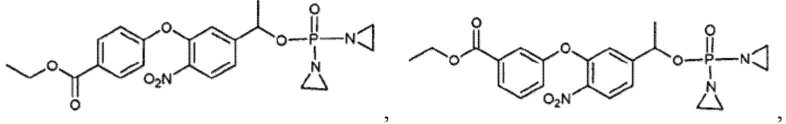

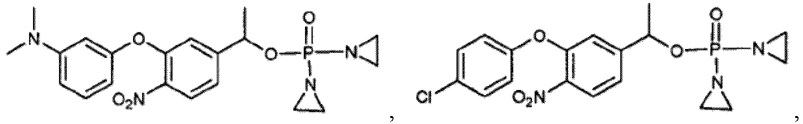

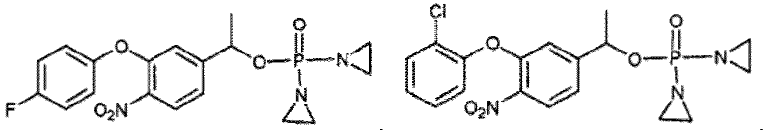

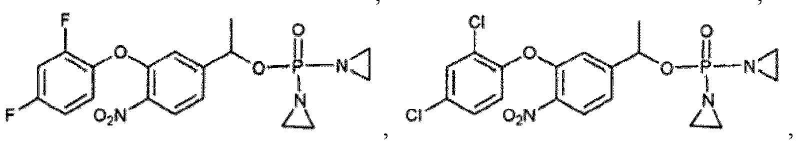

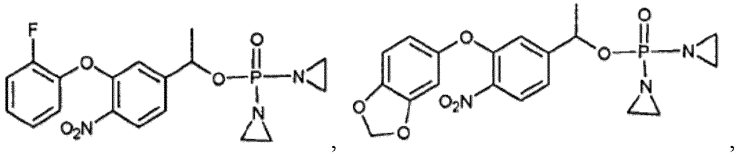

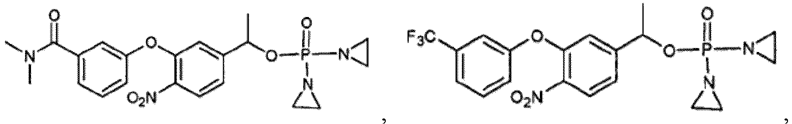

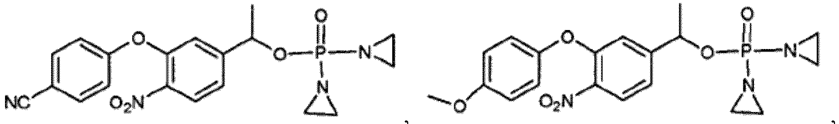

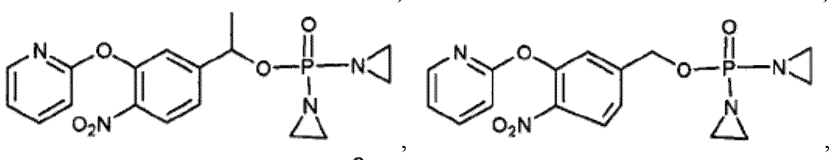

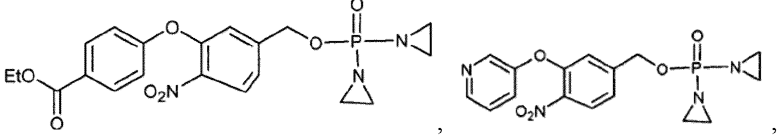

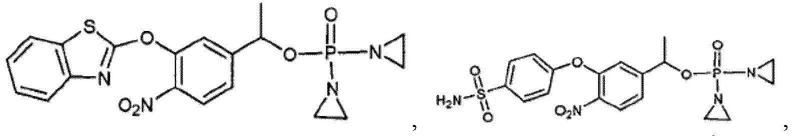

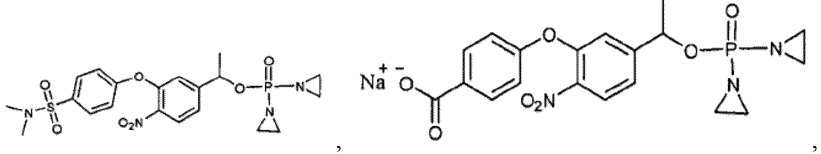

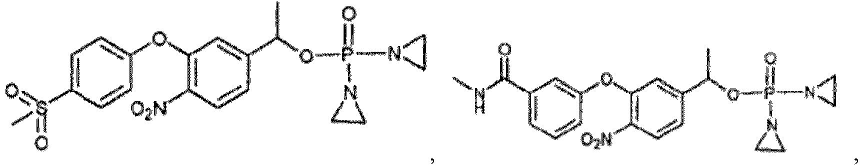

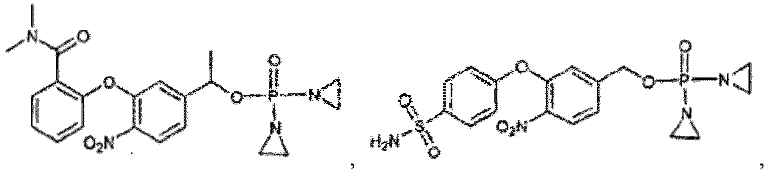

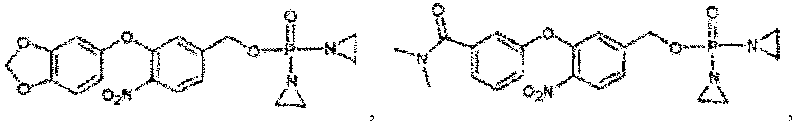

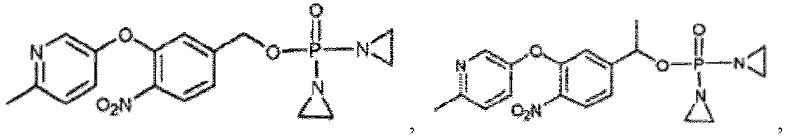

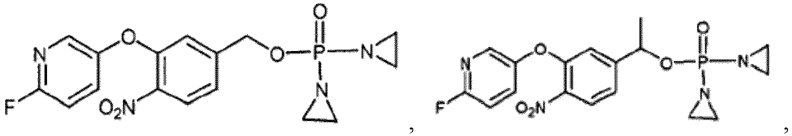

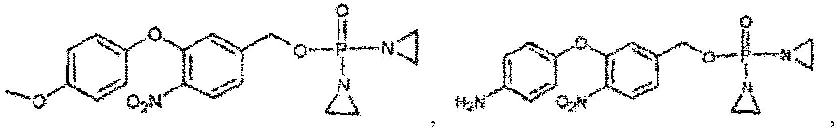

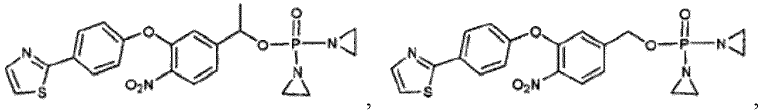

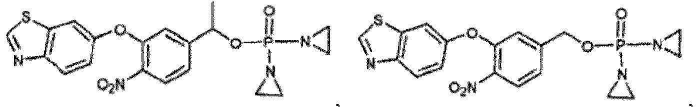

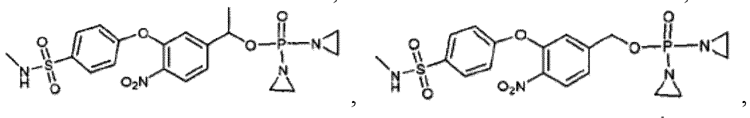

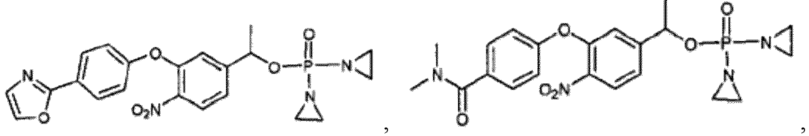

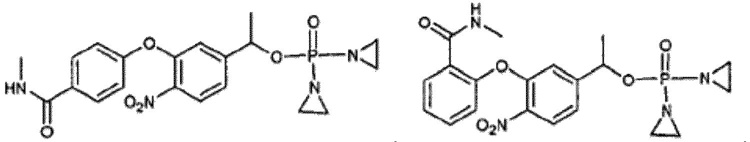

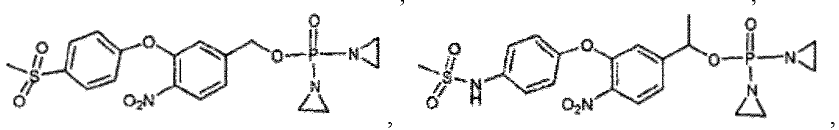

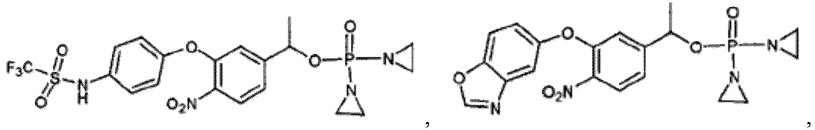

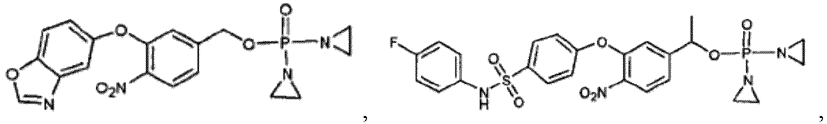

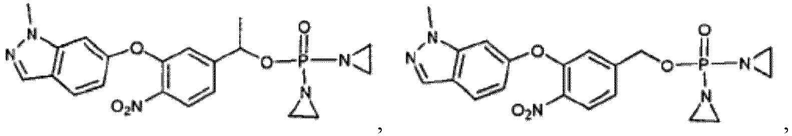

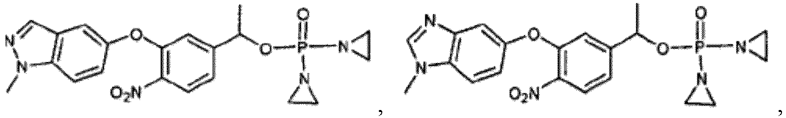

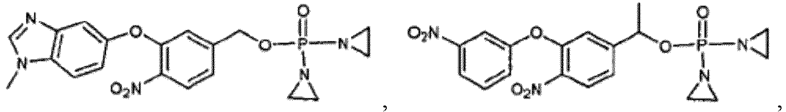

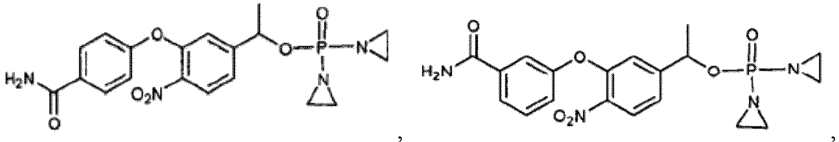

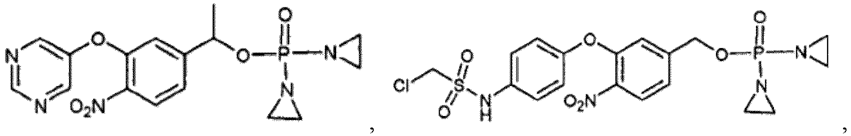

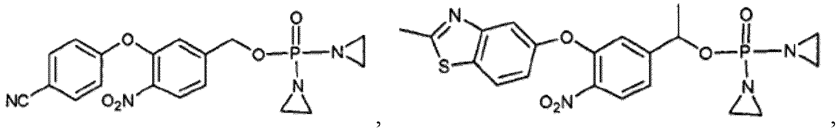

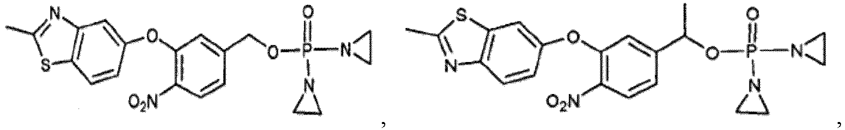

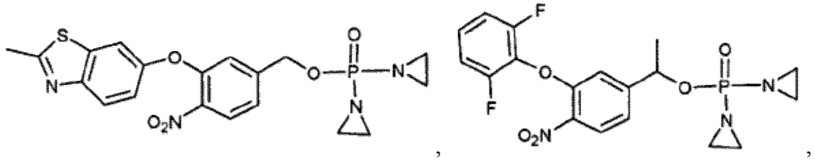

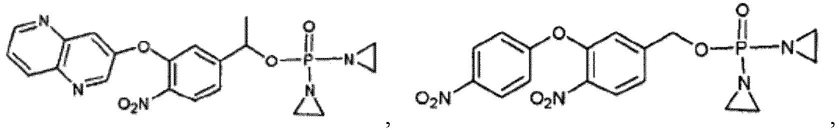

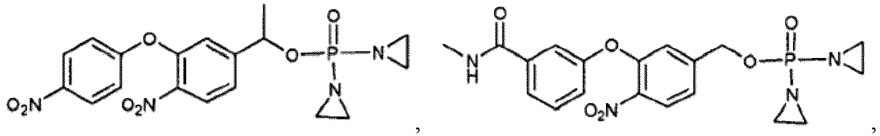

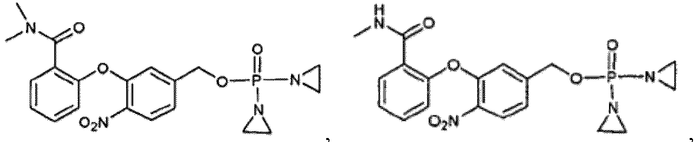

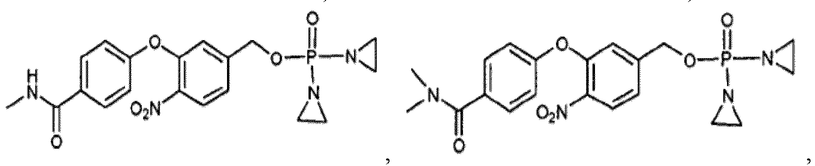

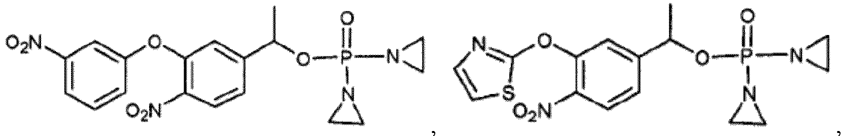

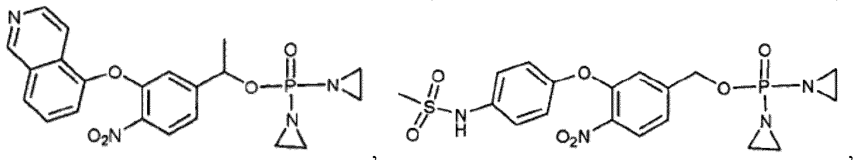

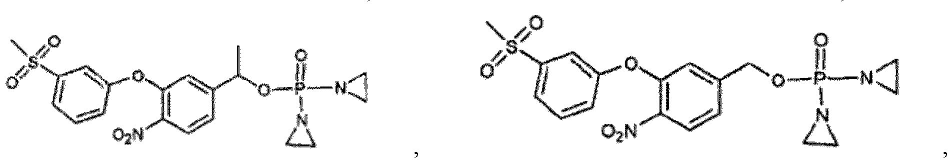

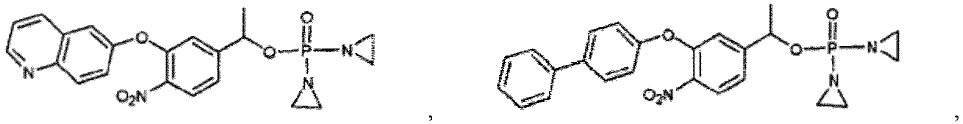

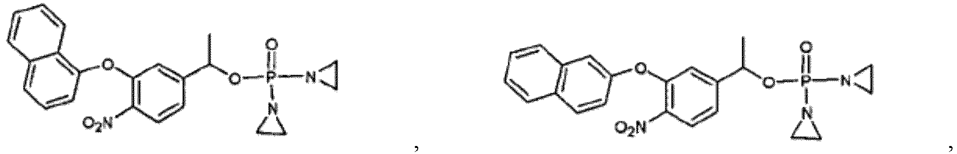

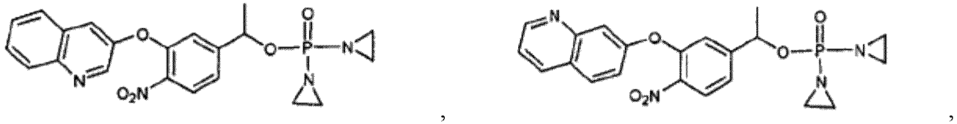

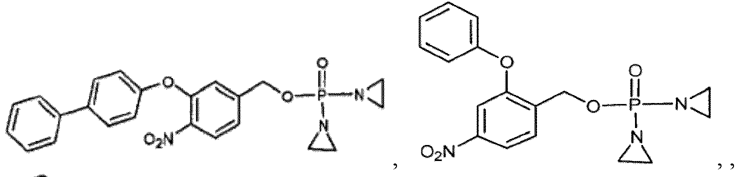

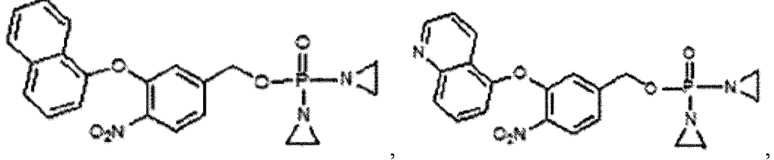

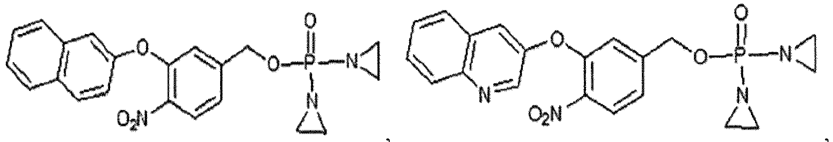

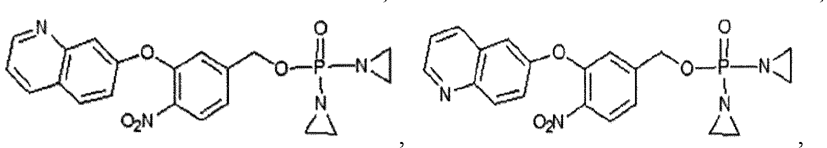

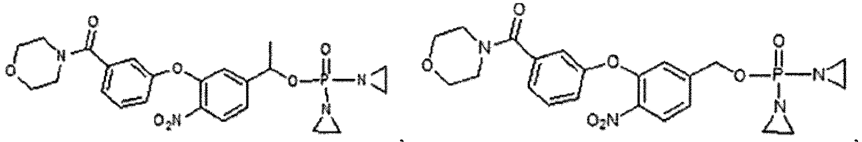

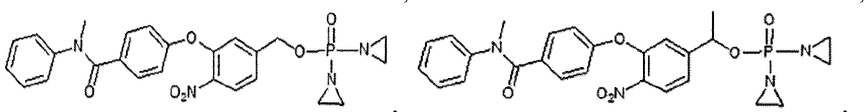

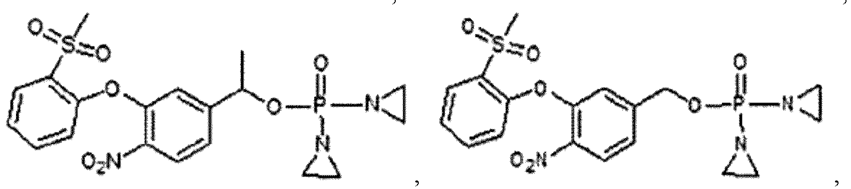

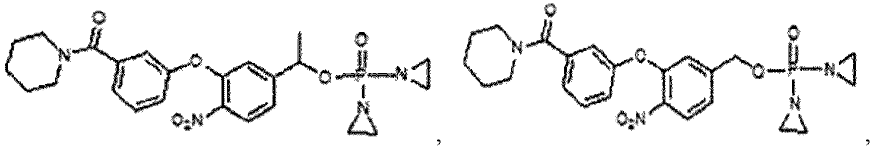

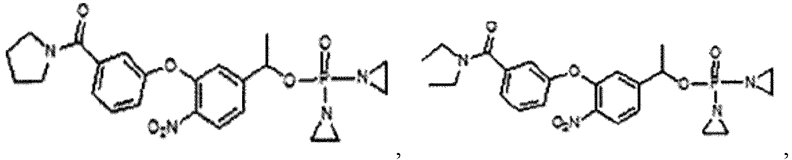

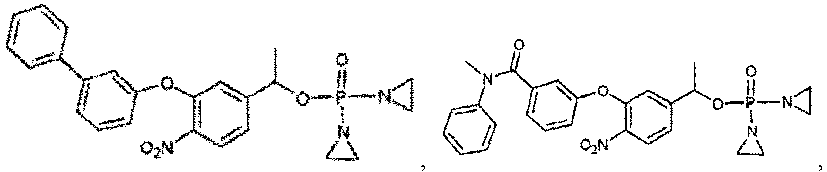

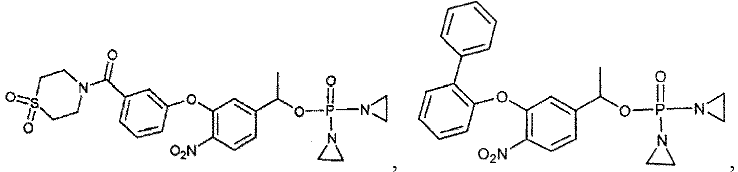

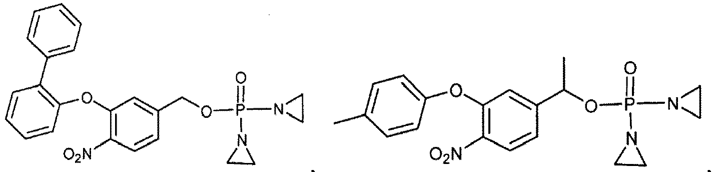

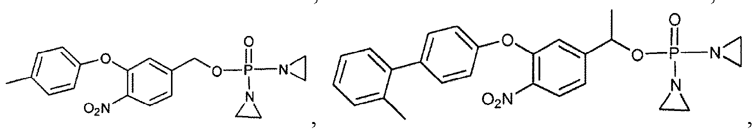

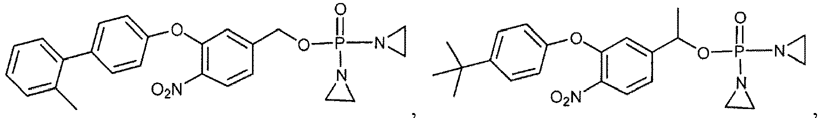

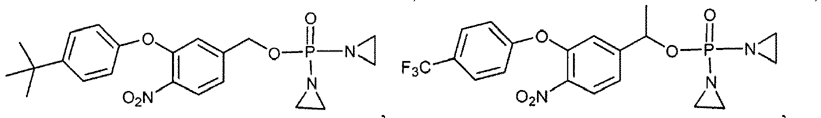

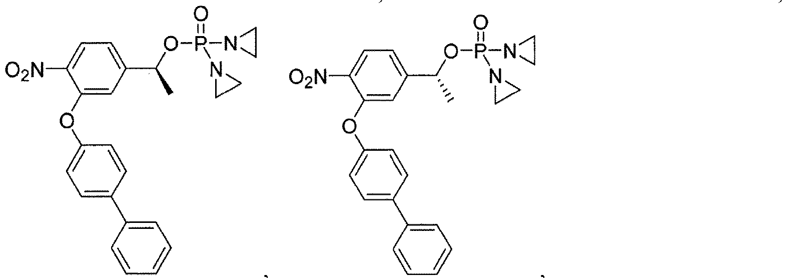

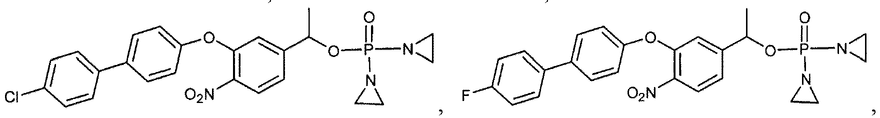

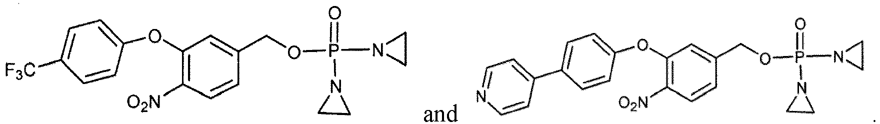

The AKR1C3 enzyme-activated prodrug compound of formula (8) is selected from the compounds of following structures: 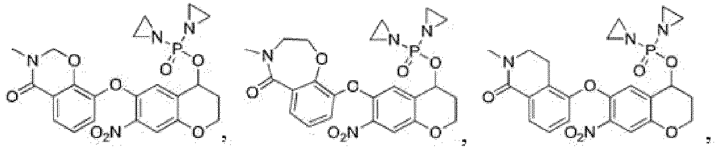 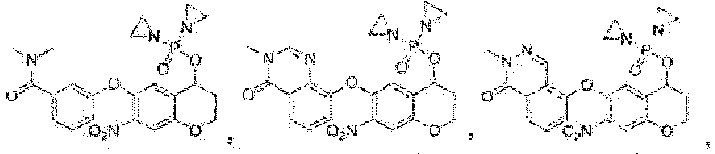 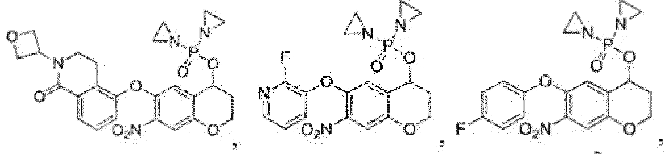 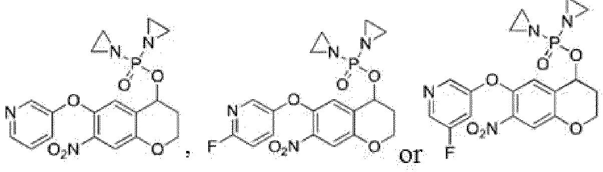 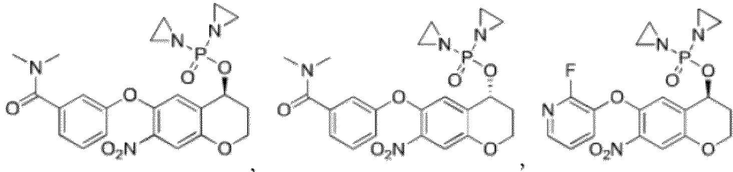 or

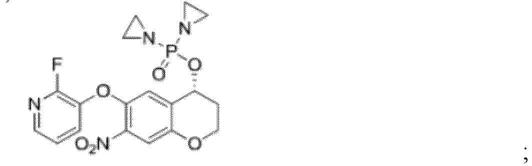

The AKR1C3 enzyme-activated prodrug compound of formula (9) is selected from the compounds of following structures:

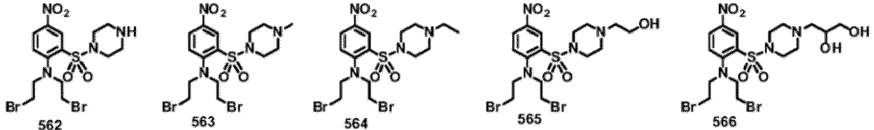

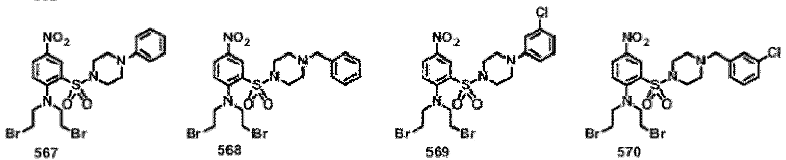

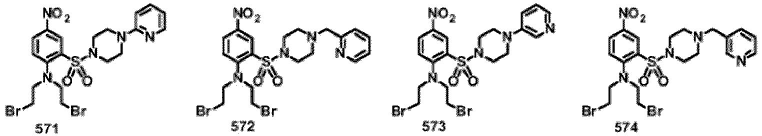

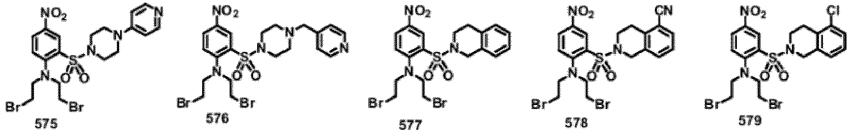

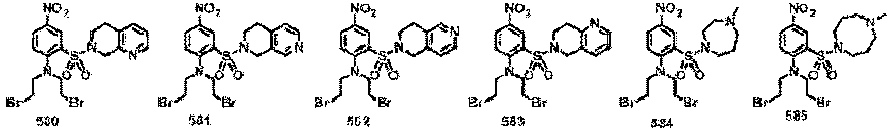

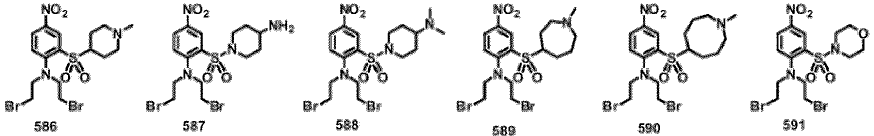

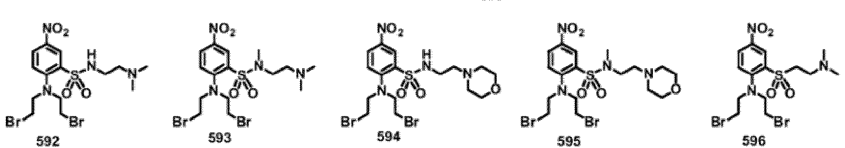

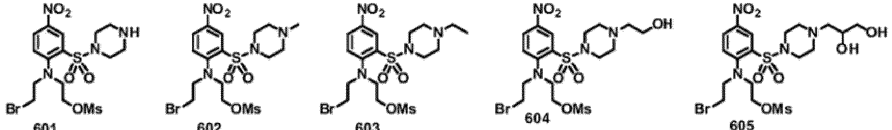

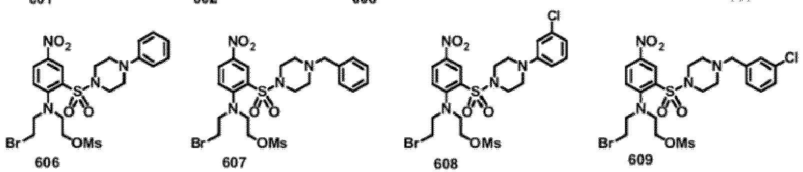

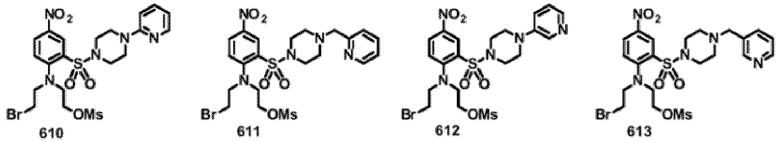

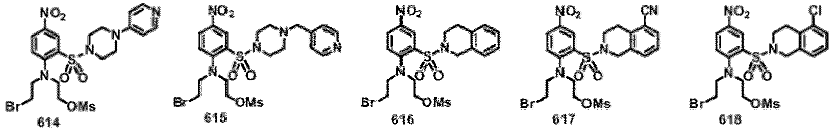

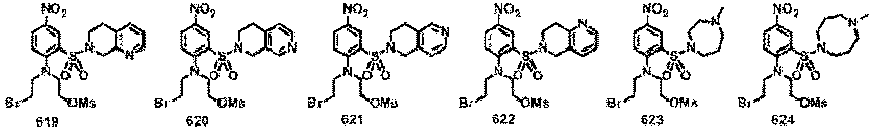

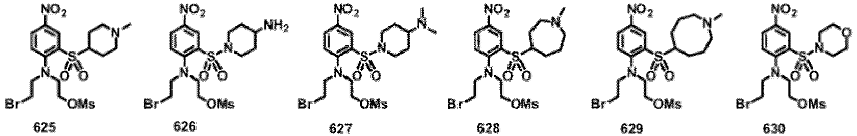

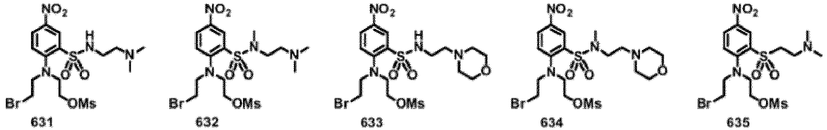

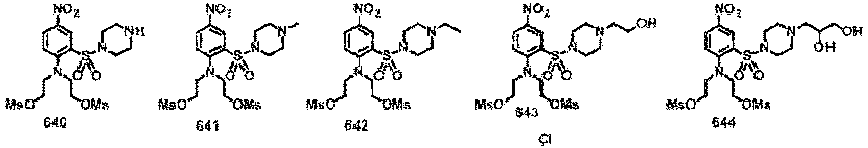

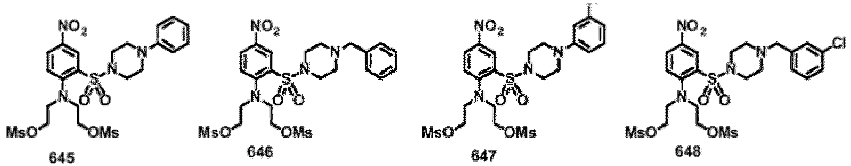

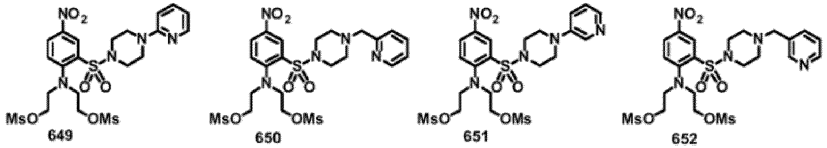

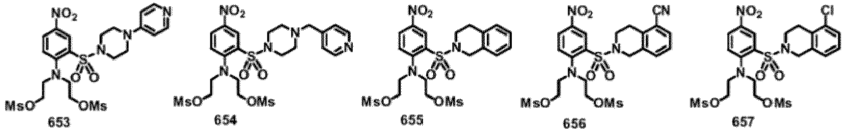

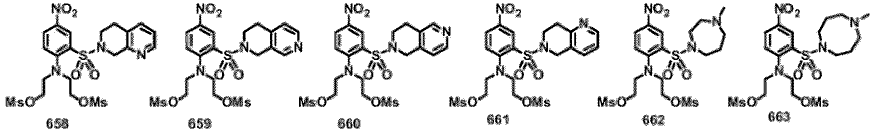

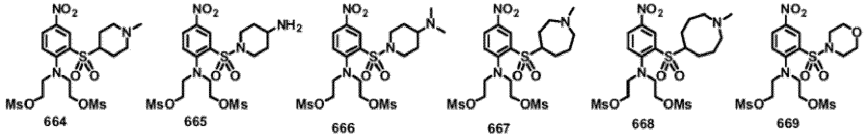

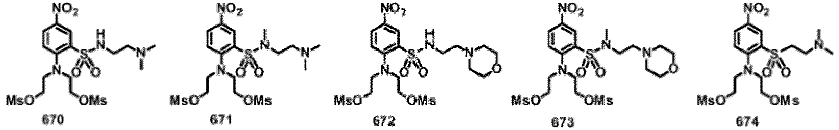

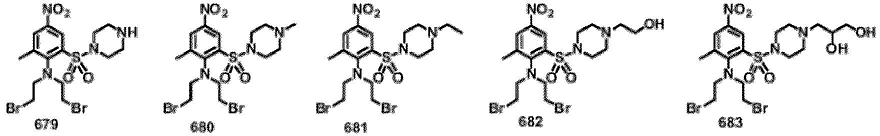

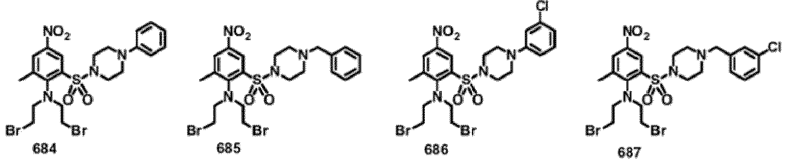

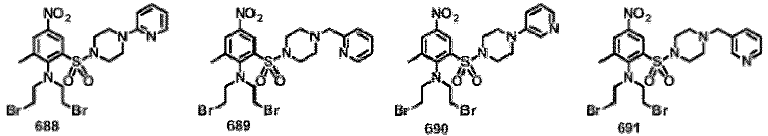

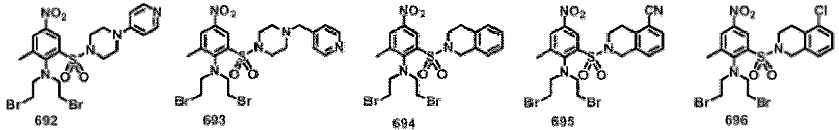

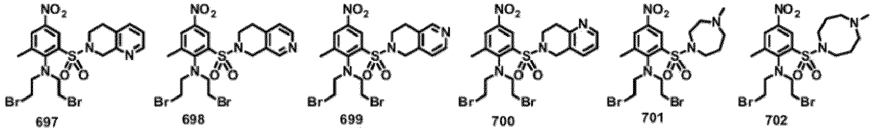

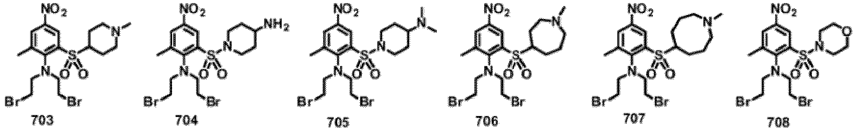

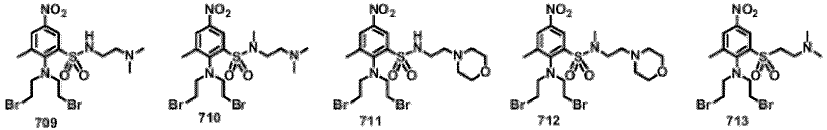

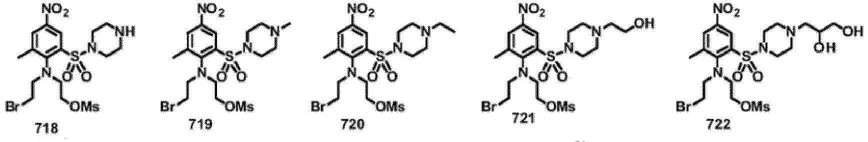

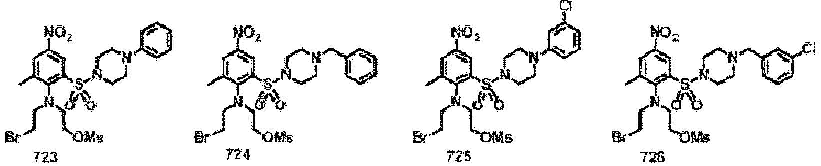

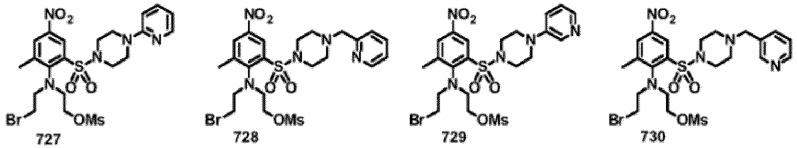

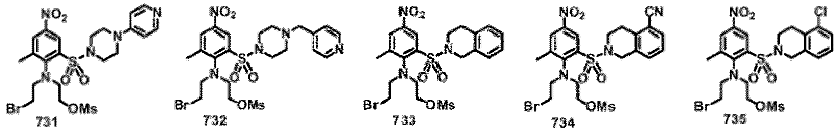

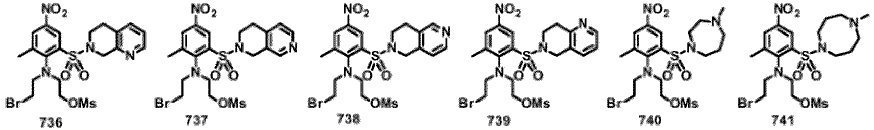

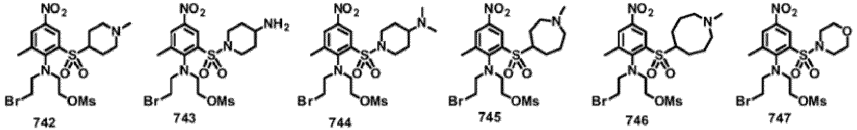

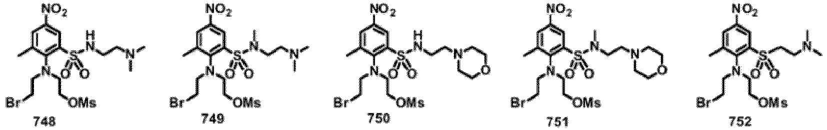

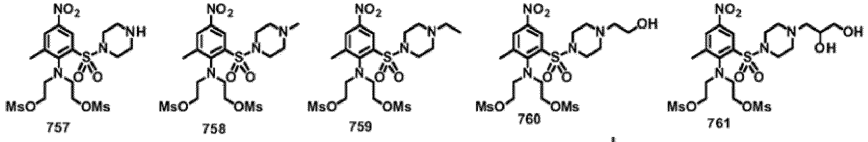

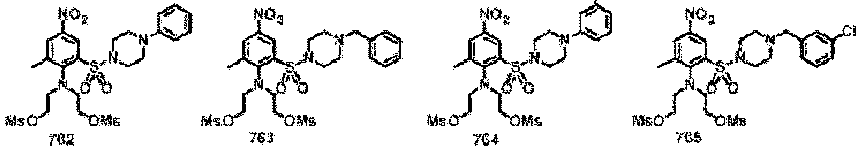

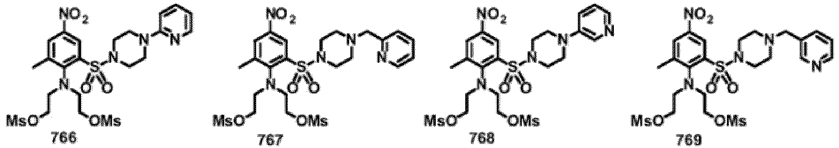

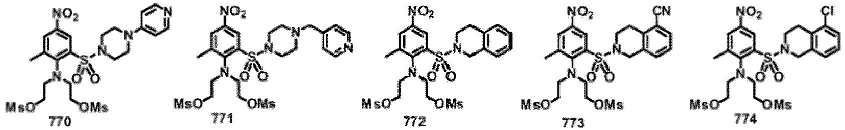

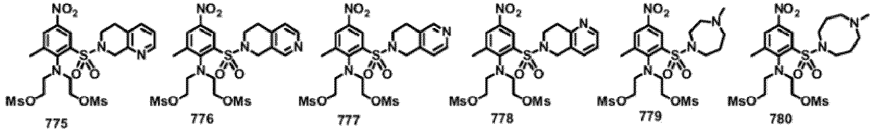

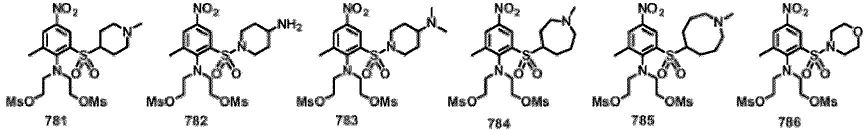

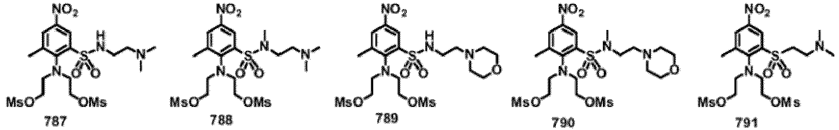

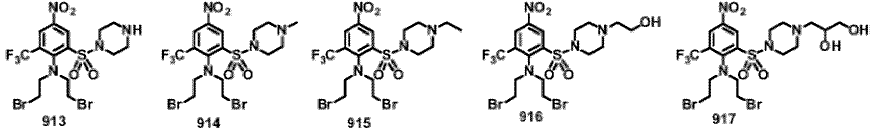

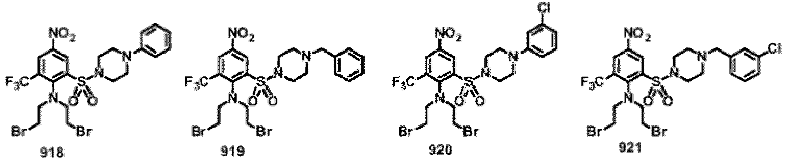

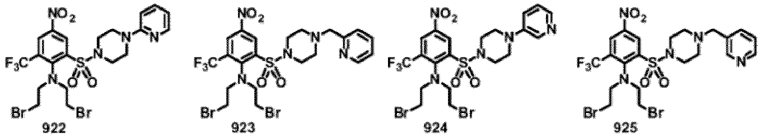

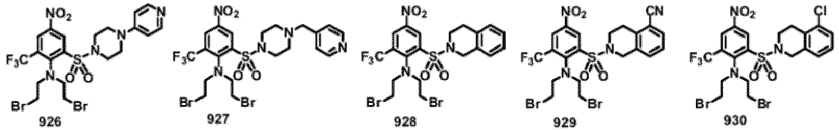

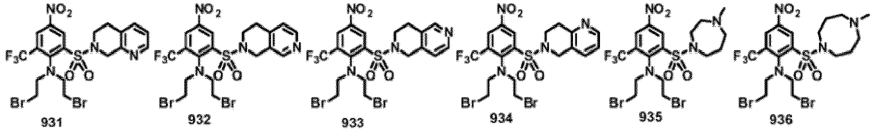

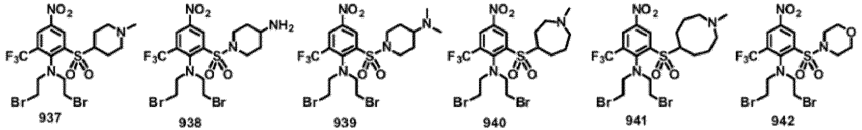

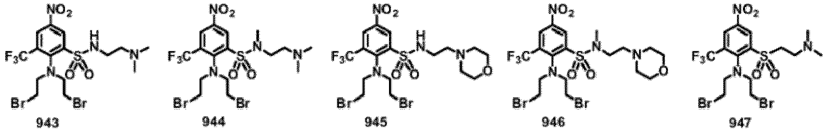

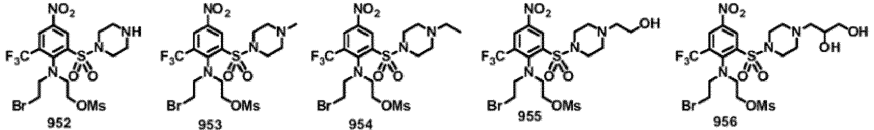

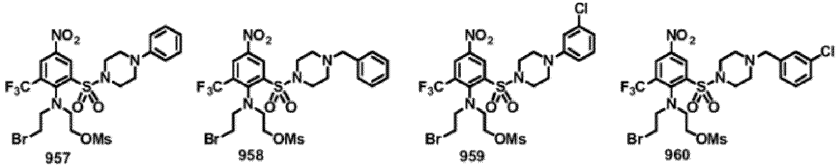

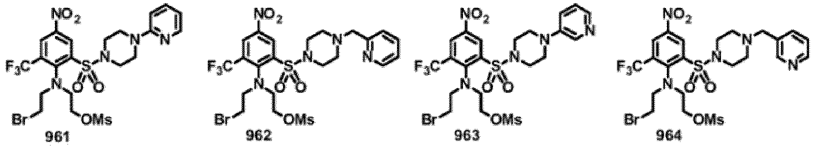

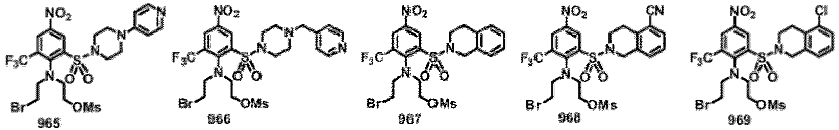

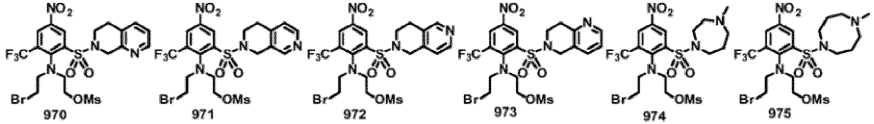

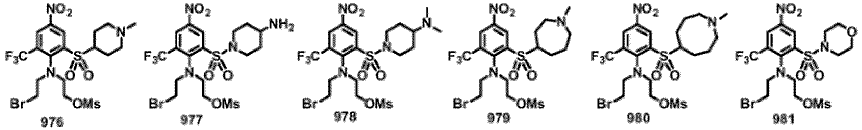

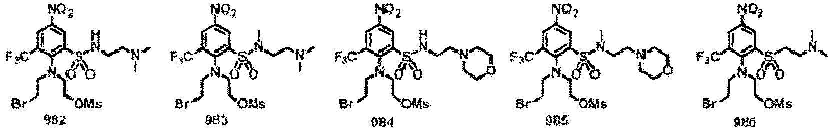

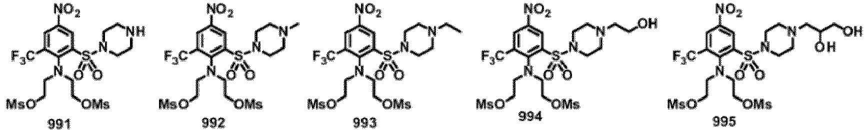

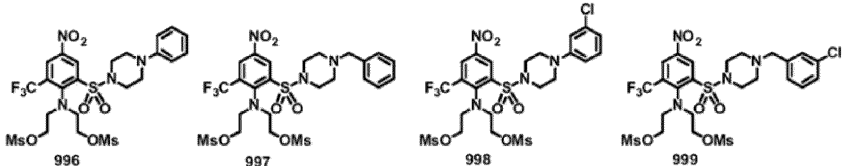

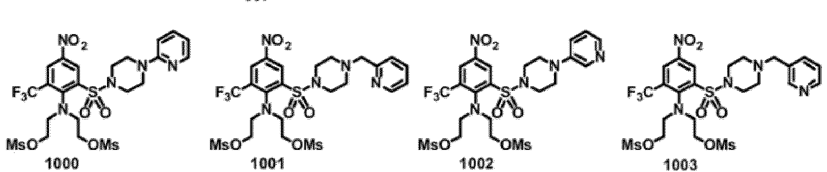

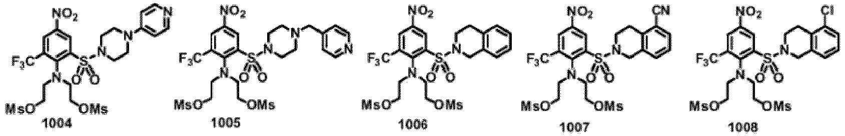

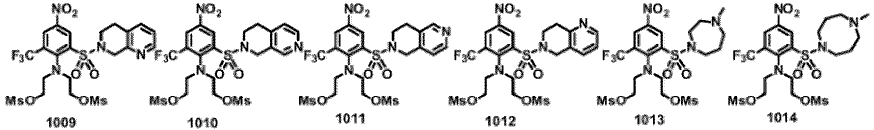

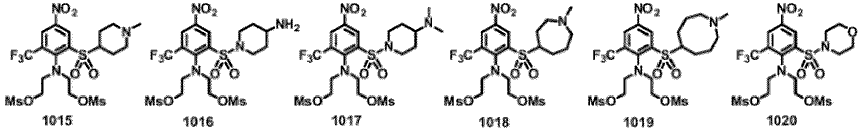

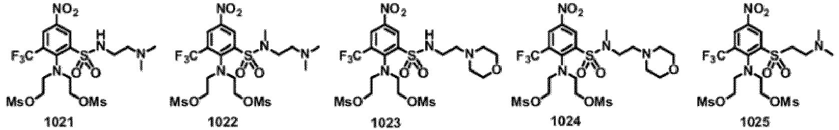

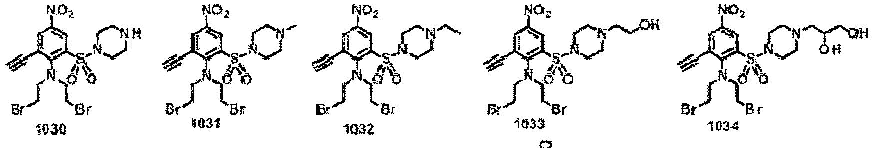

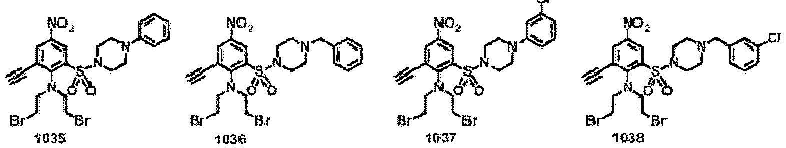

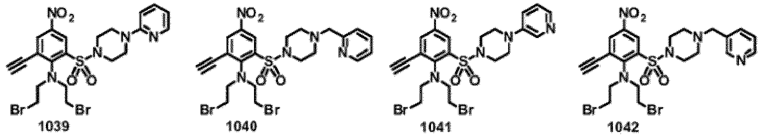

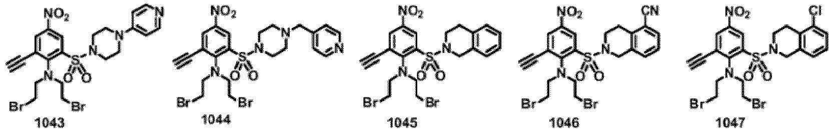

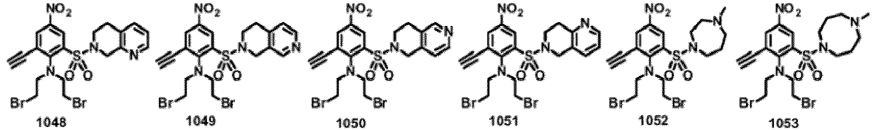

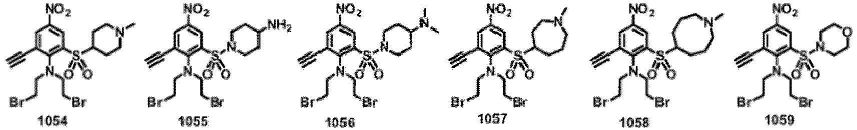

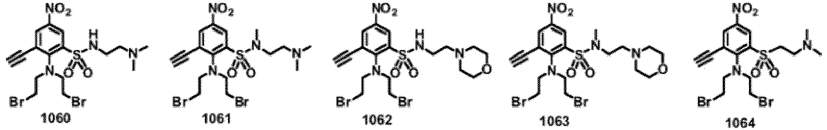

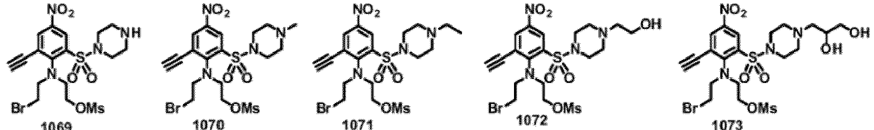

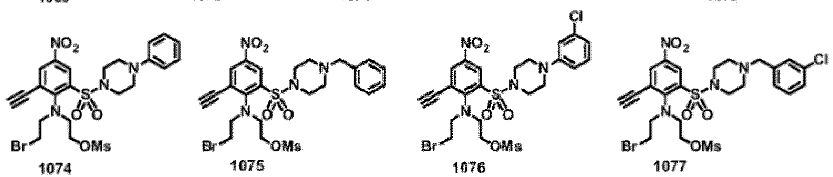

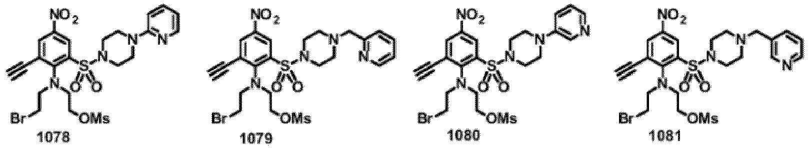

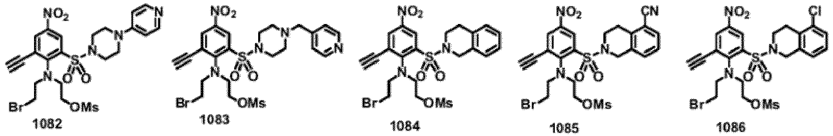

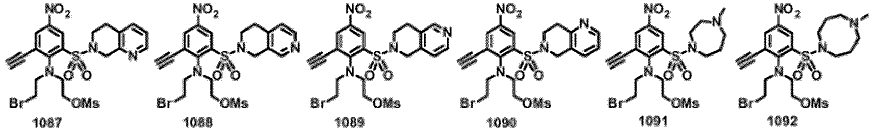

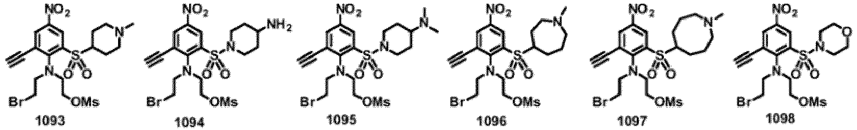

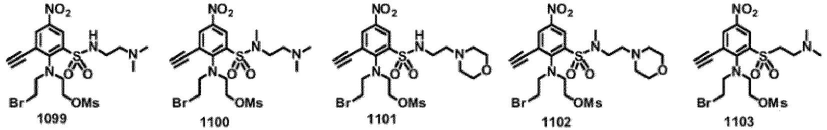

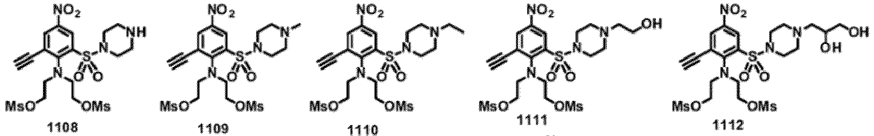

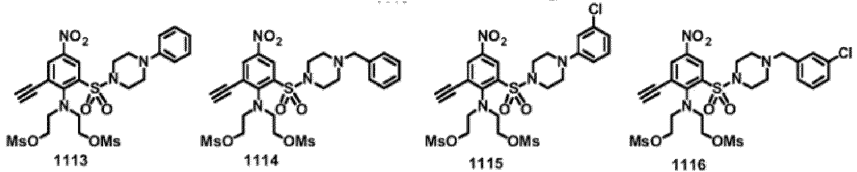

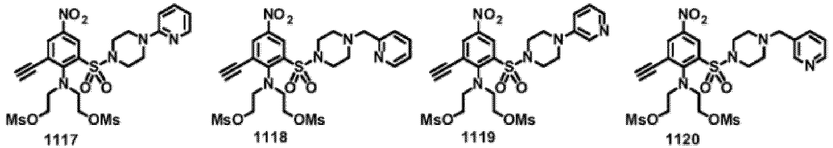

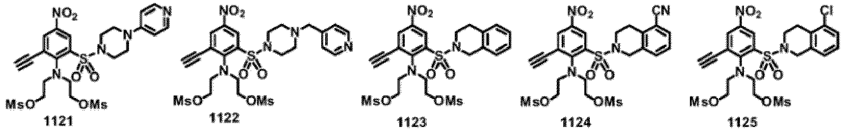

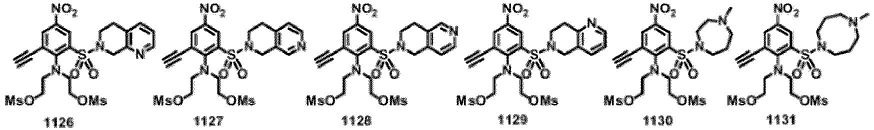

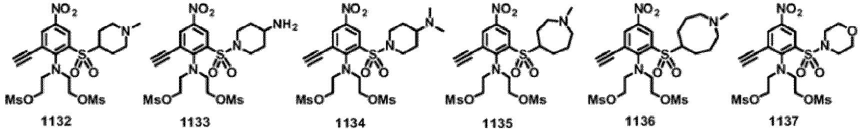

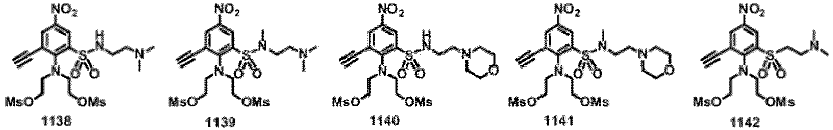

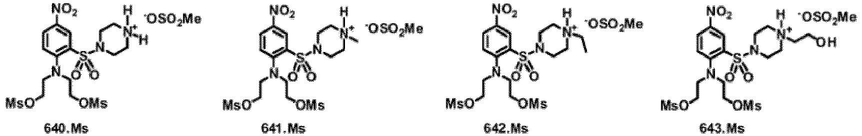

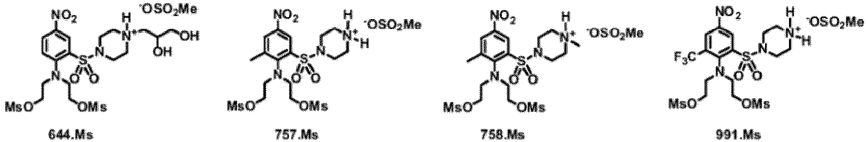

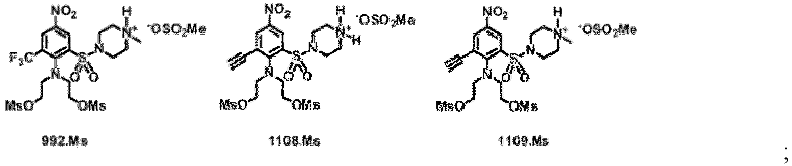

The AKR1C3 enzyme-activated prodrug compound of formula (10) is selected from the compounds of following structures:

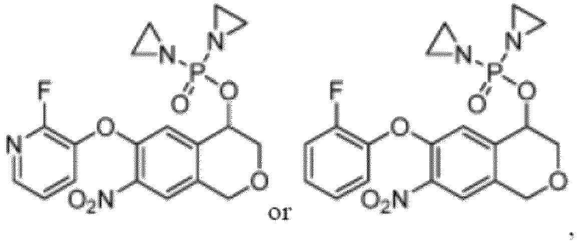

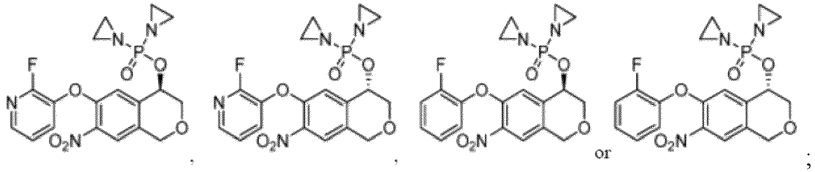

The AKR1C3 enzyme-activated prodrug compound of formula (11) is selected from the compounds of following structures:

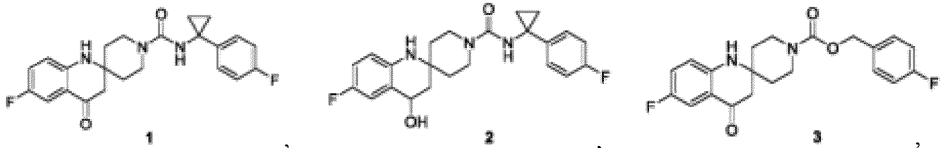

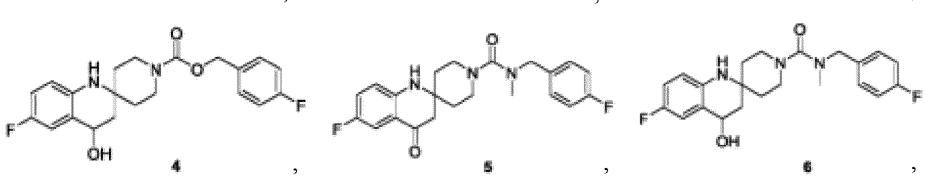

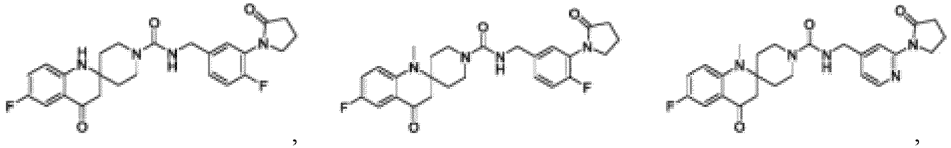

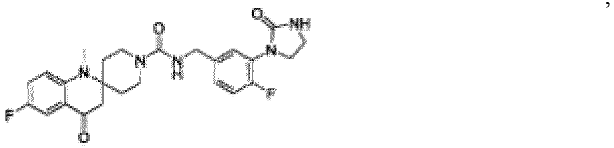

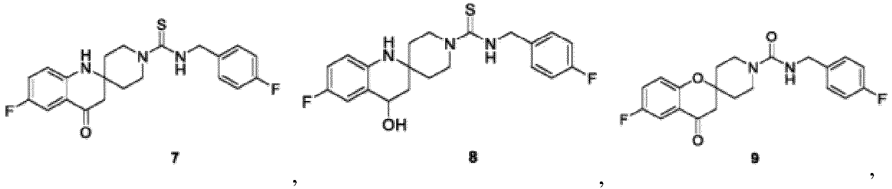

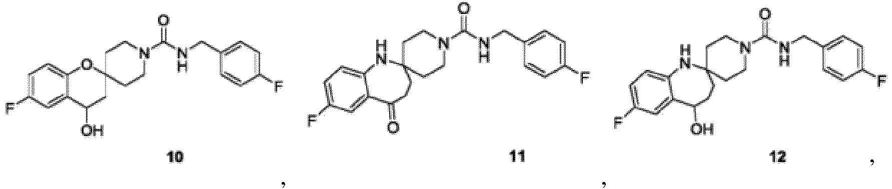

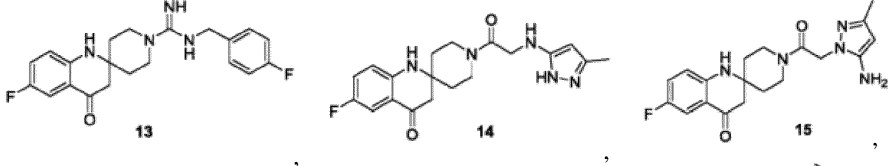

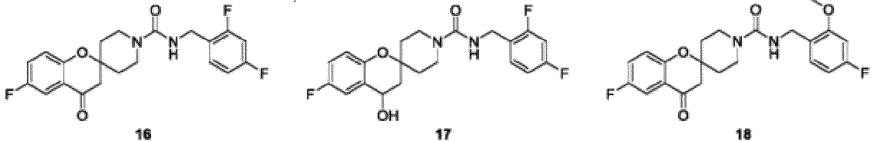

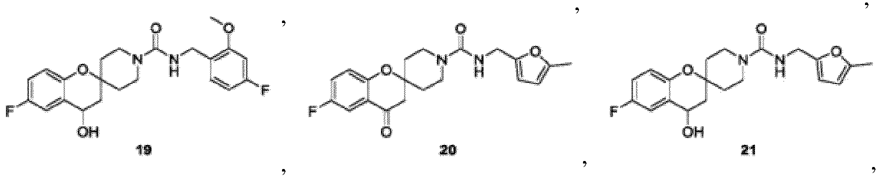

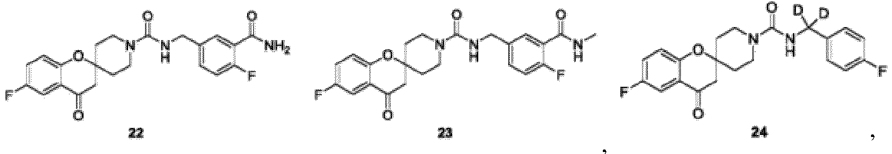

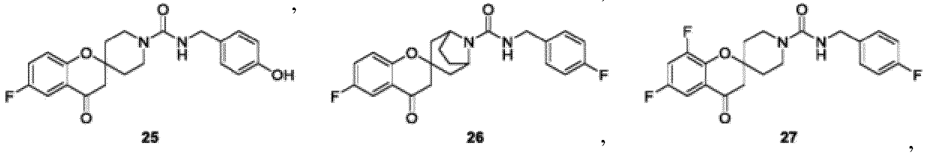

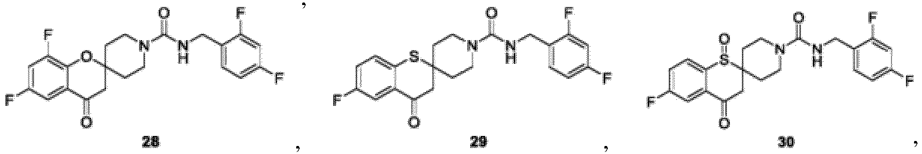

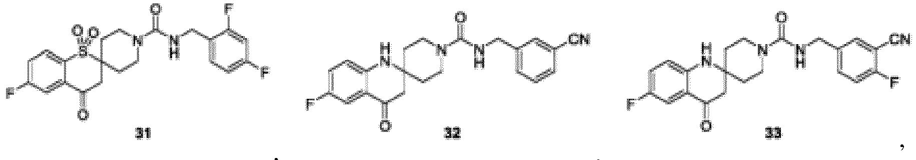

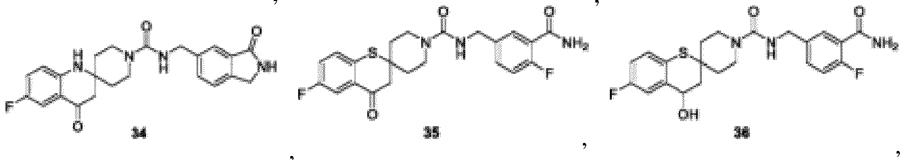

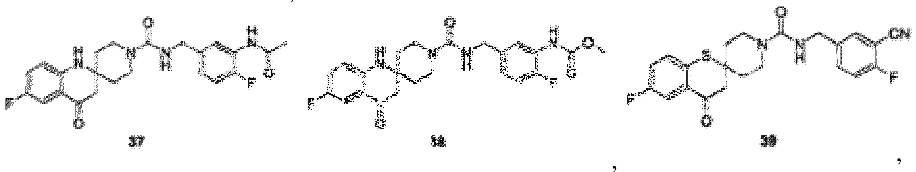

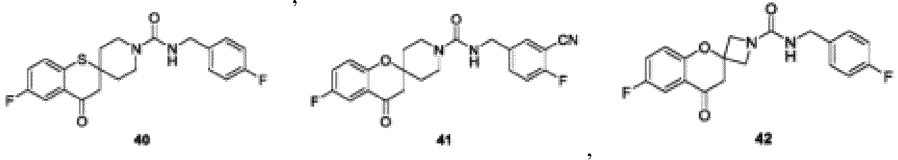

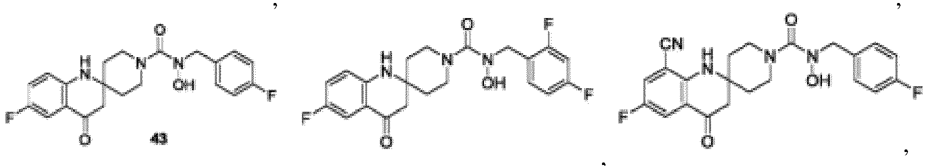

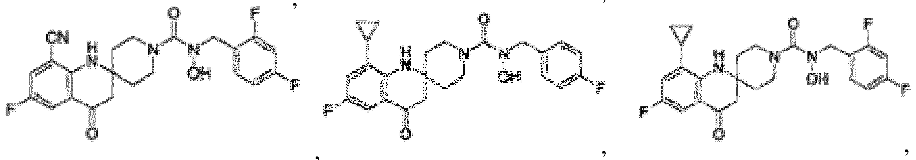

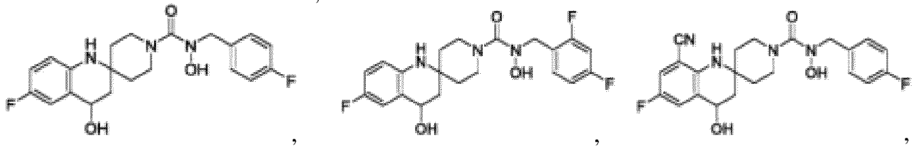

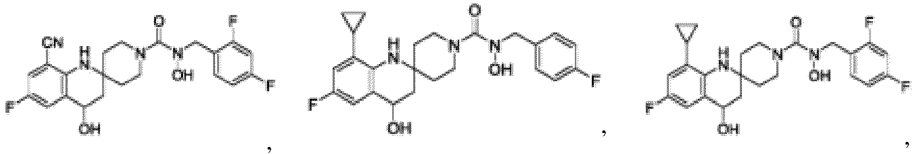

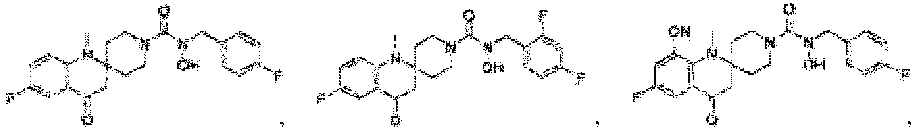

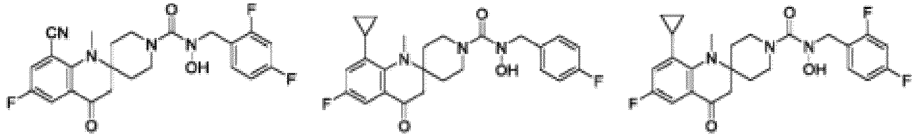

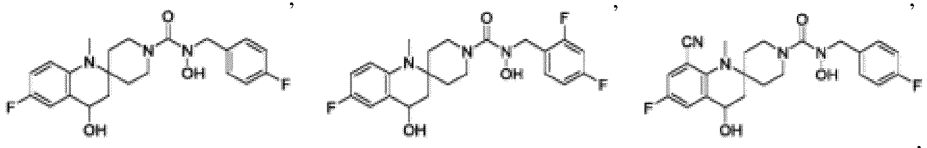

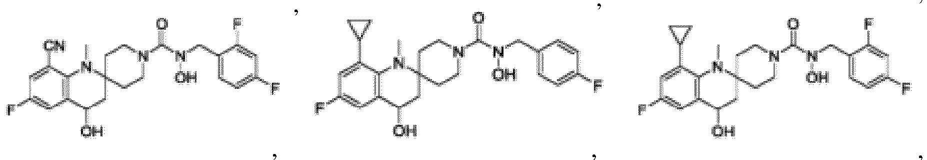

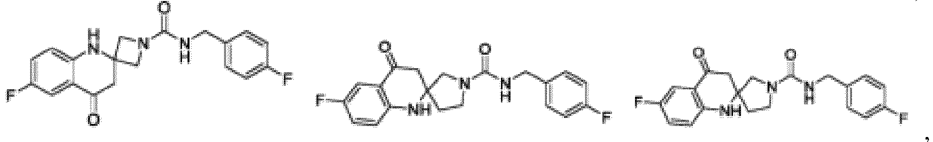

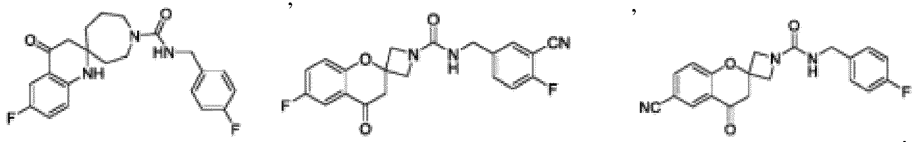

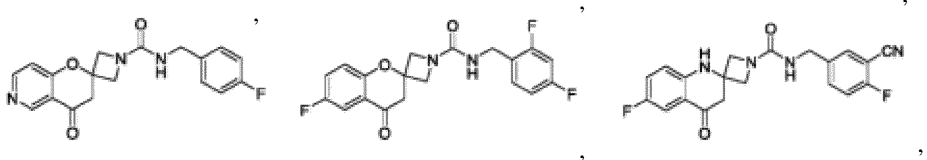

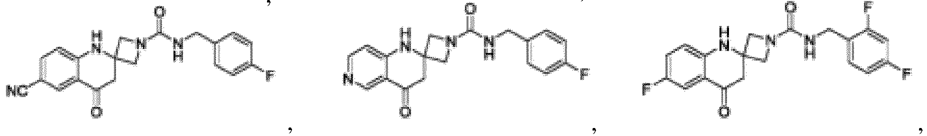

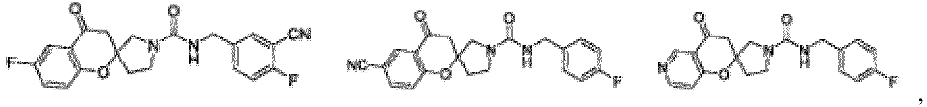

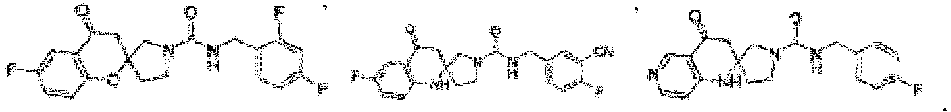

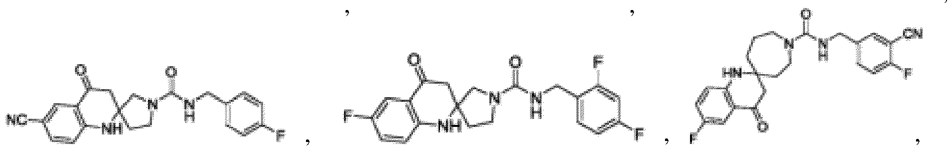

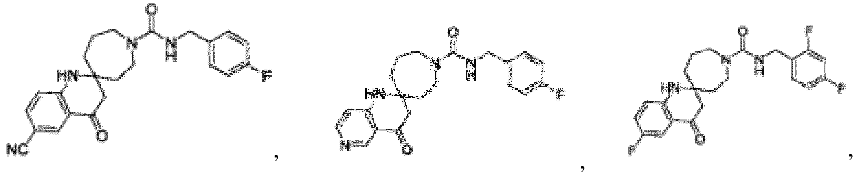

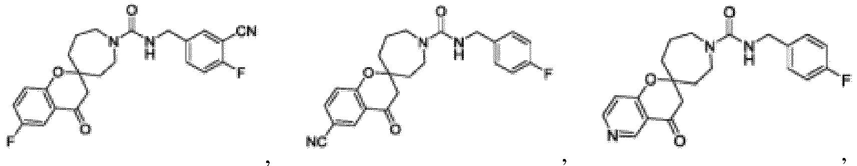

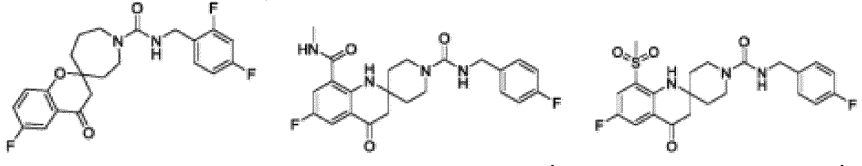

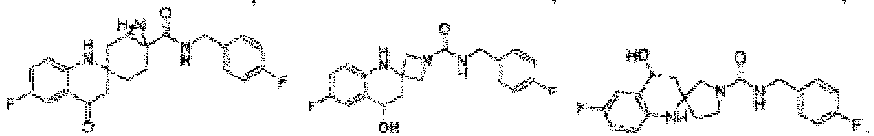

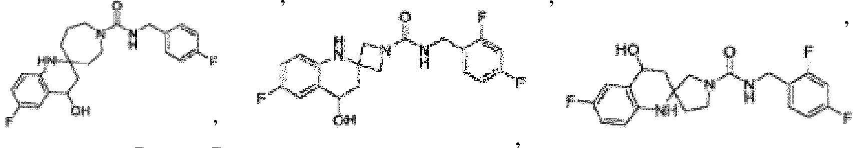

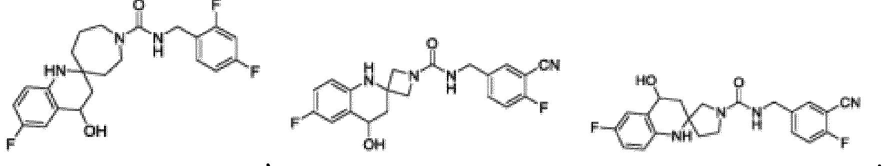

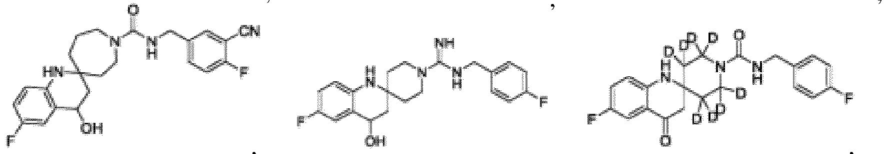

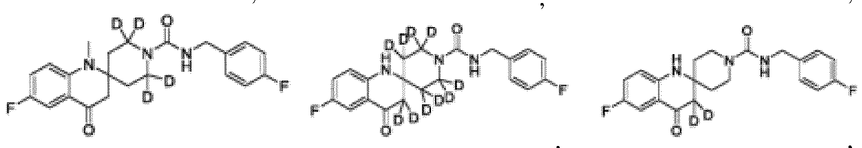

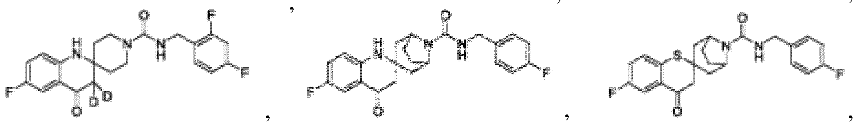

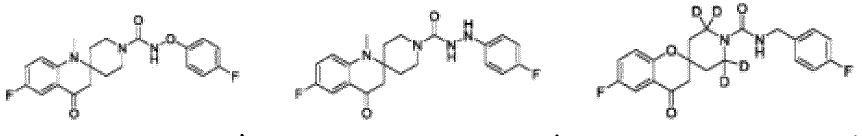

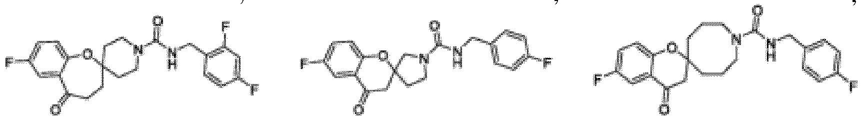

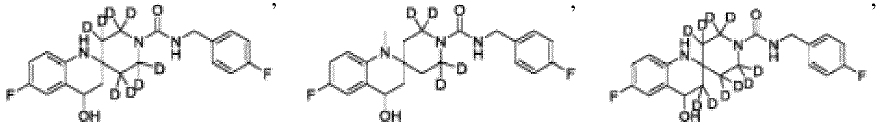

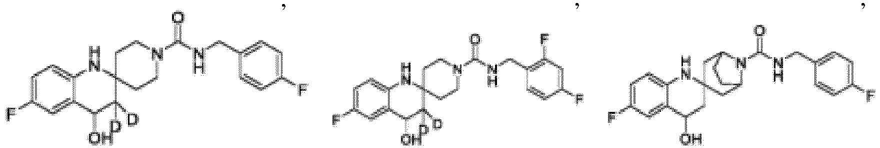

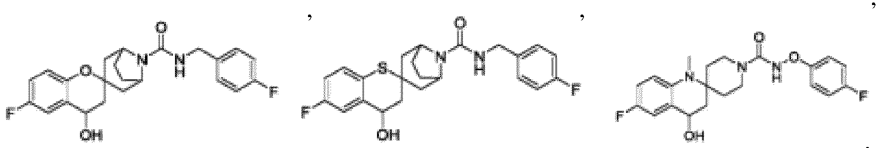

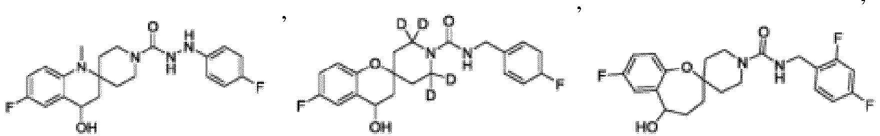

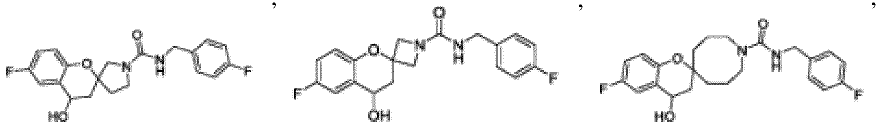

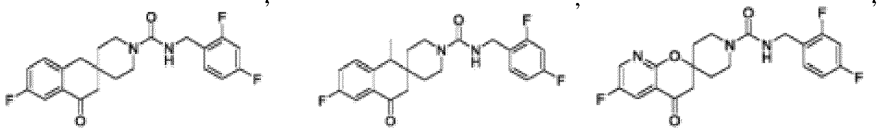

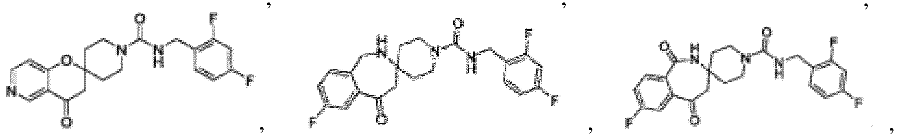

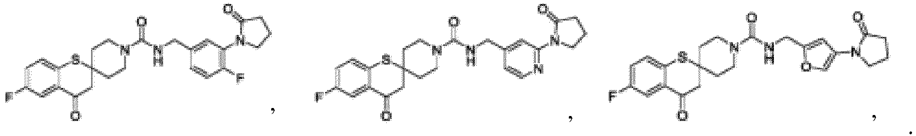

The AKR1C3 enzyme-activated prodrug compound of formula (12) is selected from the compounds of following structures:

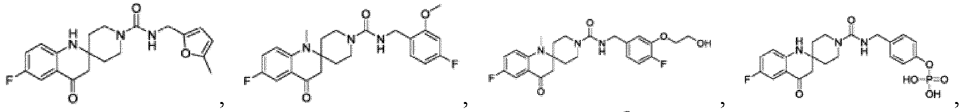

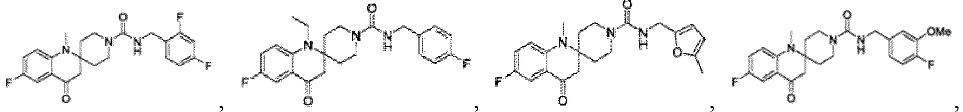

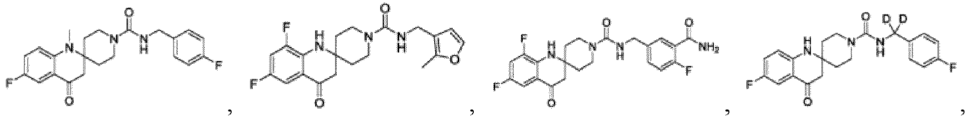

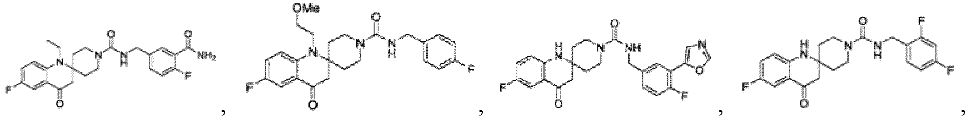

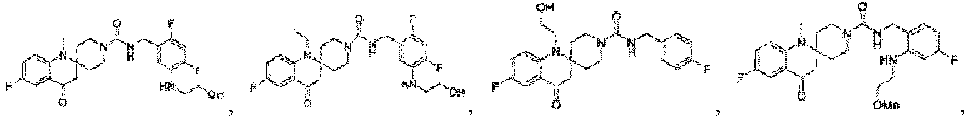

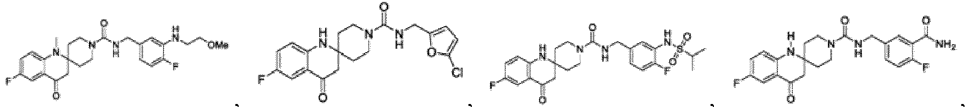

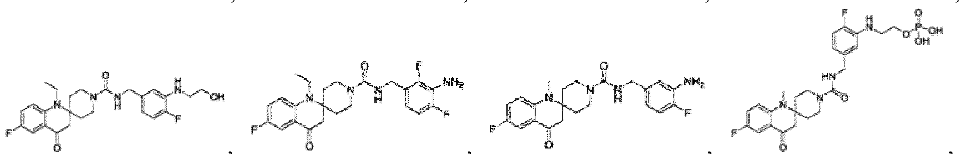

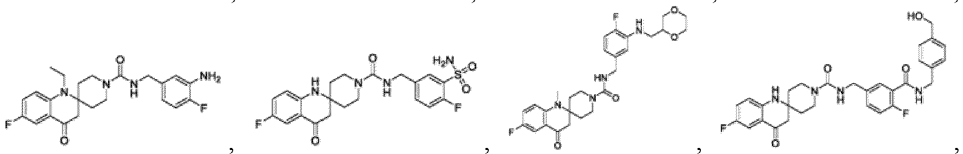

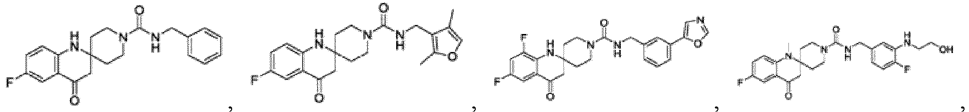

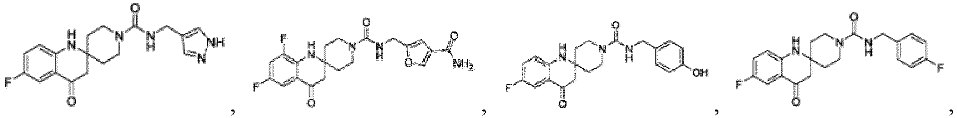

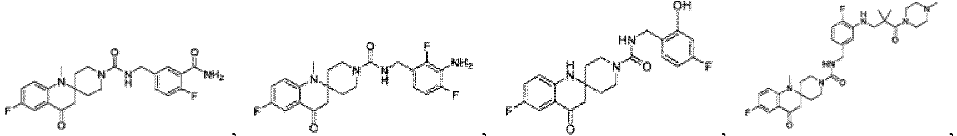

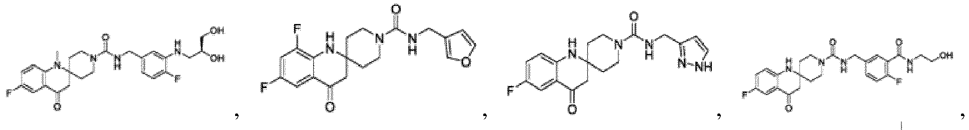

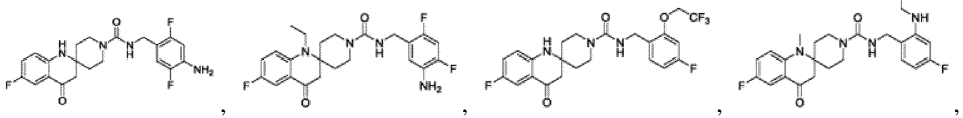

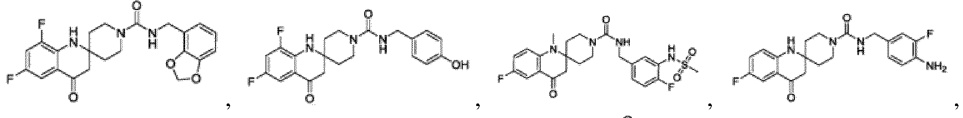

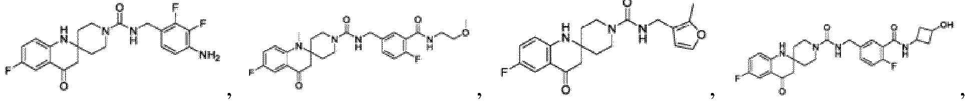

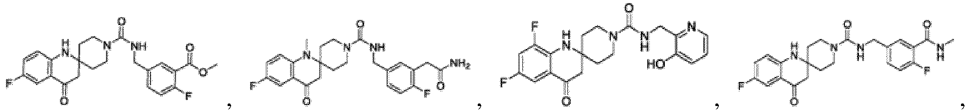

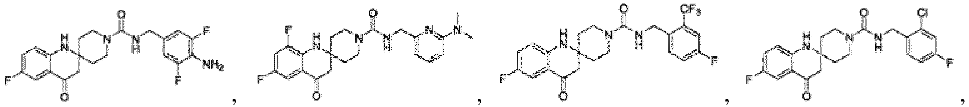

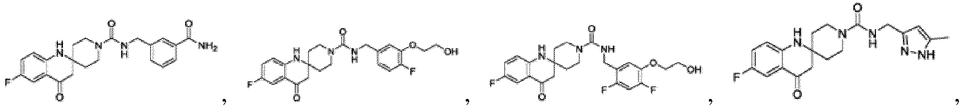

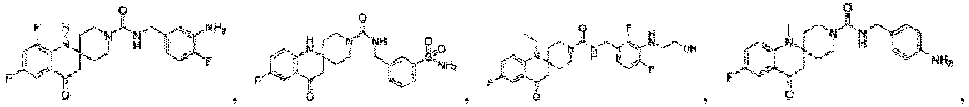

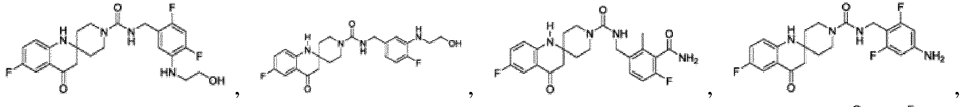

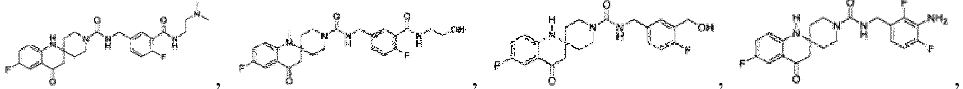

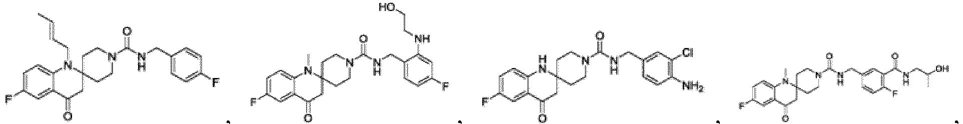

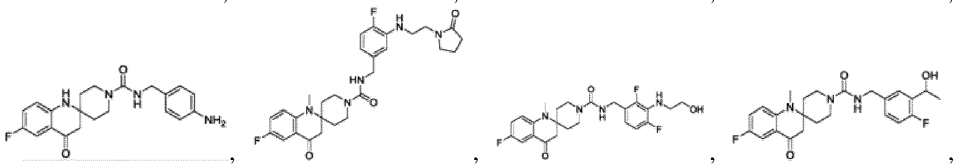

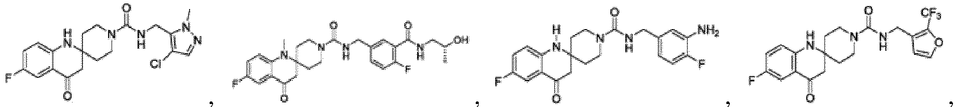

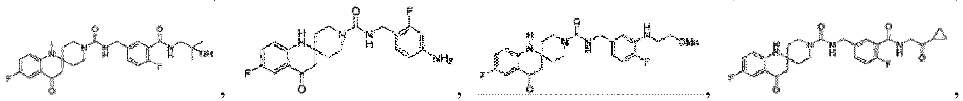

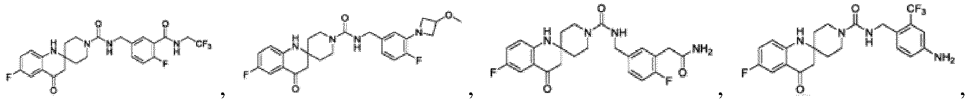

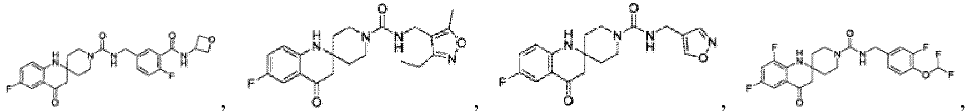

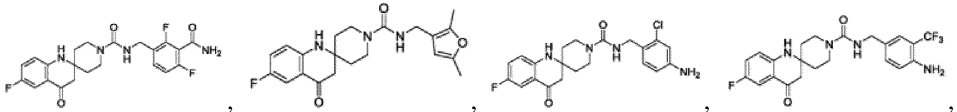

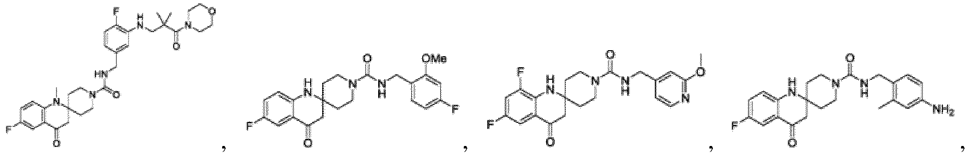

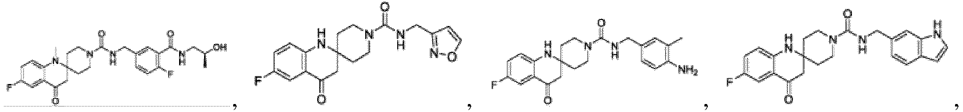

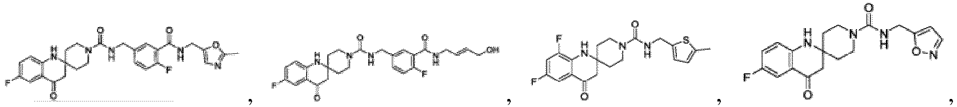

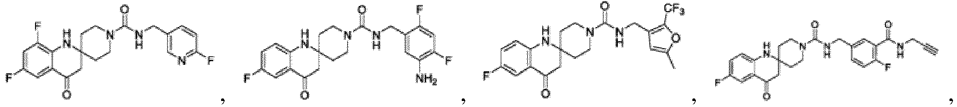

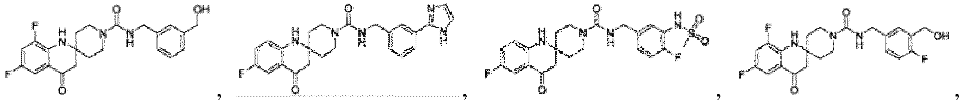

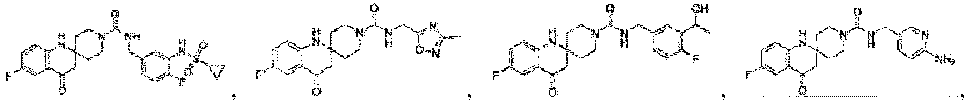

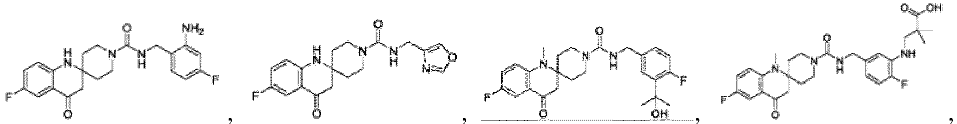

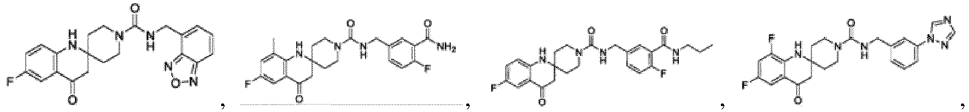

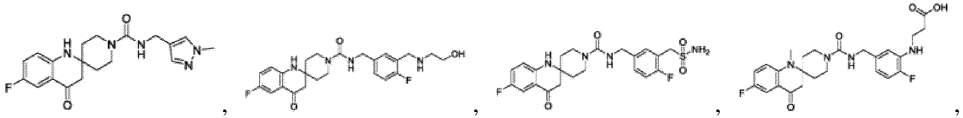

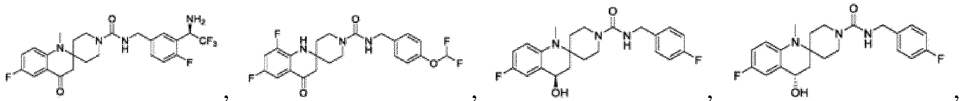

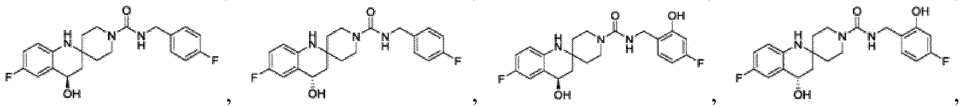

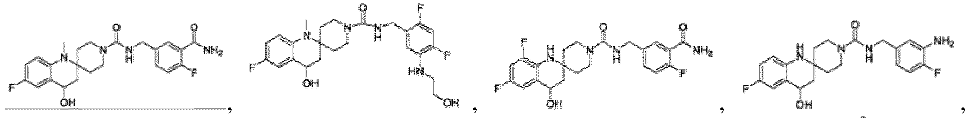

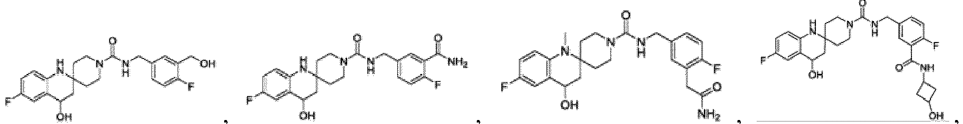

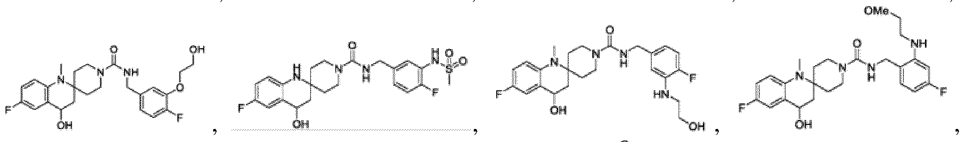

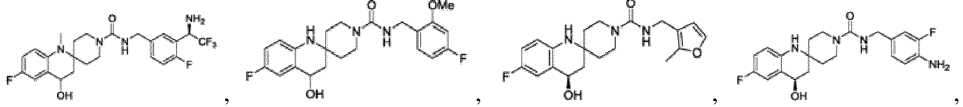

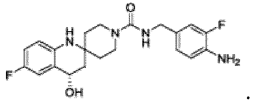

The synthesis preparation method of the specific compounds as described above may refer to the corresponding patent application documents of the specific general formula compounds (1) to (12).

### BRIEF DESCRIPTION OF THE DRAWINGS

FIG. 1 is the AKR1C3 expression levels results of Capan-1, PLC/PRF/5, HT29, and H460 cell lines measured by Western Blot assay.
FIG. 2 is the AKR1C3 expression levels results of Capan-1, HepG2, and H460 cell lines measured by Western Blot assay.
FIG. 3 is the growth curves of tumor volume in each group of mice in the *in vivo* pharmacodynamic experiments of the AST-3424 in the Capan-1 CDX model with BRCA pathogenic mutation.
FIG. 4 is the change curves of body weight in each group of mice in the *in vivo* pharmacodynamic experiments of the AST-3424 in the Capan-1 CDX model with BRCA pathogenic mutation.
FIG. 5 is the growth curves of tumor volume in each group of mice in the *in vivo* pharmacodynamic experiments of the AST-3424 in the non-BRCA pathogenic mutation model HepG2-GFP.
FIG. 6 is the change curves of body weight in each group of mice in the *in vivo* pharmacodynamic experiments of the AST-3424 in the non-BRCA pathogenic mutation model HepG2-GFP.
FIG. 7 is the growth curves of tumor volume in each group of mice in the *in vivo* pharmacodynamic experiments of the compound S in the BRCA pathogenic mutation model HuPrime^{®} gastric cancer GA6201.
FIG. 8 is the percentage change curves of body weight in each group of mice in the *in vivo* pharmacodynamic experiments of the compound S in the BRCA pathogenic mutation model HuPrime^{®} gastric cancer GA6201 during the period of treatment.
FIG. 9 is the growth curves of tumor volume in each group of mice in the *in vivo* pharmacodynamic experiment of the compound S in the non-BRCA pathogenic mutation model HuPrime^{®} lung cancer LU5173.
FIG. 10 is the percentage change curves of body weight in each group of mice in the *in vivo* pharmacodynamic experiments of the compound S in the non-BRCA pathogenic mutation model HuPrime^{®} lung cancer LU5173 during the period of treatment.
FIG. 11 is the growth curves of tumor volume in each group of mice in the *in vivo* pharmacodynamic experiments of the TH-302 in the BRCA pathogenic mutation model Capan-1.
FIG. 12 is the percentage change curves of body weight in each group of mice over the time of treatment in the *in vivo* pharmacodynamic experiments of the TH-302 in the BRCA pathogenic mutation model Capan-1.
FIG. 13 is the growth curves of tumor volume in each group of mice in the *in vivo* pharmacodynamic experiment of the TH-302 in the BRCA pathogenic mutation model HuPrime^{®} lung cancer LU6429.
FIG. 14 is the percentage change curves of body weight in each group of mice in the *in vivo* pharmacodynamic experiments of the TH-302 in the BRCA pathogenic mutation model HuPrime^{®} lung cancer LU6429 during the period of treatment.
FIG. 15 is the growth curves of tumor volume in each group of mice in the *in vivo* pharmacodynamic experiments of the TH-302 in the BRCA pathogenic mutation model HuPrime^{®} ovarian cancer OV5304.
FIG. 16 is the percentage change curves of body weight in each group of mice in the *in vivo* pharmacodynamic experiments of the TH-302 in the BRCA pathogenic mutation model HuPrime^{®} ovarian cancer OV5304 during the period of treatment.
FIG. 17 is the growth curves of tumor volume in each group of mice in the *in vivo* pharmacodynamic experiment of the TH-302 in the BRCA pathogenic mutation model HuPrime^{®} bladder cancer BL3325.
FIG. 18 is the percentage change curves of body weight in each group of mice in the *in vivo* pharmacodynamic experiments of the TH-302 in the BRCA pathogenic mutation model HuPrime^{®} bladder cancer BL3325 during the period of treatment.
FIG. 19 is the growth curves of tumor volume in each group of mice in the pharmacodynamic experiments of the TH-302 in the non-BRCA pathogenic mutation model human-derived ovarian adenocarcinoma SK-OV-3.
FIG. 20 is the change curves of body weight in each group of mice in the pharmacodynamic experiments of the TH-302 in the non-BRCA pathogenic mutation model human-derived ovarian adenocarcinoma SK-OV-3.
FIG. 21 is the growth curves of tumor volume in each group of mice in the pharmacodynamic experiment of the TH-302 in the non-BRCA pathogenic mutation model human-derived gastric cancer MKN45.
FIG. 22 is the change curves of body weight in each group of mice in the pharmacodynamic experiments of TH-302 in the non-BRCA pathogenic mutation model human-derived gastric cancer MKN45.
FIG. 23 is the growth curves of tumor volume in each group of mice in the pharmacodynamic experiments of the TH-302 in the non-BRCA pathogenic mutation model human-derived prostate cancer Vcap.
FIG. 24 is the change curves of body weight in each group of mice in the pharmacodynamic experiments of the TH-302 in the non-BRCA pathogenic mutation model human-derived prostate cancer Vcap.

### Detailed description of the invention

The present invention will be described below with reference to specific embodiments. Those skilled in the art will understand that these examples are only used to illustrate the present invention and do not limit the scope of the present invention in any way.

The experimental methods in the following examples are conventional methods unless otherwise specified. The medicinal raw materials, reagent materials, etc. used in the following examples are all commercially available products unless specified otherwise.

"Patient" and "individual" are used interchangeably and refer to a mammal in need of treatment for cancer. Typically, the patient is a human. Typically, the patient is a human diagnosed with cancer. In certain embodiments, a "patient" or "individual" may refer to a non-human mammal, such as a non-human primate, a dog, cat, rabbit, pig, mouse, or rats used for screening, characterizing, and evaluating drugs and therapies.

"Prodrug" refers to a compound that, after administration, is metabolized or otherwise converted to a biologically active or more active compound (or drug) with respect to at least one property. A prodrug, relative to the drug, is modified chemically in a manner that renders it, relative to the drug, less active or inactive, but the chemical modification is such that the corresponding drug is generated by metabolic or other biological processes after the prodrug is administered. A prodrug may have, relative to the active drug, altered metabolic stability or transport characteristics, fewer side effects or lower toxicity, or improved flavor. A prodrug may be synthesized using reactants other than the corresponding drug.

"Treatment" or "treatment of a patient" is to administer, use or apply a therapeutic effective amount of a drug to a patient in relation to the present invention.

"Administering" or "administration of" or "use of" a drug to a patient refers to direct administration, which may be administered to a patient by a medical professional or may be self-administered, and/or indirect administration, which may be the act of prescribing a drug. For example, a physician who instructs a patient to self-administer a drug and/or provides a patient with a prescription for a drug is administering the drug to the patient.

"Therapeutically effective amount" of a drug refers to an amount of a drug that, when administered to a patient with cancer, will have the intended therapeutic effect (e.g., alleviation, amelioration, palliation, or elimination of one or more clinical manifestations of cancer in the patient). A therapeutic effect does not necessarily occur by administration of one dose, and may occur only after administration of a series of doses. Thus, a therapeutically effective amount may be administered in one or more administrations.

"Treating", "treatment of" or "therapy of" a condition or a patient refers to taking steps to obtain a beneficial or desired result (including clinical results). For purposes of the present invention, beneficial or desired clinical results include, but are not limited to, alleviation or amelioration of one or more symptoms of cancer; diminishment of extent of disease; delay or slowing of disease progression; amelioration, remission, or stabilization of disease status; or other beneficial results. In some cases, treatment of cancer can result in partial response or stable disease.

"Tumor cells" refers to tumor cells of any appropriate species, e.g., mammalian such as murine, canine, feline, equine or human.

The above description of embodiments of the present invention does not limit the present invention. Those skilled in the art can make various modifications and changes according to the present invention, and any modification and change within the spirit of the present invention shall be covered in the scope of the claims appended to the present invention.

Specific experiments of the present invention are provided below to illustrate the effects of BRCA pathogenic mutation on the *in vitro* and *in vivo* pharmacodynamics of AST-3424, AST-3424 analogous compounds S and TH-302.

### AST-3424:

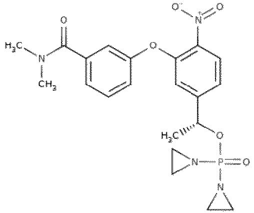

### AST-3424 analogous compound S:

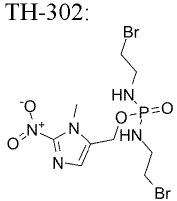

### Example 1

The effects of BRCA pathogenic mutation on the *in vitro* pharmacodynamics of AST-3424, AST-3424 analogous compounds S and TH-302 are illustrated by the experiments below.

### 1.1 Comparison of in vitro cytotoxicity of compound AST-3424 in BRCA non-pathogenic mutant and pathogenic mutant tumor cells

*In vitro* cytotoxicity of human-derived tumor cell lines (IC₅₀ Value) was used in the study to compare whether compound AST-3424 in BRCA non-pathogenic mutant and pathogenic mutant tumor cells had an obvious difference, so as to detect whether pathogenic mutation of BRCA enhanced the sensitivity of tumor cells to AST-3424 at the cellular level *in vitro.* The following cell lines were used as research objects in this experiment, which were BRCA pathogenic mutant cell line Capan-1 (human-derived pancreatic cancer cell line, wherein the BRCA2 pathogenic mutation site was p.S1982RfsTer22), BRCA non-pathogenic mutant cell line PLC/PRF/5 (human-derived liver cancer cell line) and HT29 (human-derived colon cancer cell line), respectively.

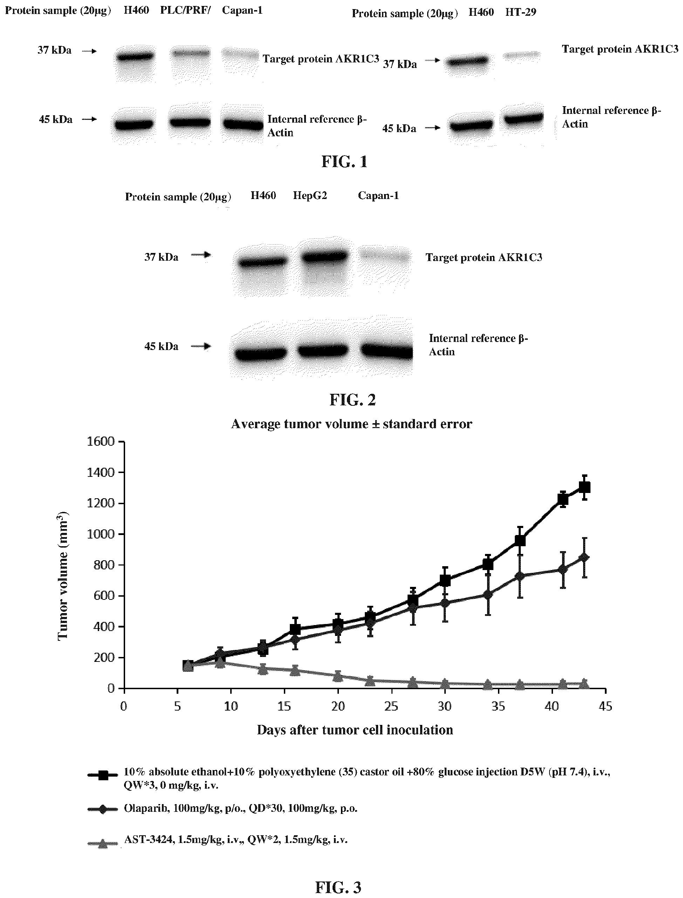

Both the activation mechanism of the prodrug AST-3424 and AST-3424 analogous compound S were to release DNA alkylating agent through the action of AKR1C3 enzyme, thereby achieving the effect of inhibiting and killing tumor cells. Therefore, in all presence or all absence of pathogenic gene mutation, the higher the expression level of AKR1C3 enzyme, the stronger the expected cytotoxicity of the compound to tumor cells, and the lower IC₅₀ value. We chose to detect the effect of BRCA pathogenic mutations of the cells on the sensitivity of AST-3424 and AST-3424 analogous compound S in the case that the expression level of AKR1C3 protein of each cell line was similar or the expression level of AKR1C3 enzyme protein of BRCA pathogenic mutant cell lines was lower than that of non-BRCA pathogenic mutant cell lines. We used Western Blot assay to identify and quantitatively analyze the expression levels of AKR1C3 protein in the above three cell lines. At the same time, human-derived lung cancer cell line H460 with high expression of AKR1C3 protein was used as a control to compare the expression levels of AKR1C3 protein in three cell lines. The results showed that the expression levels of AKR1C3 protein of Capan-1 and HT29 cell lines were close, and the expression level of AKR1C3 protein of PLC/PRF/5 cell line was slightly higher (Table 1, FiG. 1).

**Table 1: The expression levels of AKR1C3 protein of each cell line as compared with H460 cell line**

| cell lines | % AKR1C3 protein expression in H460 cell |
|---|---|
| Capan-1 | 19.45% |
| PLC/PRF/5 | 44.39% |
| HT29 | 13.76% |
| H460 | 100% |

Cell proliferation assay was used to detect the sensitivity of various cells to AST-3424, i.e., IC₅₀ value. Unless otherwise specified, IC₅₀ value was measured by the following experimental method:
(1) Cell culture
a) Different cells were cultured in different media with fetal bovine serum (Table 2), added with 1% double antibody, and placed at 37 °C and 5% CO₂ for culturing.

**Table 2: Culture conditions of each cell**

| cell lines | Fetal bovine serum ratio | Medium | Number of plated cells/well |
|---|---|---|---|
| Capan-1 | 20% | IMDM | 60000 |
| PLC/PRF/5 | 10% | MEM | 10000 |
| HT29 | 10% | McCoy's 5A | 10000 |

(2) Cell plating
a) The cells were routinely cultured until the cell saturation was 80%-90%, and when the required quantity was reached, the cells were collected.
b) The cells were resuspended with the corresponding medium and cell counts were performed to prepare a cell suspension with appropriate density.
c) The cell suspension was added to a 96-well plate with 100µL per well, and the cell density was shown in Table 2.
d) Cells were incubated overnight at 37 °C, 5% CO₂ in an incubator.

(3) Preparation of the compound
a) The compound was prepared according to the experimental requirements.
b) Blank control well was cells added with 0.5% DMSO as high read control well.
c) The well only with medium and without cell was used as low read control well.

(4) Treating cells with compound
a) After 24 hours of cell plating, the compound was used alone and each well was supplied with 99µL of growth medium, then added with 1µL of 200X compound, shaken gently to ensure even mixing, and then placed at 37 °C, 5% CO₂ in an incubator.
b) The cell plate was placed in the incubator for 72 hours.

(5) Detecting with CTG method
a) The cell plate to be tested was placed at room temperature to equilibrium for 30 minutes, and 100µL of medium was discarded per well.
b) 100µL of CTG reagent (CelltiterGlo kit) was added to each well, placed at a fast shaker to shake for 2 minutes, and placed at room temperature away from light for 30 minutes.
c) The chemiluminescence signal value was read with the Envision instrument.

(6) Data analysis
IC₅₀ was calculated with GraphPad Prism 8 software, and the IC₅₀ (half maximal inhibitory concentration) of the compound was obtained by using the following nonlinear fitting formula: Y = Bottom + (Top-Bottom)/(1 + 10 ^ ((LogIC)₅₀-X) * HillSlope))
X: Compound concentration log value, Y: inhibition rate (% inhibition) inhibition rate (% inhibition) = (high-read control read - compound well read)/(high-read control read - low-read control read) × 100

The IC₅₀ values of compound AST-3424 to be tested in different cells measured by the above experimental method were listed in Table 3 below.

**Table 3: IC₅₀ data of the inhibitory effect of compound AST-3424 on the three tumor cells**

| Compound | Cell lines | IC₅₀ (nmol/L) | Fold Change |
|---|---|---|---|
| AST-3424 | Capan-1 | 8.33 | / |
| | PLC/PRF/5 | 186.60 | 22 |
| | HT29 | 559.00 | 67 |

The test results showed that compared with the BRCA pathogenic mutant cell line Capan-1, the IC₅₀ of AST-3424 in the BRCA non-pathogenic mutant cell line HT29 was 67 times that of Capan-1 cell line. Therefore, there was an obvious difference between the IC₅₀ value of AST-3424 in the BRCA pathogenic mutant cell line Capan-1 with similar AKR1C3 protein expression and the IC₅₀ value of the BRCA non-pathogenic mutant cell line HT29. It showed that tumor cells with BRCA pathogenic mutation were more sensitive to AST-3424 under the condition of similar AKR1C3 protein expression. Even though the expression level of AKR1C3 protein of BRCA pathogenic mutant cell line Capan-1 was lower than that of BRCA non-pathogenic mutant cell line PLC/PRF/5, the IC₅₀ of AST-3424 in BRCA non-pathogenic mutant cell line PLC/PRF/5 was still 22 times that of the Capan-1 cell line. Therefore, tumor cells with BRCA pathogenic mutations were more sensitive to AST-3424.

### 1.2 Comparison of in vitro cytotoxicity of AST-3424 analogous compound S in BRCA non-pathogenic mutant and pathogenic mutant tumor cells

The same experimental methods and cell lines as described above were used in the study to further test whether Compound S in BRCA non-pathogenic mutant and pathogenic mutant tumor cells had an obvious difference, so as to detect whether pathogenic mutation of BRCA enhanced the sensitivity of tumor cells to Compound S. The *in vitro* cytotoxicity results of Compound S on the above three cells were listed in Table 4 below.

**Table 4: IC₅₀ data of the inhibitory effect of Compound S on the three tumor cells**

| Compound | Cell lines | IC₅₀ (nmol/L) | Fold Change |
|---|---|---|---|
| **AST-3424 analogous Compound S** | Capan-1 | 403.40 | / |
| | PLC/PRF/5 | 2015.00 | 5 |
| | HT29 | 4883.00 | 12 |

The test results showed that compared with the BRCA pathogenic mutant cell line Capan-1, the IC₅₀ of AST-3424 analogous Compound S in the BRCA non-pathogenic mutant cell line HT29 was 12 times that of Capan-1 cell line. Therefore, there was also an obvious difference between the IC₅₀ value of AST-3424 analogous Compound S in the BRCA pathogenic mutant cell line Capan-1 with similar AKR1C3 protein expression and the IC₅₀ value of the BRCA non-pathogenic mutant cell line HT29. Even though the expression level of AKR1C3 protein of BRCA pathogenic mutant cell line Capan-1 was lower than that of BRCA non-pathogenic mutant cell line PLC/PRF/5, the IC₅₀ of AST-3424 analogous Compound S in BRCA non-pathogenic mutant cell line PLC/PRF/5 was still 5 times that of the Capan-1 cell line. Therefore, tumor cells with BRCA pathogenic mutation model were more sensitive to AST-3424 analogous Compound S.

Both the above two experimental results showed that under the condition of similar AKR1C3 protein expression, AST-3424 and AST-3424 analogous Compound S had more obvious *in vitro* killing effects on tumors with BRCA pathogenic mutation model. Even though the expression level of AKR1C3 protein of BRCA pathogenic mutant cell line was lower than that of BRCA non-pathogenic mutant cell line, the *in vitro* killing effects of AST-3424 and AST-3424 analogous Compound S on tumors with BRCA pathogenic mutation model were more obvious.

### 1.3 Comparison of in vitro cytotoxicity of compound TH-302 in BRCA knockdown and wild type tumor cells

TH-302 is a small molecule prodrug that can be activated under hypoxic conditions to release cytotoxin, thereby killing tumor cells and tumor tissues. In order to evaluate the correlation between TH-302 and BRCA pathogenic mutations at the cellular level *in vitro,* we selected human-derived colon cancer cell line DLD1 and DLD1-BRCA2-/-tumor cell line with BRCA2 protein knockout, and detected whether the killing effects of TH-302 to the above two tumor cell lines with or without BRCA2 protein expression under hypoxic conditions had a difference.

The IC₉₀ (90% inhibitory concentration) value was used to evaluate the ability of the killing effect of compound TH-302 to the cells in this colony formation assay. The specific experimental method was as follows:
(1) Cell culture
   a) DLD1 and DLD1-BRCA2-/-cells were cultured in RPMI medium, added with 10% FBS and 1% double antibody, and placed at 37°C and 5% CO₂ for culturing.
(2) Cell plating
   a) The cells were routinely cultured until the cell saturation was 80%-90%, and when the required quantity was reached, the cells were collected.
   b) The cells were resuspended with the corresponding medium and cell counts were performed to prepare a cell suspension with appropriate density.
   c) The cell suspension was added to 10 cm glass dish, the cell density of DLD1-BRCA2^{-/-} was 3.0 × 10⁵/dish and the cell density of DLD1 was 300/dish.
   d) Cells were incubated for 2 days at 37 °C, 5% CO₂ in an incubator.
(3) Preparation of the compound
   The compound was prepared according to the experimental requirements.
(4) Treating cells with compound
   A) The cells were treated with the compound for 3 h under the condition of oxygen content < 0.01% (hypoxia).
   b) The cells were washed once with 1 × PBS and were digested with trypsin, and then cell counts were performed.
   c) Cells were resuspended with 3 mL of medium and inoculated in a 6-well culture plate, wherein the cell density of DLD1-BRCA2-/- was 2000/well and the cell density of DLD1 was 300/well.
   d) The cell plate was placed in the incubator for 10 days.
   e) The medium was discarded and stained with crystal violet for 40 min after the cells were fixed.
   f) Cell clone counts were performed using Colony Counter (VWR) and IC₉₀ values were calculated according to CalcuSyn software (http://www.biosoft.com/w/calcusyn.htm).

The IC₉₀ values of compound TH-302 to be tested in the two cells measured by the above experimental method were listed in Table 5 below.

**Table 5: IC₉₀ data of the inhibitory effect of compound TH-302 on the two tumor cells under hypoxic conditions**

| Compound | Cell lines | IC₉₀ (nmol/L) | Fold Change |
|---|---|---|---|
| TH-302 | DLD1 | 210 | / |
| | DLD1-BRCA2^{-/-} | 3.01 | 70 |

The experimental results showed that after compound TH-302 was activated by hypoxia, there was significantly difference between the *in vitro* cytotoxicity of compound TH-302 on DLD1 BRCA2 wild type and DLD1-BRCA2^{-/-} cell with the deletion of BRCA2. Compared with DLD1-BRCA2^{-/-} cell, the *in vitro* IC₉₀ value of BRCA2 wild-type DLD1 was increased by 70-fold. The above results showed that under hypoxic conditions, the deletion of BRCA2 protein led to a significantly enhancement of the sensitivity level of DLD1 cell to TH-302.

### Example 2

**The effects of BRCA pathogenic mutation on the *in vivo* pharmacodynamics of AST-3424, AST-3424 analogous Compounds S and TH-302 were illustrated by the *in vivo* animal experiments below.**

If not specified, the relevant parameters in the following chart were defined as follows:
1. Data were expressed as "mean ± standard error", i.e., *x̅*±S;
2. T/C% = T_{RTV}/C_{RTV}× 100% or T/C% = T_{TV}/C_{TV}× 100% (T_{RTV}: mean RTV of treatment group; C_{RTV}: mean RTV of vehicle control group; RTV = Vₜ/V₀, V₀ was the tumor volume of the animal at the time of grouping, Vₜ was the tumor volume of the animal after treatment, the same below); TGI% = (1-T/C) × 100% (T and C were the mean relative tumor volumes (RTV) of the treatment and control groups at a specific time point, respectively).
3. CR ratio (complete clearance) represented the ratio of the number of animals with complete tumor clearance to the number of animals in the group corresponding to the drug dose group.
4. P Value, i.e., the statistical P measurement value. P < 0.05 was defined as a significant difference.

According to the method described in the literature (Meng, F., Li, W. F., Jung, D., Wang, C. C., Qi, T., Shia, C. S., Hsu, R. Y, Hsieh, Y. C., & Duan, J. (2021). A novel selective AKR1C3-activated product AST-3424/OBI-3424 exhibits broad anti-tumor activity. American journal of cancer research, 11 (7), 3645-3659.), the RNA expression level of AKR1C3 in the above gastric cancer GA6021, pancreatic cancer PA1222, and lung cancer LU11693 tissues was analyzed using RNA-Seq (AKR1C3 RNA expression level and quantified using Log2 FPKM).

### 2.1 Comparison of in vivo pharmacodynamics of compound AST-3424 in BRCA non-pathogenic mutant and pathogenic mutant tumor models

In order to verify whether there was a difference in the *in vivo* pharmacodynamics of AST-3424 in BRCA pathogenic mutant and non-pathogenic mutant tumor cells, we selected two CDX models, namely human-derived pancreatic cancer Capan-1 (BRCA2 pathogenic mutant model, wherein the mutation site was p.S1982RfsTer22) and BRCA non-pathogenic mutant model HepG2 (human-derived liver cancer model). The mRNA expression levels of AKR1C3 in Capan-1 and HepG2 cell lines detected and obtained were LOG2 (FPKM) = 6.6363 and LOG2 (FPKM) = 6.8572, respectively, indicating that the mRNA expression levels of AKR1C3 in the two models were similar. A comparison of the expression levels of AKR1C3 protein with that of the H460 cell line was shown in Table 6 and FIG. 2. The results of protein expression showed that the expression level of AKR1C3 protein in BRCA pathogenic mutant Capan-1 cell was lower than that in BRCA non-pathogenic mutant HepG2 cell. On this basis, we compared the effects of BRCA pathogenic mutations on the *in vivo* pharmacodynamics of AST-3424.

**TABLE 6: The expression levels of AKR1C3 protein of each cell compared with H460 cell line**

| Cell lines | % AKR1C3 protein expression in H460 cell line |
|---|---|
| Capan-1 | 19.45% |
| HepG2 | 117.36% |
| H460 | 100% |

### 2.1.1 The in vivo pharmacodynamic experiments of AST-3424 in the BRCA pathogenic mutant Capan-1 CDX model

BALB/c nude mice were inoculated subcutaneously with human-derived pancreatic cancer Capan-1 cell to establish a subcutaneous transplantation model of human-derived pancreatic cancer. The experiment was divided into test drug Olaparib 100 mg/kg monotherapy group, AST-3424 1.5 mg/kg monotherapy group and 10% absolute ethanol + 10% polyoxyethylene (35) castor oil + 80% glucose injection D5W (pH 7.4) vehicle control group, with 6 mice in each group. The test drug Olaparib monotherapy group was administered orally by gavage once a day for a total of 30 days. Both the vehicle control group and AST-3424 monotherapy group were administered through the tail vein, wherein the vehicle control group was administered once a week for three weeks; AST-3424 monotherapy group was administered once a week for two weeks. Specific dosage regimens and TGIs were shown in Table 7. The test drug Olaparib 100 mg/kg treatment group had a slight tumor inhibitory effect on the 43rd day after tumor cell inoculation, and the relative tumor inhibition rate TGI (%) was 37.43%, with no statistically significant difference compared with the control group (p > 0.05). The test drug AST-3424 1.5 mg/kg treatment group had an extremely significant tumor inhibitory effect on the 43rd day (Day 43) after tumor cell inoculation, and the relative tumor inhibition rate TGI (%) was 97.97%; the tumors of four mice were completely cleared, and the complete tumor inhibition rate was 66.7%, with statistically significant difference compared with the control group (p < 0.001).

**Table 7: Pharmacodynamic analysis table of each group in Capan-1 subcutaneous model of human-derived pancreatic cancer**

| **Experimental group** | **Day 43 after cell inoculation** | | | | | |
|---|---|---|---|---|---|---|
| | **Tumor volume mm³ (*x̅*±S)** | **Relative tumor volume mm³ (*x̅*±S)** | **TGI (%)** | **T/C (%)** | **P Value (Compared with the control group)** | **CR ratio (Complete clearance)** |
| Vehicle control group | 1301.38±76.61 | 9.15±0.70 | - | - | - | 0/6 |
| Olaparib, 100 mg/kg | 846.86±128.85 | 5.72±0.54 | 37.43 | 62.57 | >0.05 | 0/6 |
| AST-3424, 1.5 mg/kg | 30.74±21.47 | 0.19±0.12 | 97.97 | 2.03 | <0.001 | 4/6 |

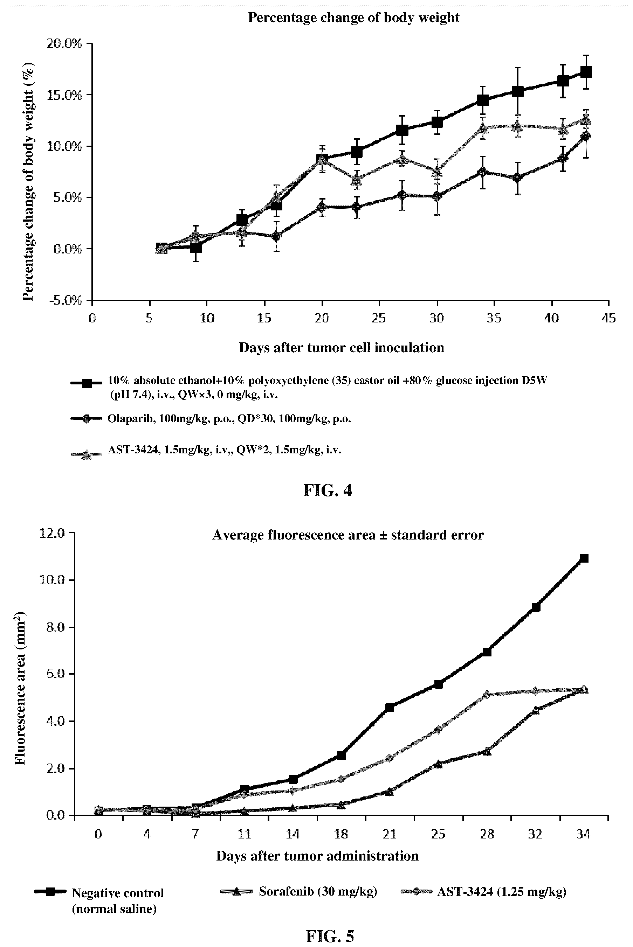

The corresponding curves of tumor volume and the percentage change of body weight of mice over time were shown in FIG. 3 and FIG. 4.

The experimental results showed that in the BRCA pathogenic mutant Capan-1 CDX model, the AST-3424 5mg/kg monotherapy group showed a significant tumor inhibitory effect, and the TGI reached 97.97% at the endpoint of the experiment, and in this experimental group, the tumors of 4 mice were completely cleared. The data of percentage body weight from the above animal experiments showed that there was no body weight loss of the experimental animals in the AST-3424 administration group, demonstrating that the drug was well tolerated.

### 2.1.2 The in vivo pharmacodynamic experiments of AST-3424 in the non-BRCA pathogenic mutant model HepG2-GFP

Liver tissue of BALB/c nude mice were inoculated in situ with human-derived liver cancer HepG2-GFP cell to establish an orthotopic transplantation model of human-derived liver cancer, and the fluorescence area of green fluorescent protein GFP was used to define the tumor size. The experiment was divided into the test drug Sorafenib monotherapy group, AST-3424 1.25 mg/kg monotherapy group and the normal saline injection vehicle control group, with 10 mice in each group. The test drug Sorafenib monotherapy group was administered orally by gavage once a day for a total of 35 times. Both the vehicle control group and AST-3424 monotherapy group were administered through the tail vein once a week for two weeks and after a one-week suspension, the administration was continued once a week for two consecutive weeks. Specific dosage regimens and TGIs were shown in Table 8.

**Table 8: Pharmacodynamic analysis table of each group in HepG2-GFP orthotopic tumor model of human-derived liver cancer**

| **Experimental group** | Day 35 after **administration** | | | | |
|---|---|---|---|---|---|
| | **Mean tumor fluorescence area** mm² (*x̅*±S) | **Mean tumor weight** (g) (*x̅*±S) | **TGI** (%) | **P Value (Compared with the control group)** | **CR ratio (Complete clearance)** |
| Normal saline | 10.9±6.0 | 0.0879±0.0590 | - | - | |
| Sorafenib 30 mg/kg | 5.3±5.3 | 0.0432±0.0373 | 51 | <0.05 | 0/10 |
| AST-3424, 1.25 mg/kg | 5.3±3.1 | 0.0350±0.0318 | 60 | <0.05 | 0/10 |

| | | | | | |
|---|---|---|---|---|---|
| Note: TGI was calculated using the following formula (calculated according to the tumor weight): TGI% = (1-treatment (T)/control (C)) × 100% (T and C were tumor weights of the treatment group and the control group at endpoint of the experimental, respectively). | | | | | |

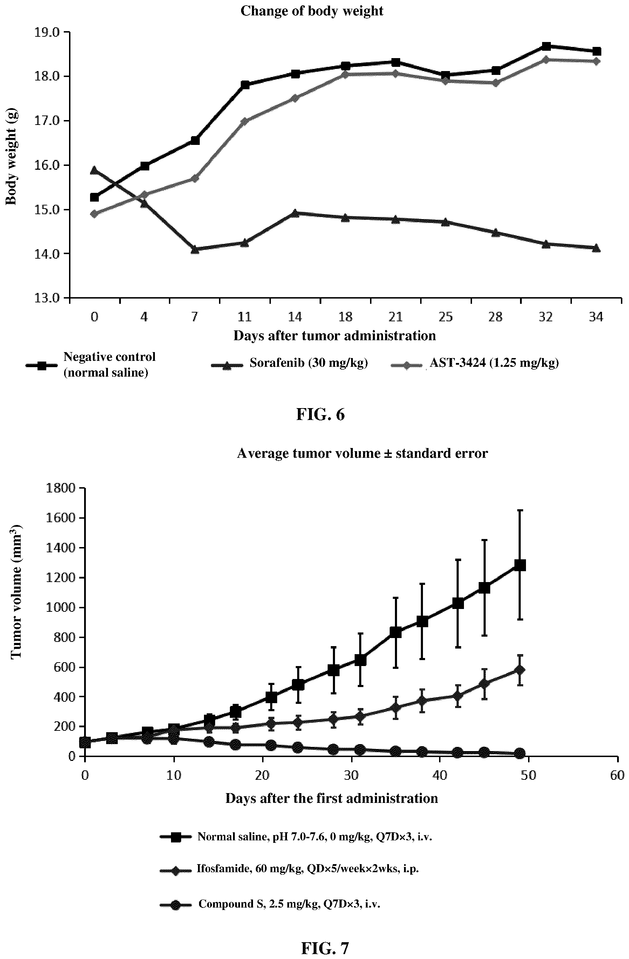

The corresponding curves of tumor volume and body weight of mice over time were shown in FIG. 5 and FIG. 6.

The experimental results showed that in the BRCA non-pathogenic mutant model, the tumor fluorescence area reading of AST-3424 1.25 mg/kg group was significantly smaller than that of vehicle control group, but had no statistically significant difference with vehicle control group (p > 0.05) and the TGI was 60%, suggesting that although AST-3424 1.25 mg/kg had a growth inhibition trend on human-derived liver cancer HepG2-GFP in situ model, there was no statistically significant inhibitory effect, and no tumor in the mice was completely cleared. The data of body weight from the above animal experiments showed that there was no body weight loss of the experimental animals in the AST-3424 administration group, demonstrating that the drug was well tolerated.

### 2.1.3 Summary of the experiment

From the above two experiments, we found that there was an obvious difference in the *in vivo* pharmacodynamics of AST-3424. AST-3424 1.5 mg/kg (administered once a week for two weeks) had a very significant *in vivo* pharmacodynamics on the BRCA pathogenic mutant Capan-1 CDX model, and TGI reached 97.97%, while in the 6 mice of AST-3424 administration group, the tumors of 4 mice were completely cleared, and the clearance rate reached 67%. However, provided that the total dose exceeded that of the Capan-1 model, AST-3424 1.25 mg/kg (administered once a week for two weeks, and after a one-week suspension, the administration was continued once a week for two consecutive weeks) had no significant pharmacodynamics on the BRCA non-pathogenic mutant model HepG2-GFP model, and TGI was 60%, with no significant difference with the control group, and no tumor in mice in the administration group was completely cleared. The above results showed that although the expression level of AKR1C3 protein in Capan-1 model was lower than that in HepG2-GFP model, there was a significant difference in the *in vivo* pharmacodynamics of AST-3424 due to the presence of BRCA pathogenic mutation in Capan-1 model and the absence of BRCA pathogenic mutation in HepG2-GFP model. The pathogenic mutation of BRCA enhanced the sensitivity of Capan-1 model to AST-3424 and promoted the killing of AST-3424 on tumor tissue.

### 2.2 Comparison of in vivo pharmacodynamics of Compound S in BRCA non-pathogenic mutant and pathogenic mutant tumor models

In order to verify whether there was a difference in the *in vivo* pharmacodynamics of **AST-3424 analogous Compound** S in BRCA pathogenic mutant and non-pathogenic mutant models, we selected two PDX models, namely human-derived gastric cancer PDX model GA6201 (BRCA2 pathogenic mutant model, wherein the mutation site was L1732PfsTer9) and BRCA non-pathogenic mutant PDX model LU5173 (human-derived lung cancer PDX model). According to the results detected by existing methods, the mRNA expression levels of AKR1C3 in GA6201 and LU5173 tumor tissues were LOG2 (FPKM) = 6.78 and LOG2 (FPKM) = 8.88, respectively, suggesting that the RNA expression levels of AKR1C3 in the two models were similar. Immunohistochemistry (IHC) was used to detect the expression levels of AKR1C3 protein in the two models, and the results were shown in Table 9. Immunohistochemistry and database suggested that the expression levels of AKR1C3 protein of the two PDX models were similar. In this part of the study, the two models with similar expression levels of AKR1C3 protein were used to compare the effect of the BRCA pathogenic mutations on the *in vivo* pharmacodynamics of **AST-3424 analogous Compound S.**

**Table 9. Comparison of the expression of AKR1C3 protein and RNA in two PDX subcutaneous model**

| **Model** | **AKR1C3 protein IHC (percentage of different staining intensities)** | | | | **AKR1C3 RNA LOG2 (FPKM)** |
|---|---|---|---|---|---|
| | **None** | **Weak** | **Medium** | **Strong** | |
| GA6201 | 1.07 | 8.75 | 30.54 | 59.63 | 6.78 |
| LU5173 | 1.97 | 7.83 | 20.77 | 69.43 | 8.88 |

| | | | | | |
|---|---|---|---|---|---|
| Note: the scoring standard of AKR1C3 **protein expression** IHC staining referred to the literature (Guise et al., Cancer Res, 2010 (70): 1573), wherein weak corresponded to Focal in the literature, medium corresponded to Moderate in the literature, and strong corresponded to Diffuse in the literature. | | | | | |

### 2.2.1 The in vivo pharmacodynamic experiments of Compound S in the BRCA pathogenic mutant model HuPrime^{®} gastric cancer GA6201

BALB/c nude mice were subcutaneously inoculated with HuPrime^{®} model GA6201 tumor blocks to establish the subcutaneous transplantation tumor model of human gastric cancer. The test was divided into test drug Ifosfamide 60 mg/kg group, test drug Compound S 2.5 mg/kg group and normal saline (pH 7.0-7.6) vehicle control group, with 5 mice in each group. Among them, normal saline (pH 7.0-7.6) vehicle control group and the test drug **Compound** S 2.5 mg/kg group were administered through tail vein injection, once a week, for a total of three weeks, and observed for four weeks. Ifosfamide 60 mg/kg group was administered by intraperitoneal injection, for five consecutive days and rested for two days in each week, for a total of two weeks, and observed for five weeks. Specific dosage regimens and TGIs were shown in Table 10.

**Table 10: Pharmacodynamic analysis table of each group in HuPrime^{®} gastric cancer GA6201 model**

| **Experimental group** | **Day 3 after the end of all administrations (i.e., Day 38)** | | | | |
|---|---|---|---|---|---|
| | **Tumor volume mm³** (*x̅*±S) | **Relative tumor volume mm³** (*x̅*±S) | **TGI** (%) | **T/C** (%) | **P Value (Compared with the control group)** |
| Normal saline, pH 7.0-7.6 | 905.75±252.4 | 9.34±2.42 | - | - | - |
| Ifosfamide 60 mg/kg | 371.02±75.96 | 3.85±0.63 | 58.83 | 41.17 | 0.857 |
| **Compound S** 2.5 mg/kg | 28.69±8.2 | 0.29±0.08 | 96.85 | 3.15 | 0.000135 |

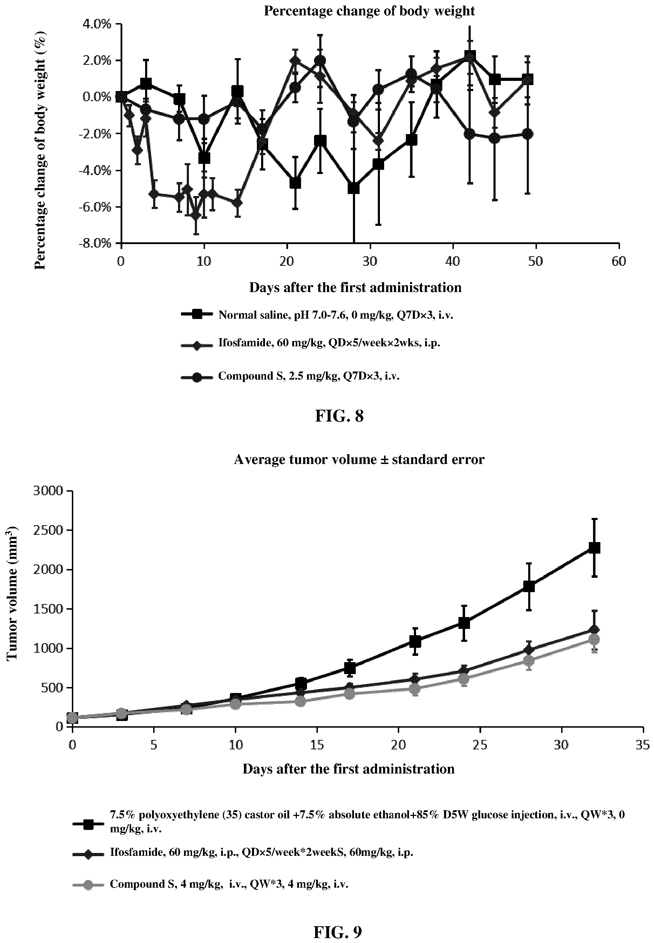

The corresponding curves of tumor volume and the percentage change of body weight of mice over time were shown in FIG. 7 and FIG. 8.

The experimental results showed that in the BRCA pathogenic mutant HuPrime^{®} gastric cancer GA6201 model, the test drug Ifosfamide (60 mg/kg) treatment group showed a certain tumor inhibitory effect on the 3rd day (Day38) after the end of all administration cycles, with no statistically significant difference compared with the control group (p = 0.857), and the relative tumor inhibition rate TGI (%) was 58.83%. The test drug Compound S (2.5 mg/kg) treatment group showed a significant tumor inhibitory effect on the 3rd day (Day 38) after the end of all administration cycles, all with statistically significant difference compared with the control group (p = 0.000135), and the relative tumor inhibition rate TGI (%) was 96.85%. There was no body weight loss of the mice in the test drug Compound S (2.5 mg/kg) treatment group, which showed no obvious drug toxicity, and the drug was well tolerated during treatment.

### 2.2.2 The in vivo pharmacodynamic experiment of Compound S in the non-BRCA pathogenic mutant model HuPrime^{®} lung cancer LU5173

NOD/SCID female mice were subcutaneously inoculated with the HuPrime^{®} model LU5173 tumor blocks to establish a subcutaneous transplantation tumor model of human lung cancer. The test was divided into positive control drug Ifosfamide 60 mg/kg group, test drug **Compound S** 4 mg/kg group and 7.5% absolute ethanol + 7.5% polyoxyethylene (35) castor oil + 85% glucose injection D5W vehicle control group, etc., with 6 mice in each group. Among them, 7.5% absolute ethanol + 7.5% polyoxyethylene (35) castor oil + 85% glucose injection D5W vehicle control group and test drug **Compound** S group were administered through tail vein injection, once a week, for three consecutive weeks. Positive control drug Ifosfamide 60 mg/kg group was administered by intraperitoneal injection, once a day, for 5 consecutive days, and rested for two days, for two consecutive weeks. Specific dosage regimens and TGIs were shown in Table 11.

**Table 11: Pharmacodynamic analysis table of each group in HuPrime^{®} lung cancer LU5173 model**

| **Experimental group** | **32nd Day after the first administration ( i.e., Day 32)** | | | | |
|---|---|---|---|---|---|
| | **Tumor volume** mm³ (*x̅*±S) | **Relative tumor volume mm³** (*x̅*±S) | **TGI** (%) | **T/C** (%) | **P Value (Compared with the control group)** |
| 7.5% absolute ethanol + 7.5% polyoxyethylene (35) castor oil + 85% glucose injection D5W, i.v. | 2276.68±367.07 | 21.55±3.26 | - | - | - |
| Ifosfamide, 60 mg/kg, i.p. | 1229.15±246.65 | 11.97±2.43 | 44.45% | 55.55% | >0.05 |
| **Compound S**, 4 mg/kg, i.v. | 1107.01±162.08 | 10.22±1.11 | 52.58% | 47.42% | <0.05 |

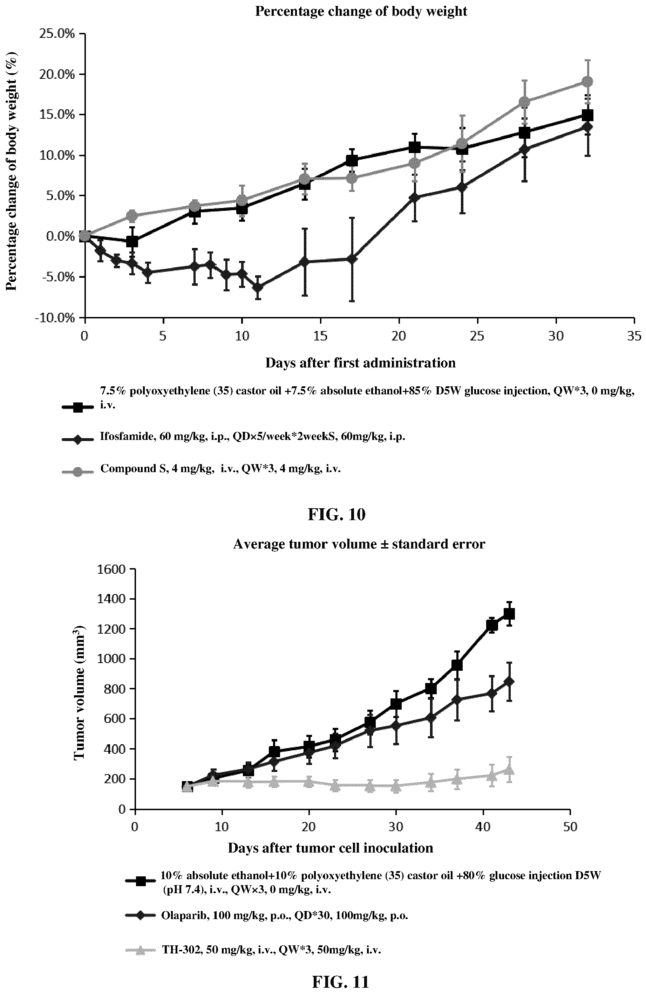

The corresponding curves of tumor volume and the percentage change of body weight of mice over time were shown in FIG. 9 and FIG. 10.

The experimental results showed that the test drug Ifosfamide (60 mg/kg) treatment group had a certain tumor inhibitory effect on the 32nd day after the first administration in the non-BRCA pathogenic mutant HuPrime^{®} lung cancer LU5173 model, and the relative tumor inhibition rate TGI (%) was 44.45%, with no statistically significant difference compared with the control group (p > 0.05). The test drug Compound S 4 mg/kg treatment group had an obvious tumor inhibitory effect on the 32nd day after the first administration, and the relative tumor inhibition rate TGI (%) was 52.58%, with statistically significant difference compared with the control group (p < 0.05). There was no any obvious body weight loss of the mice in the test drug **Compound S** (4 mg/kg) treatment group and the drug was well tolerated during treatment.

### 2.2.3 Summary of the experiment

From the above two experiments, we found that there was an obvious difference in the *in vivo* pharmacodynamic of **Compound S** in two models with similar AKR1C3 expression levels. In the BRCA pathogenic mutant HuPrime^{®} gastric cancer GA6201 model, the test drug **Compound S** 2.5 mg/kg (administered once a week for three weeks) treatment group showed a significant tumor inhibitory effect, all with statistically significant difference compared with the control group (p = 0.000135), and the relative tumor inhibition rate TGI (%) was 96.85%. In the non-BRCA pathogenic mutant HuPrime^{®} lung cancer LU5173 model, higher dose of test drug Compound S 4 mg/kg treatment group had an obvious tumor inhibitory effect on the 32nd day after the first administration, and the relative tumor inhibition rate TGI (%) was 52.58%. However, the inhibition rate was significantly lower than that of HuPrime^{®} gastric cancer GA6201 model. The above results showed that, **Compound S** in the non-BRCA pathogenic mutant model, even with a higher dose of 4 mg/kg, the tumor inhibitory effect of **Compound S** was also lower than that of the lower dose of 2.5 mg/kg administration group in the BRCA pathogenic model. This also demonstrated that pathogenic mutations of BRCA enhanced the sensitivity of HuPrime^{®} gastric cancer GA6201 model to **Compound S**, and enhanced the killing effect of Compound S on tumor tissue.

### 2.3 Comparison of in vivo pharmacodynamics of compound TH-302 in BRCA non-pathogenic mutant and pathogenic mutant tumor models

In order to detect whether there was a difference in the pharmacodynamics of TH-302 in BRCA pathogenic mutant and non-pathogenic mutant models, we selected 4 BRCA pathogenic mutant models and 3 BRCA non-pathogenic mutant models. BRCA pathogenic mutant models were human-derived pancreatic cancer Capan-1 model (BRCA2 pathogenic mutant model, wherein the mutation site was p.S1982RfsTer22), human-derived lung cancer PDX model LU6429 (BRCA2 pathogenic mutant model, wherein the mutation site was p.E2258Ter), human-derived ovarian cancer PDX model OV5304 (BRCA1 pathogenic mutant site was p.F580Kfs) and human-derived bladder cancer PDX model BL3325 (BRCA2 pathogenic mutant site was p.S2156Nfs), respectively; three BRCA non-pathogenic mutant CDX models were SK-OV-3 (human-derived ovarian adenocarcinoma), MKN45 (human-derived gastric cancer) and Vcap (human-derived prostate cancer), and the effects of BRCA pathogenic mutations on the *in vivo* pharmacodynamics of TH-302 were compared in these seven models.

### 2.3.1 The in vivo pharmacodynamics of TH-302 in the BRCA pathogenic mutant model Capan-1

BALB/c nude mice were subcutaneously inoculated with human-derived pancreatic cancer Capan-1 cell to establish a subcutaneous transplantation model of human-derived pancreatic cancer. The test was divided into the test drug Olaparib 100 mg/kg monotherapy group, TH-302 50 mg/kg monotherapy group, and 10% absolute ethanol + 10% polyoxyethylene (35) castor oil + 80% glucose injection D5W (pH 7.4) vehicle control group, etc., with 6 mice in each group. Both the vehicle control group and TH-302 monotherapy group were administered through tail vein, once a week for a total of three weeks; the test drug Olaparib monotherapy group was administered orally by gavage once a day for a total of 30 days. The specific administration regimens and TGIs were shown in Table 12.

**Table 12: Pharmacodynamic analysis table of each group in the human-derived pancreatic cancer Capan-1 subcutaneous model**

| **Experimental group** | **43rd Day after cell inoculation** | | | | | |
|---|---|---|---|---|---|---|
| | **Tumor volume** mm³ (*x̅*±S) | **Relative tumor volume mm³** (*x̅*±S) | **TGI** (%) | **T/C** (%) | **P Value (Compared with the control group)** | **CR Ratio (Complete clearance)** |
| 10% absolute ethanol +10% polyoxyethylene (35) castor oil +80% glucose injection D5W (pH 7.4) | 1301.38±76.61 | 9.15±0.70 | - | - | - | 0/6 |
| Olaparib, 100 mg/kg | 846.86±128.85 | 5.72±0.54 | 37.43 | 62.57 | >0.05 | 0/6 |
| TH-302, 50 mg/kg | 263.45±86.54 | 1.81±0.64 | 80.18 | 19.82 | <0.001 | 2/6 |

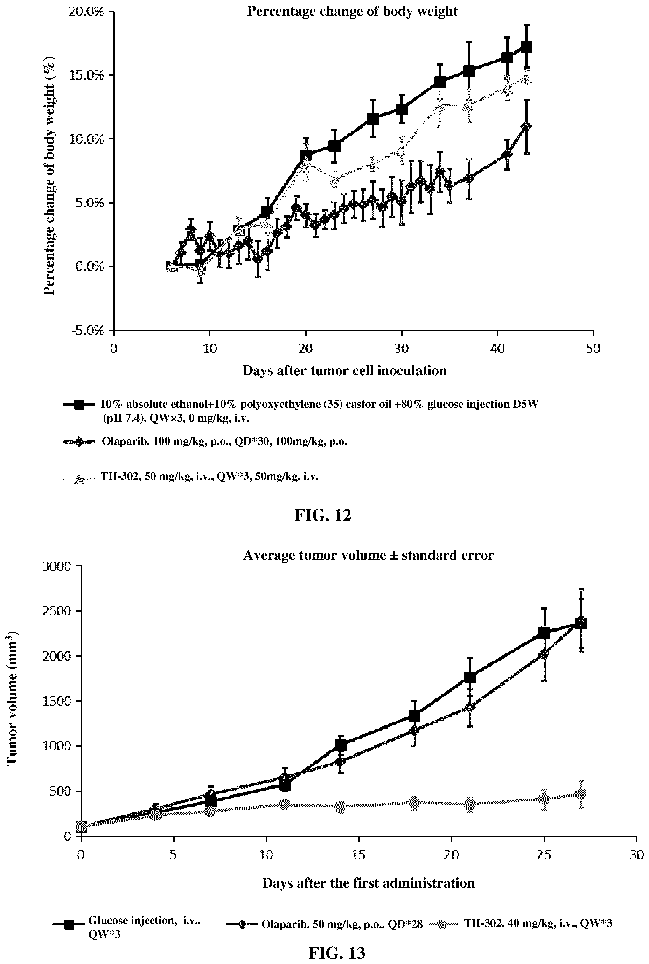

The corresponding curves of tumor volume and the percentage change of body weight of mice over time were shown in FIG. 11 and FIG. 12.

The experimental results showed that in the BRCA pathogenic mutant Capan-1 CDX model, the test drug Olaparib 100 mg/kg treatment group showed a slight tumor inhibitory effect on the 43rd day after tumor cell inoculation, and the relative tumor inhibition rate TGI (%) was 37.43%, with no statistically significant difference compared with the control group (p > 0.05). The test drug TH-302 50 mg/kg treatment group showed a significant tumor inhibitory effect on the 43rd day after tumor cell inoculation, the relative tumor inhibition rate TGI (%) was 80.18%, and the complete tumor inhibition rate was 33.3%, with statistically significant difference compared with the control group (p < 0.001). The data of percentage body weight from the above animal experiments showed that there was no body weight loss of the experimental animal in TH-302 administration group, demonstrating that the drug was well tolerated.

### 2.3.2 In vivo pharmacodynamics of TH-302 in BRCA pathogenic mutant model HuPrime^{®} lung cancer LU6429

BALB/c nude female mice were subcutaneously inoculated with HuPrime^{®} model LU6429 tumor blocks to establish a subcutaneous transplantation tumor model of human lung cancer. The test was divided into the test drug Olaparib 50 mg/kg group, the test drug **TH-302** 40 mg/kg group and glucose injection D5W vehicle control group, etc., with 6 mice in each group. Among them, the glucose injection D5W vehicle control group and test drug TH-302 group were administered through tail vein injection once a week for three consecutive weeks. The test drug Olaparib 50 mg/kg group was administered orally by gavage, once a day for 28 consecutive days. The specific administration regimens and TGIs were shown in Table 13.

**Table 13: Pharmacodynamic analysis table of each group in the HuPrime^{®} lung cancer LU5173 model**

| **Experimental group** | **27th day after the first administration** | | | | | |
|---|---|---|---|---|---|---|
| | **Tumor volume** mm³ (*x̅*±S) | **Relative tumor volume mm³** (*x̅*±S) | **TGI** (%) | **T/C** (%) | **P Value (Compared with the control group)** | **CR Ratio (Complete clearance)** |
| glucose injection D5W | 2362.40±274.50 | 23.14±3.50 | - | - | - | 0/6 |
| Olaparib, 50 mg/kg | 2390.86±344.38 | 23.33±2.54 | -0.83 | 100.83 | >0.05 | 0/6 |
| TH-302, 40 mg/kg | 466.06±149.54 | 4.55±0.64 | 80.33 | 19.97 | <0.05 | 0/6 |

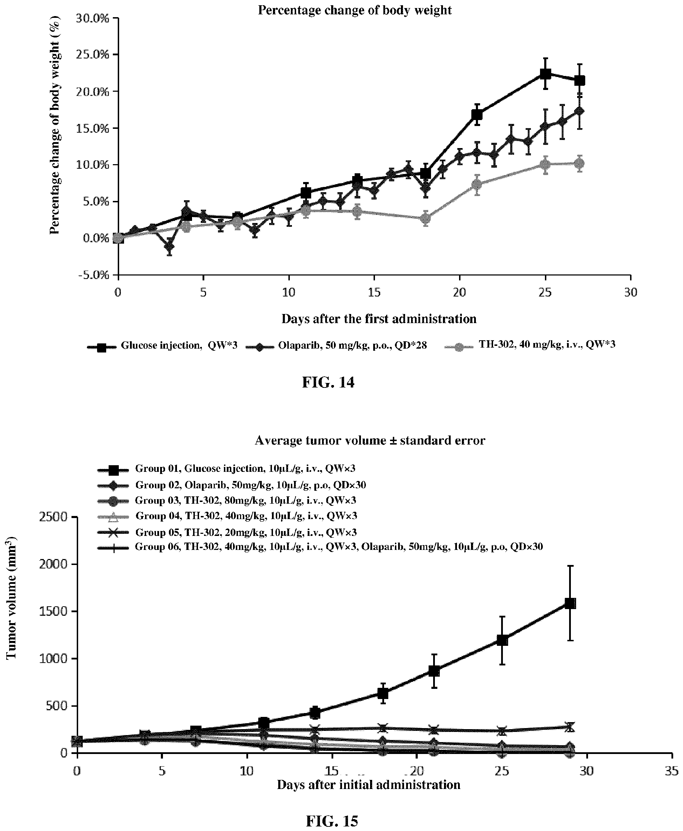

The corresponding curves of tumor volume and the percentage change of body weight of mice over time were shown in FIG. 13 and FIG. 14.

The experimental results showed that in the BRCA pathogenic mutant HuPrime^{®} lung cancer LU6429 model, the test drug Olaparib (50 mg/kg) treatment group showed no tumor inhibitory effect on the 27th day after the first administration, and the relative tumor growth inhibition rate TGI (%) was -0.83%, with no statistically significant difference compared with the control group (p > 0.05). The test drug compound TH-302 40 mg/kg treatment group showed a significant tumor inhibitory effect on the 27th day after the first administration, and the relative tumor growth inhibition rate TGI (%) was 80.33%, with statistically significant difference compared with the control group (p < 0.05). There was no any obvious body weight loss of the mice in the test drug TH-302 (40 mg/kg) treatment group, and the drug was well tolerated during the treatment period.

### 2.3.3 In vivo pharmacodynamics of TH-302 in the BRCA pathogenic mutant model HuPrime^{®} ovarian cancer OV5304

Balb/c nude female mice were subcutaneously inoculated with HuPrime^{®} ovarian cancer OV5304 tumor blocks to establish a subcutaneous transplantation tumor model of human ovarian cancer. The test was divided into the test drug Olaparib 50 mg/kg monotherapy group (Group 02), TH-302 80 mg/kg monotherapy group (Group 03), TH-302 40 mg/kg monotherapy group (Group 04), TH-302 20 mg/kg monotherapy group (Group 05), the combined administration group of TH-302 40 mg/kg and Olaparib 50 mg/kg (Group 06), and glucose injection vehicle control group (Group 01). There was a total of 6 groups and 6 mice in each group. Among them, both the vehicle control group and TH-302 were administered through tail vein, once a week for a total of 3 weeks; Olaparib was administered by gavage, once a day for a total of 30 days. The specific administration regimens and pharmacodynamics were shown in Table 14.

**Table 14: Pharmacodynamics analysis table of each group in the HuPrime^{®} ovarian cancer OV5304 subcutaneous model**

| **Experimental group** | **30th day after initial administration** | | | | | |
|---|---|---|---|---|---|---|
| | **Tumor volume** (*x̅*±S) | **Relative tumor volume** (*x̅*±S) | **TGI** (%) | **T/C** (%) | **P Value (Compared with the control group)** | **Tumor clearance rate** |
| **Group 1** glucose injection, 10 µL/g, *i.v.,* QW×3 | 1585.25±397.93 | 13.01±2.88 | | | - | 0/6 |
| **Group 2** Olaparib,50 mg/kg,10 µL/g,*p.o.*, QD×30 | 60.08±16.91 | 0.50±0.13 | 96.16 | 3.84 | <0.001 | 0/6 |
| **Group 3** TH-302 80 mg/kg, 10 µE/g, *i.v*., QW×3 | 0.00±0.00 | 0.00±0.00 | 100.00 | 0.00 | <0.001 | 6/6 |
| **Group 4** TH-302 40 mg/kg, 10 µL/g, *i.v.*, QW×3 | 31.19±10.09 | 0.26±0.09 | 97.98 | 2.02 | <0.001 | 1/6 |
| **Group 5** TH-302 20 mg/kg, 10 µL/g, *i.v.*, QW×3 | 270.95±47.32 | 2.24±0.34 | 82.81 | 17.19 | <0.01 | 0/6 |
| **Group 6** TH-302 40 mg/kg,10 µL/g, *i. v. ,* QW×3+Olaparib,50 mg/kg,10 µL/g, *p.o.*, QD×30 | 0.00±0.00 | 0.00±0.00 | 100.00 | 0.00 | <0.001 | 6/6 |

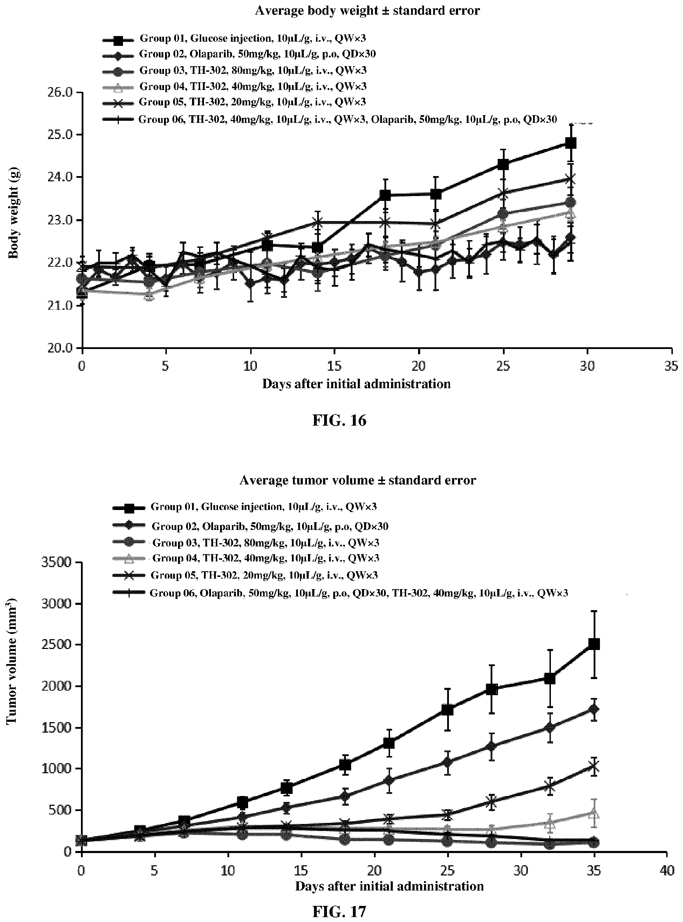

The corresponding curves of tumor volume and the percentage change of body weight of mice over time were shown in FIG. 15 and FIG. 16.

The above experimental results showed that in the BRCA1 pathogenic mutant OV5304 model, each TH-302 monotherapy at the tested dosage in this study had significant anti-tumor effects and showed dose-dependency on the HuPrime^{®} ovarian cancer OV5304 subcutaneous model. Tumor clearance rate of the combined group of test drug TH-302 40 mg/kg and Olaparib 50 mg/kg was 100%. Meanwhile, the tumor inhibitory effect of the combined treatment group of test drug TH-302 40 mg/kg and Olaparib 50 mg/kg was significantly superior to those of the Oplaparib 50 mg/kg monotherapy treatment group and the TH-302 40 mg/kg monotherapy treatment group, with statistically significance. There was no any obvious body weight loss of the mice in each treatment group, and the drug was well tolerated during the treatment period.

### 2.3.4 In vivo pharmacodynamics of TH-302 in the BRCA pathogenic mutant model HuPrime^{®} bladder cancer BL3325

Balb/c nude female mice were subcutaneously inoculated with HuPrime^{®} bladder cancer BL3325 tumor blocks to establish a subcutaneous transplantation tumor model of human bladder cancer. The test was divided into the test drug Olaparib 50 mg/kg monotherapy group (Group 02), TH-302 80 mg/kg monotherapy group (Group 03), TH-302 40 mg/kg monotherapy group (Group 04), TH-302 20 mg/kg monotherapy group (Group 05), the combined administration group of TH-302 40 mg/kg and Olaparib 50 mg/kg (Group 06), and glucose injection vehicle control group (Group 01). There was a total of 6 groups and 6 mice in each group. Among them, both the vehicle control group and TH-302 were administered through tail vein, once a week for a total of 3 weeks; Olaparib was administered by gavage, once a day for a total of 30 days. The specific administration regimens and pharmacodynamics were shown in Table 15.

**Table 15: Pharmacodynamics analysis table of each group in the HuPrime^{®} bladder cancer BL3325 subcutaneous model**

| **Experimental group** | **36th day after initial administration** | | | | | |
|---|---|---|---|---|---|---|
| | **Tumor volume** (*x̅*±S) | **Relative tumor volume** (*x̅*±S) | **TGI** (%) | **T/C** (%) | **P Value (Compared with the control group)** | **Tumor clearance rate** |
| **Group 1** glucose injection, 10 µL/g, *i.v.,* QW×3 | 2506.70±403.02 | 19.30±2.69 | | | - | 0/6 |
| **Group 2** Olaparib, 50 mg/kg, 10 µL/g, *p.o.,* QD×30 | 1717.98±129.57 | 13.43±0.40 | 30.42 | 69.58 | >0.05 | 0/6 |
| **Group 3** TH-302 80 mg/kg, 10 µL/g, *i.v.,* QW×3 | 103.73±38.44 | 0.76±20.26 | 96.05 | 3.95 | <0.001 | 1/6 |
| **Group 4** TH-302 40 mg/kg, 10 µL/g, *i. v. ,* QW×3 | 464.64±168.62 | 3.78±1.60 | 80.42 | 19.58 | <0.001 | 0/6 |
| **Group 5** TH-302 20 mg/kg, 10 µL/g, *i. v. ,* QW×3 | 1028.58±111.81 | 8.08±0.81 | 58.16 | 41.84 | <0.05 | 0/6 |
| **Group 6** TH-302 40 mg/kg, 10 µL/g, *i. v. ,* QW×3+Olaparib, 50 mg/kg, 10 µL/g, *p.o.*, QD×30 | 129.97±9.59 | 1.03±0.09 | 94.66 | 5.34 | <0.001 | 0/6 |

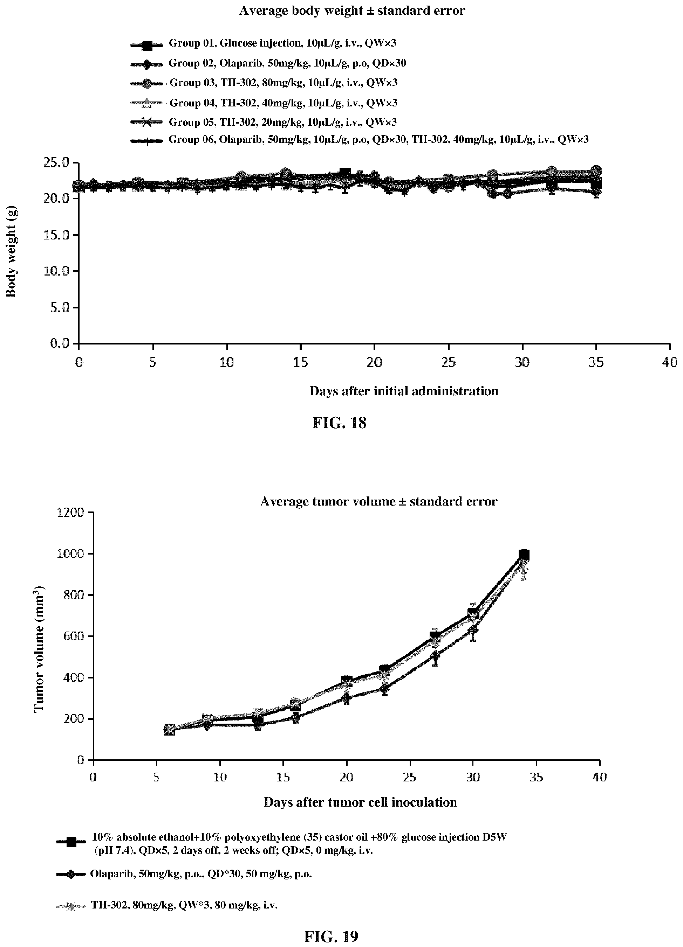

The corresponding curves of tumor volume and the percentage change of body weight of mice over time were shown in FIG. 17 and FIG. 18.

The above experimental results showed that in the BRCA2 pathogenic mutant BL3325 model, each TH-302 monotherapy group at the tested dosage in this study had significant anti-tumor effects and showed dose-dependency on the HuPrime^{®} bladder cancer BL3325 subcutaneous model. The combined therapeutic effect of the test drug TH-302 40 mg/kg and Olaparib 50 mg/kg showed a significant difference compared with those of the control group and Oplaparib at 50 mg/kg monotherapy treatment group (both p < 0.01), but showed no significant difference compared with that of TH-302 at 40 mg/kg monotherapy treatment group (p > 0.05). There was no any obvious body weight loss of the mice in each treatment group, and the drug was well tolerated during the treatment period.

### 2.3.5 In vivo pharmacodynamic experiment of TH-302 in non-BRCA pathogenic mutant model SK-OV-3 (human-derived ovarian adenocarcinoma), MKN45 (human-derived gastric cancer) and Vcap (human-derived prostate cancer)

BALB/c nude mice were subcutaneously inoculated with human-derived SK-OV-3 cell, human-derived MKN45 cell and human-derived Vcap cell respectively to establish the above three human-derived CDX models. The administrations of TH-302 in the three models were identical. The test was divided into the test drug Olaparib 50 mg/kg monotherapy group, TH-302 80 mg/kg monotherapy group and the vehicle control group, with 6 mice in each group. Both the vehicle control group and TH-302 80 mg/kg monotherapy group were administered through tail vein; wherein the vehicle control group was administered once a day for 5 consecutive days, rested for 2 days, and then rested for 2 weeks; and then once a day for 5 consecutive days daily, and then once a day; the test drug TH-302 80 mg/kg was administered once a week for 3 consecutive weeks; the test drug Olaparib group was administered orally by gavage, once a day for a total of 30 days. The specific administration regimens and TGIs were shown in Table 16.

**Table 16: Pharmacodynamics analysis table of each group in the three non-BRCA pathogenic mutant subcutaneous model**

| **Model** | **Experimental group** | **Tumor volume** mm³ (*x̅*±S) | **Relative tumor volume mm³** (*x̅*±S) | **TGI** (%) | **T/C** (%) | **P Value (Compared with the control group)** | **CR Ratio (Complete clearance)** |
|---|---|---|---|---|---|---|---|
| SK-OV-3 | 10% absolute ethanol +10% polyoxyethylene (35) castor oil +80% glucose injection D5W (pH 7.4) | 994.19±79.53 | 7.02±0.71 | - | - | - | 0/6 |
| | Olaparib, 50 mg/kg | 962.76±54.37 | 6.72±0.46 | 4.29 | 95.71 | >0.05 | 0/6 |
| | **TH-302**, 80 mg/kg | 943.37±68.94 | 6.50±0.47 | 7.38 | 92.62 | >0.05 | 2/6 |
| MKN45 | glucose injection DSW (pH 7.4) | 1957.94±251.25 | 13.55±1.13 | - | - | - | 0/6 |
| | Olaparib 50 mg/kg | 1504.93±305.84 | 10.14±1.71 | 25.18 | 74.82 | >0.05 | 0/6 |
| | **TH-302**, 80 mg/kg | 1542.86±234.59 | 10.67±1.51 | 21.23 | 78.77 | >0.05 | 0/6 |
| Vcap | glucose injection D5W (pH 7.4) | 1394.61±188.29 | 9.81±1.15 | - | - | - | 0/6 |
| | Olaparib 50 mg/kg | 1216.20±214.90 | 8.51±1.28 | 13.22 | 86.78 | >0.05 | 0/6 |
| | TH-302, 80 mg/kg | 1036.24±118.37 | 7.33±0.67 | 25.26 | 74.74 | >0.05 | 0/6 |

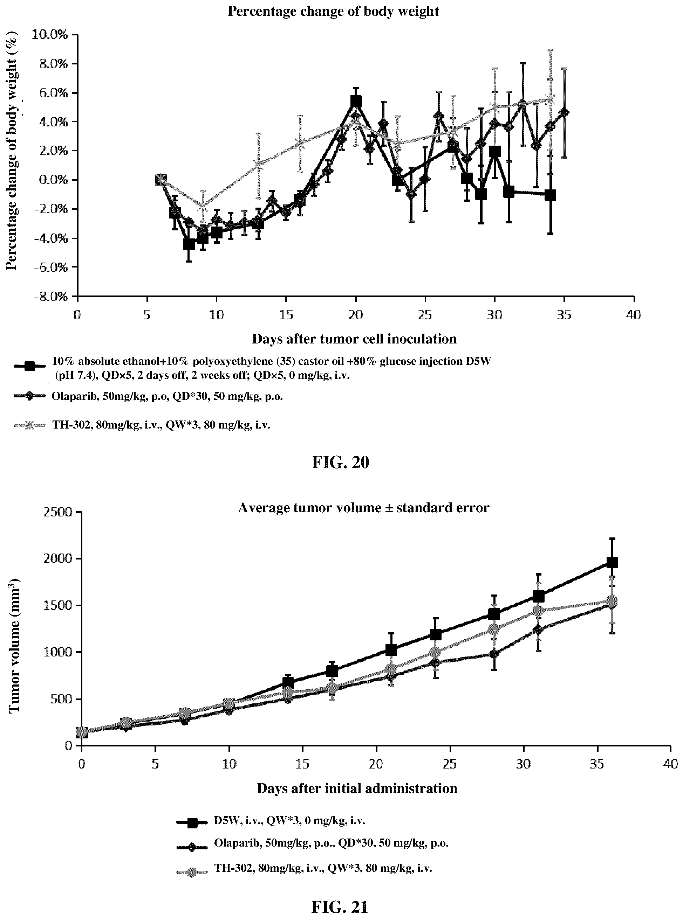

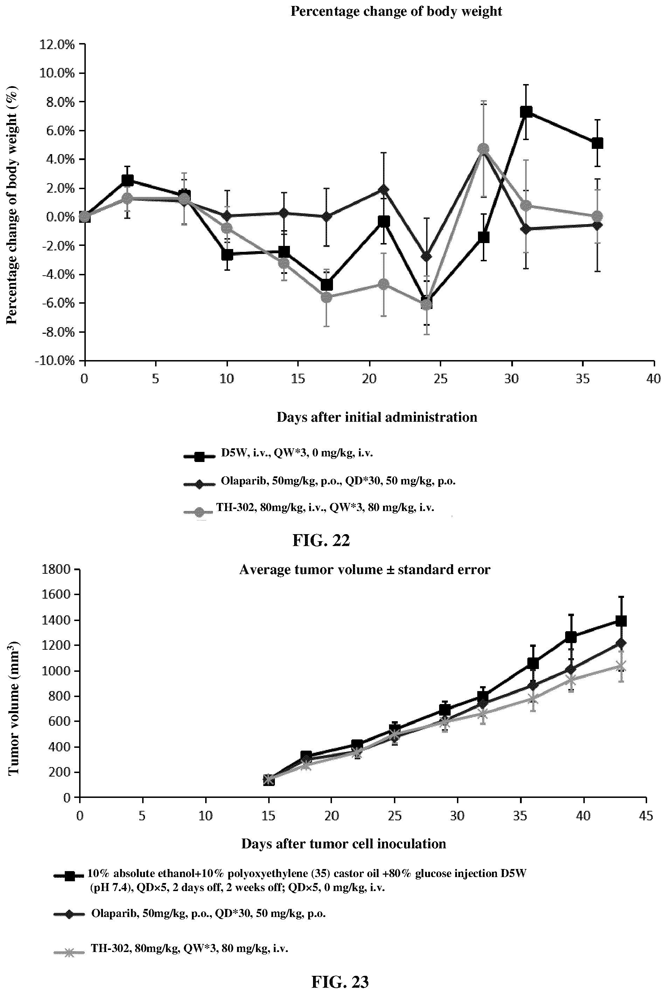

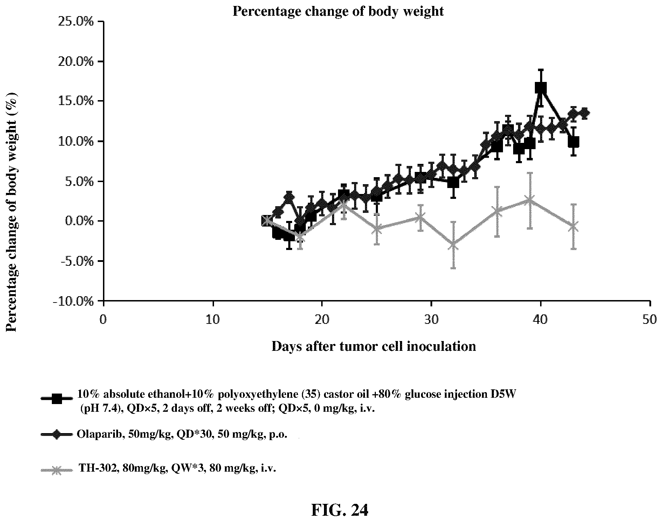

The corresponding curves of tumor volume and the percentage change of body weight of mice over time were shown in FIGs. 19 to 24.

The experimental results showed that in the three BRCA non-pathogenic mutant CDX models of human-derived SK-OV-3, MKN45 and Vcap, there was no statistically significant tumor inhibitory effect in the test drug TH-302 80 mg/kg treatment group (p-values were all greater than 0.05), and the relative tumor growth inhibition rates TGI (%) were 7.38%, 21.33% and 25.26%, respectively. The data of body weight from the above animal experiments showed that there was no body weight loss of the experimental animal in TH-302 administration group, demonstrating that the drug was well tolerated.

### 2.3.6 Summary of the experiment

The above seven experimental results showed that the *in vivo* pharmacodynamics of TH-302 had a significant difference in the BRCA pathogenic mutant and the non-pathogenic mutant models. TH-302 50 mg/kg (administered once a week for three weeks) had a very obvious tumor growth inhibitory effect on BRCA2 pathogenic mutant Capan-1 CDX model, had a significant tumor inhibitory effect on the 43rd day after tumor cell inoculation, and the relative tumor growth inhibition rate TGI (%) was 80.18%, wherein the tumors of two mice were completely cleared, and the tumor complete inhibition rate was 33.3%, with statistically significant difference compared with the control group (p < 0.001). TH-302 40 mg/kg (administered once a week for three weeks) also showed a very significant tumor inhibitory effect in the BRCA2 pathogenic mutant human-derived lung cancer PDX model LU6429, and the relative tumor growth inhibition rate (%) was 80.33% on the 27th day after the administration, with statistically significantly difference compared with the control group (p < 0.05). In addition, each monotherapy group of 80 mg/kg, 40 mg/kg and 20 mg/kg of TH-302 showed significant tumor inhibitory effects in BRCA1 pathogenic mutant ovarian cancer OV5304 and BRCA2 pathogenic mutant bladder cancer BL3325. However, the higher dose of TH-302 80 mg/kg (administered once a week for three weeks) had no tumor inhibitory effect in the three non-BRCA pathogenic mutant models. These results suggested that the BRCA pathogenic mutant model was more sensitive to TH-302 than that in the non-pathogenic mutant model.

Our results also found that in the BRCA1 pathogenic mutant ovarian cancer OV5304 model, the combination of TH302 with Olaparib had a statistically different combination-enhanced antitumor effect compared with each monotherapy.

In summary, the results of *in vitro* and *in vivo* pharmacodynamic experiments of AST-3424, Compound S and TH-302 showed that BRCA pathogenic mutant cell lines and mouse tumor models were more sensitive to the above three compounds, suggesting that in future pre-clinical pharmacodynamic experiment and development of clinical test, BRCA pathogenic mutation can be applied as a target to screen animal models and clinical patients that are more sensitive to AST-3424, Compound S, and TH-302.

According to the literatures (Meng, F., Li, W. F., Jung, D., Wang, C. C., Qi, T., Shia, C. S., Hsu, R. Y., Hsieh, Y. C., & Duan, J. (2021). A novel selective AKR1C3-activated prodrug AST-3424/OBI-3424 exhibits broad anti-tumor activity. American journal of cancer research, 11(7), 3645-3659; Evans, K., Duan, J., Pritchard, T., Jones, C. D., McDermott, L., Gu, Z., Toscan, C. E., El-Zein, N., Mayoh, C., Erickson, S. W., Guo, Y, Meng, F., Jung, D., Rathi, K. S., Roberts, K. G., Mullighan, C. G., Shia, C. S., Pearce, T., Teicher, B. A., Smith, M. A., Lock, R. B. (2019). OBI-3424, a Novel AKR1C3-Activated Prodrug, Exhibits Potent Efficacy against Preclinical Models of T-ALL. Clinical cancer research: an official journal of the American Association for Cancer Research, 25(14), 4493-4503; Yanlan Wang, Yue Liu, Changhua Zhou, Chunnian Wang, Ning Zhang, Donglin Cao, Qing Li & Zhong Wang (2020) An AKR1C3-specific prodrug with potent anti-tumor activities against T-ALL, Leukemia & Lymphoma, 61(7), 1660-1668; Meng, F., James W. Evans, J.W., , Deepthi Bhupathi, D., Monica Banica, M., Leslie Lan, L., Lorente, G., Duan, J., Cai, X , Mowday, J. M., Guise, C. P., Maroz, A., Anderson, R.F., Patterson, A. V., Stachelek, G.C., Glazer, P.M., Matteucci, M.D., and Hart, C.P.(2012) Molecular and Cellular Pharmacology of the Hypoxia-Activated Prodrug TH-302. Mol Cancer Ther 2012; 11:740-75111:740-751)
as well as the corresponding patent applications and the specific compounds described in the claims of the following patent applications: 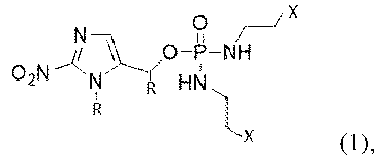 WO2010048330A1 , WO2012142520A2 and WO2008083101A1; 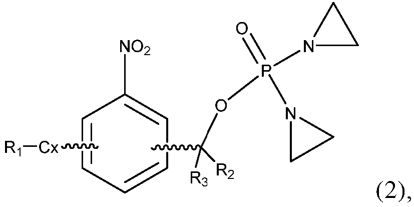 Patent Application PCT/CN2020/114519 with Publication No. WO2021120717A1; 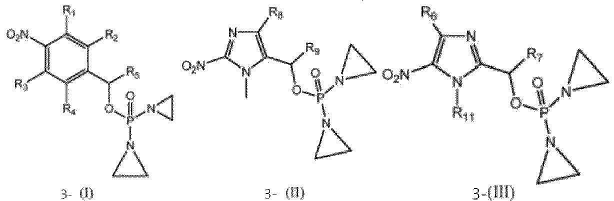 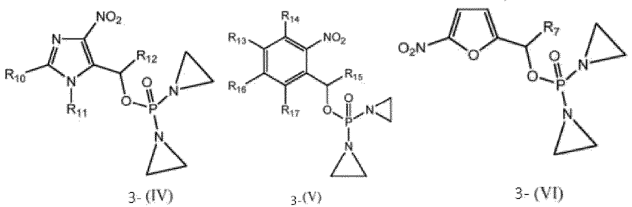
Patent Application PCT/US2016/039092 with Publication No. WO2016210175A1 (corresponding to the Chinese Application No. 2016800368985 with Publication No. CN108024974A); 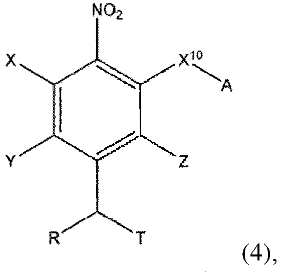 Patent Application PCT/US2016/062114 with Publication No. WO2017087428A1 (corresponding to the Chinese Application No. 2016800200132 with Publication No. CN108136214A); 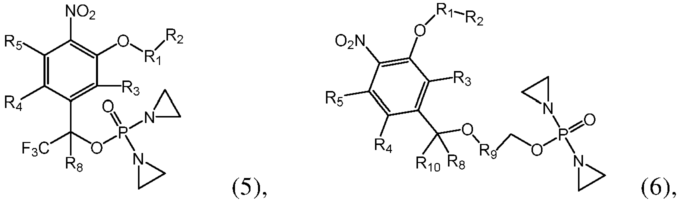 Patent Application PCT/CN2020/089692 with Publication No. WO2020228685A1; 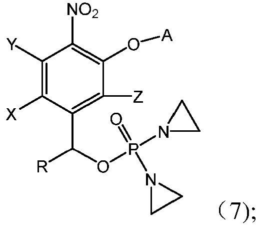 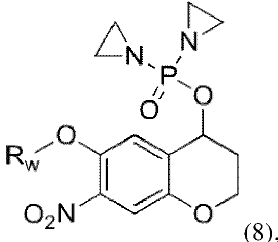 Patent Application PCT/CN2020/120281 with Publication No. WO2021068952A1; 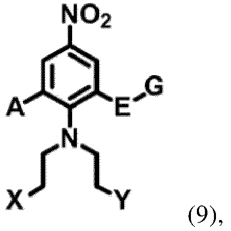 Patent Application PCT/NZ2019/050030 with Publication No. WO2019190331A1 (corresponding to the Chinese Application No. 2019800234236 with Publication No. CN111918864A); 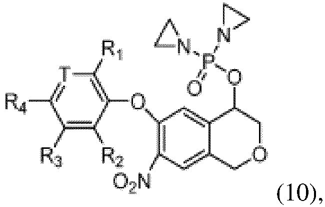 Patent Application PCT/CN2021/118597 with Publication No. WO2022057838A1;
the above compounds were similar to the toxin structures of AST-3424, Compound S, TH-302, and they are all AKR1C3-activated/hypoxia-activated anticancer prodrugs, which cleave into DNA alkylating agents with similar toxin structures after being activated under the conditions of AKR1C3 enzyme or hypoxia. Therefore, in combination with the above experimental results of AST-3424, Compound S, and TH-302, it can be expected that the AKR1C3-activated/hypoxia-activated DNA alkylating agent prodrugs with similar toxin structures have more significant therapeutic effects than drug monotherapy or in combination other therapeutic drugs for treating patients with BRCA pathogenic mutated cancer and tumors, and thus the use of BRCA mutation as a marker to select patients is feasible.

Further, the toxins of the above AKR1C3-activated or hypoxia-activated DNA alkylating agent are all alkylating agents that act on DNA double-strand to make them crosslink, and therefore the applicant expected that the compounds as described above should have more excellent therapeutic effects on the tumor model with mutations in homologous DNA double-strand damage repair genes than that in tumor model without mutations, i.e., AKR1C3-activated/hypoxia-activated DNA alkylating agent prodrugs with similar toxin structures have more significant therapeutic effects than drug monotherapy or in combination other therapeutic drugs for treating patients with homologous DNA double-strand damage repair gene-mutated cancer and tumors, and thus the use of homologous DNA double-strand damage repair gene mutation as a marker to select patients is feasible. 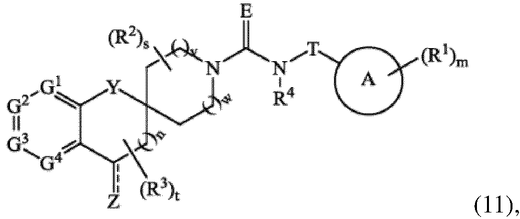 which is described in the claims of Patent Application CN202210585771.6 with Publication No. CN115403579A; and 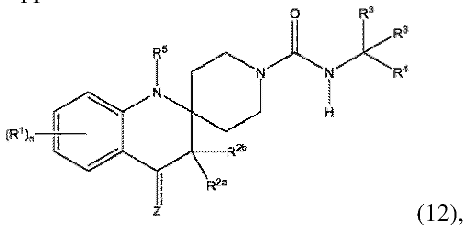 or pharmaceutically acceptable salt thereof, wherein, the definitions of R¹, R^{2a}, R^{2b}, R³, R⁴, R⁵, n and Z are described in the claims of Patent Application PCT/IB2020/057285 with Publication No. WO2021005586A1 (corresponding to the Chinese Application No. CN202080053804.1 with Publication No. CN114206870A).

Such compounds are AKR1C3-activated KARS inhibitor prodrugs. KARS is a lysine-tRNA ligase, which acts on the translation process of amino acids that changes a combination of four bases into a combination of 20 amino acids, and specifically links lysine to the tRNA encoding lysine. The tRNA-to-amino acid correspondence is determined by aminoacyl tRNA synthetase, which needs to correctly recognize the substrate amino acid and the corresponding tRNA. There are often structurally similar amino acids that ordinary enzymes are unable to distinguish finely, such as Ile/Val, Thr/Ser, etc. Therefore, ARS has to have stronger recognition ability to prevent incorrect aminoacylation, and KARS can accurately recognize lysine and link lysine with tRNA encoding for lysine, and ultimately achieve the correct linkage of the amino acid sequences to form a polypeptide chain.

KARS inhibitors can specifically inhibit the activity of KARS enzyme, which leads to errors in the translation procedure of the DNA-RNA-amino acid process, and ultimately makes the cell fail to replicate normally and apoptosis, i.e., KARS inhibitors act on the translation process of RNA-amino acid. Obviously, if the mutations occur in homologous DNA double-strand damage repair gene in the above process, leading to problems in the DNA-RNA process as well, it will undoubtedly further exacerbate apoptosis, and thus organisms with mutations in homologous DNA double-strand damage repair genes will possibly have a stronger response, and greater sensitivity, to AKR1C3-activated KARS inhibitor prodrugs.

The homologous DNA double-strand damage repair genes can be selected from the corresponding genes of BRCA (including BRCA1, BRCA2), FANCA, FANCD1, FANCD2, ATM, ATR, CHEK1, CHEK2, CTP, BARD1, BRIP1, PALB2, RAD51D, RAD51C, RAD52, RAD54, RAD55, RAD57, FAM175, NBN, Rad50, MRE11, p53, NBS1, XRS2, XRCC2, XRCC3, XRCC4/XPF, ERCC1, ERCC2/XPD, ERCC3/XPB, ERCC4/XPF, XRCC1, Ku80, MHS6, MGMT, PARP, ERCC5/XPG, CCNH, CDK7, CETN2, DDB1, DDB2, ERCC5/XPG, ERCC6/CSB, ERCC8/CSA, LIG1/DNA ligase I, MMS19, MNAT1, RAD23A, RAD23B, RPA1, RPA2, TFIIH, XAB2, XPA, XPC, MBD4, NEIL1, BAP1, CDK12, EXO1, FAAP20, FAN1, FANCE, FANCM, MDC1, NONO, POLQ, RAD51B, RBBP8, SMC5, USP11, WRN, and AP endonuclease, end processing enzyme, DNA polymerase, Flap endonuclease, DNA ligase.

## Claims

1. A method for treating BRCA gene-mutated cancer, which uses a drug monotherapy containing hypoxia-activated prodrug compounds of formulas (1), (2), (3) or AKR1C3 enzyme-activated prodrug compounds of formulas (4), (5), (6), (7), (8), (9), (10), (11), (12) and salts, esters, solvates, and isotopic isomers thereof or in combination with other drugs to treat patients with BRCA gene-mutated cancer and tumors: 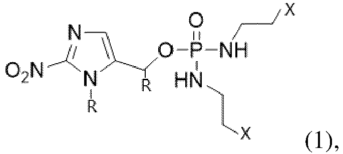
wherein the R is each independently selected from H, -CH₃, -CH₂CH₃, -CF₃, X is each independently selected from Cl, Br, MsO, TsO and other leaving functional groups; 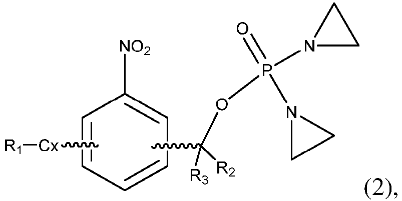
wherein the definitions of R₁, R₂, R₃ and Cx are described in the claims of Patent Application PCT/CN2020/114519 with Publication No. WO2021120717A1; 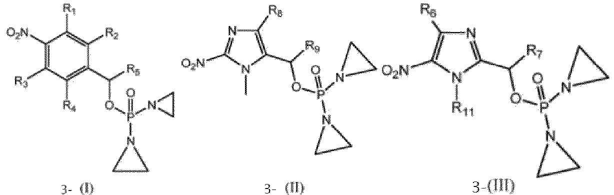 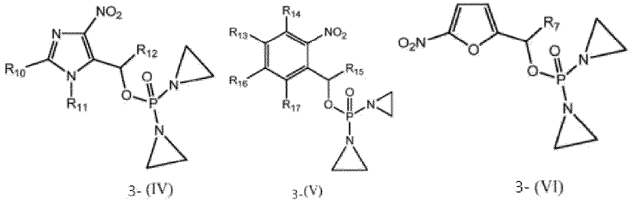
wherein the definitions of R₁, R₂, R₃, R₄, R₅, R₆, R₇, R₈, R₉, R₁₀, R₁₁, R₁₂, R₁₃, R₁₄, R₁₅, R₁₆ and R₁₇ are described in the claims of Patent Application PCT/US2016/039092 with Publication No. WO2016210175A1 (corresponding to Chinese Patent Application No. 2016800368985 with Publication No. CN108024974A); 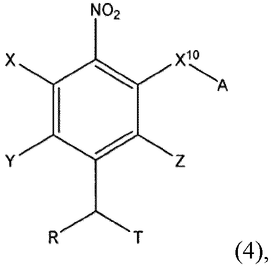
wherein the definitions of X, Y, Z, R, T, A and X¹⁰ are described in the claims of Patent Application PCT/US2016/021581 with Publication No. WO2016145092A1 (corresponding to Chinese Patent Application No. CN201680015078.8 with Publication No. CN107530556A); 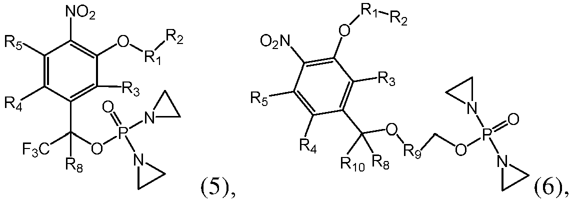
wherein the definitions of R₁, R₂, R₃, R₄, R₅, R₈, R₉ and R₁₀ are described in the claims of Patent Application PCT/CN2020/089692 with Publication No. WO2020228685A1; 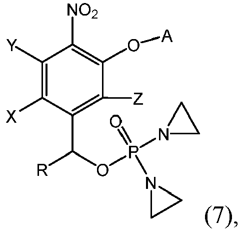
wherein:
A is a substituted or unsubstituted C₆-C₁₀ aryl, biaryl or substituted biaryl, 5-15 membered heteroaryl, or -N=CR¹R², wherein the substituents are selected from the group consisting of halogeno, -CN, -NO₂, -O-(CH₂)-O-, -CO₂H and salts thereof, -OR¹⁰⁰, -CO2R¹⁰⁰, -CONR¹⁰¹R¹⁰², -NR¹⁰¹R¹⁰², -NR¹⁰⁰SO₂R¹⁰⁰, -SO₂R¹⁰⁰, -SO₂NR¹⁰¹R¹⁰², C₁-C₆ alkyl, and C₃-C₁₀ heterocyclyl;
wherein R¹⁰⁰, R¹⁰¹ and R¹⁰² are each independently hydrogen, C₁-C₈ alkyl, or C₆-C₁₂ aryl; or R¹⁰¹ and R¹⁰² together with the nitrogen atom to which they are attached form a 5-7 membered heterocycle;
wherein the alkyl group and the aryl group are each substituted by 1-3 halogeno groups or 1-3 C₁-C₆ alkyl groups;
R¹ and R² are each independently phenyl or methyl;
X, Y and Z are each independently hydrogen or halogeno; and
R is hydrogen or C₁-C₆ alkyl or halogen-substituted alkyl; 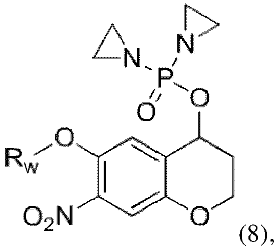
wherein the definition of R_{w} is described in the claims of Patent Application PCT/CN 2020/120281 with Publication No. WO2021068952A1; 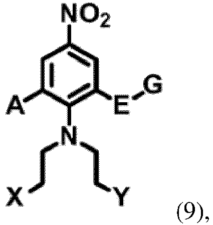
wherein the definitions of A, E, G, X and Y are described in the claims of Patent Application PCT/NZ2019/050030 with Publication No. WO2019190331A1 (corresponding to Chinese Patent Application No. 2019800234236 with Publication No. CN111918864A); 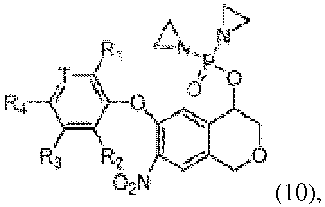
wherein the definitions of R₁, R₂, R₃, R₄, and T are described in the claims of Patent Application PCT/CN2021/118597 with Publication No. WO2022057838A1; 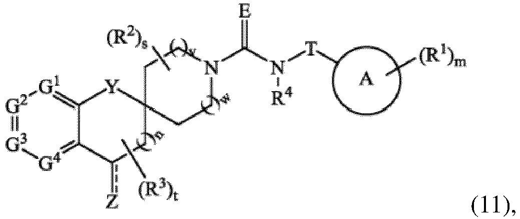
wherein the definitions of R₁, R₂, R₃, R₄, G₁, G₂, G₃, G₄, E, T, Y, Z, m, n, s, t, v, w, and ring A are described in the claims of Patent Application CN202210585771.6 with Publication No. CN115403579A; 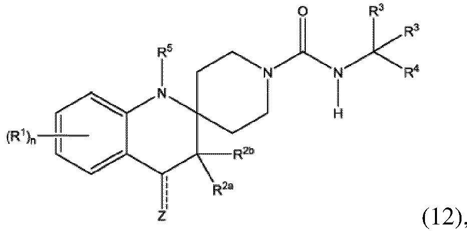 or pharmaceutically acceptable salt thereof,
wherein the definitions of R¹, R^{2a}, R^{2b}, R³, R⁴, R⁵, n and Z are described in the claims of Patent Application PCT/IB2020/057285 with Publication No. WO2021005586A1 (corresponding to Chinese Patent Application No. CN202080053804.1 with Publication No. CN114206870A).

2. The treatment method according to claim 1, wherein,
tumors or cancer tissues of the patient are detected to have mutations in either or both of the genes corresponding to BRCA1 and BRCA2; or
the patient is detected to have mutations in either or both of the genes corresponding to BRCA1 and BRCA2.

3. The treatment method according to claim 1, wherein BRCA1 and BRCA2 mutations include BRCA1 and BRCA2 mutations of germline mutations (gBRCAm) and somatic mutations (sBRCAm), preferably pathogenic mutations.

4. A method for treating gene-mutated cancer, which uses a drug monotherapy containing hypoxia-activated prodrug compounds of formulas (1), (2), (3) or AKR1C3 enzyme-activated prodrug compounds of formulas (4), (5), (6), (7), (8), (9), (10), (11), (12) and salts, esters, solvates, and isotopic isomers thereof or in combination with other drugs to treat patients with gene-mutated cancer and tumors: 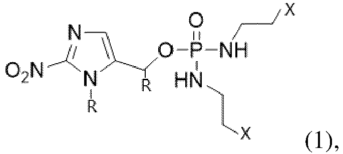
wherein the R is each independently selected from H, -CH₃, -CH₂CH₃, -CF₃, X is each independently selected from Cl, Br, MsO, TsO and other leaving functional groups; 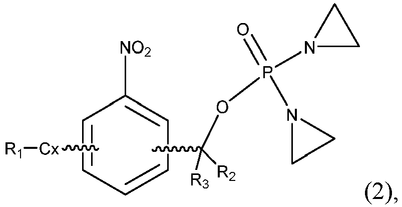
wherein the definitions of R₁, R₂, R₃ and Cx are described in the claims of Patent Application PCT/CN2020/114519 with Publication No. WO2021120717A1; 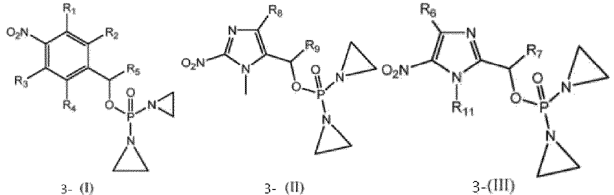 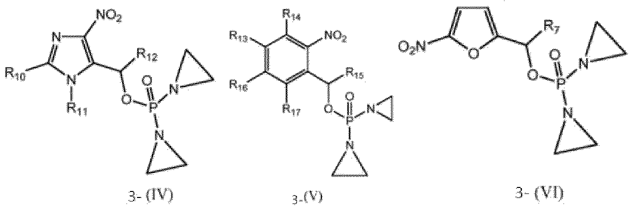
wherein the definitions of R₁, R₂, R₃, R₄, R₅, R₆, R₇, R₈, R₉, R₁₀, R₁₁, R₁₂, R₁₃, R₁₄, R₁₅, R₁₆ and R₁₇ are described in the claims of Patent Application PCT/US2016/039092 with Publication No. WO2016210175A1 (corresponding to Chinese Patent Application No. 2016800368985 with Publication No. CN108024974A); 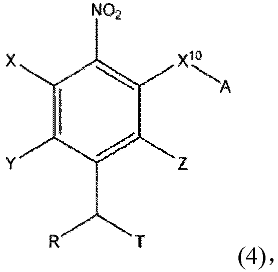
wherein the definitions of X, Y, Z, R, T, A and X¹⁰ are described in the claims of Patent Application PCT/US2016/062114 with Publication No. WO2017087428A1 (corresponding to Chinese Patent Application No. 2016800200132 with Publication No. CN108136214A); 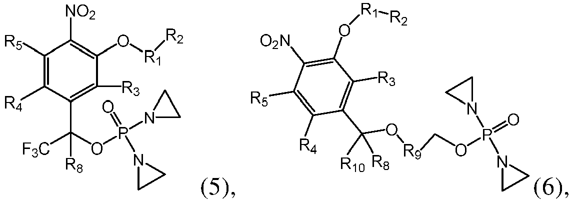
wherein the definitions of R₁, R₂, R₃, R₄, R₅, R₈, R₉ and R₁₀ are described in the claims of Patent Application PCT/CN2020/089692 with Publication No. WO2020228685A1; 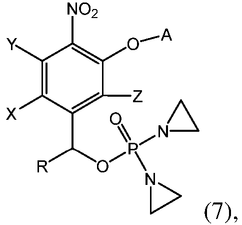
wherein:
A is a substituted or unsubstituted C₆-C₁₀ aryl, biaryl or substituted biaryl, 5-15 membered heteroaryl, or -N=CR¹R², wherein the substituents are selected from the group consisting of halogeno, -CN, -NO₂, -O-(CH₂)-O-, -CO₂H and salts thereof, -OR¹⁰⁰, -CO₂R¹⁰⁰, -CONR¹⁰¹R¹⁰², -NR¹⁰¹R¹⁰², -NR¹⁰⁰SO₂R¹⁰⁰, -SO₂R¹⁰⁰, -SO₂NR¹⁰¹R¹⁰², C₁-C₆ alkyl, and C₃-C₁₀ heterocyclyl;
wherein R¹⁰⁰, R¹⁰¹ and R¹⁰² are each independently hydrogen, C₁-C₈ alkyl, or C₆-C₁₂ aryl; or R¹⁰¹ and R¹⁰² together with the nitrogen atom to which they are attached form a 5-7 membered heterocycle;
wherein the alkyl group and the aryl group are each substituted by 1-3 halogeno groups or 1-3 C₁-C₆ alkyl groups;
R¹ and R² are each independently phenyl or methyl;
X, Y and Z are each independently hydrogen or halogeno; and
R is hydrogen or C₁-C₆ alkyl or halogen-substituted alkyl; 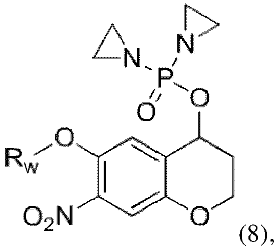
wherein the definition of R_{w} is described in the claims of Patent Application PCT/CN 2020/120281 with Publication No. WO2021068952A1; 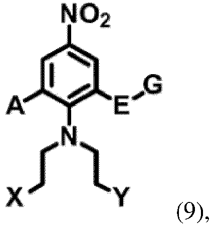
wherein the definitions of A, E, G, X and Y are described in the claims of Patent Application PCT/NZ2019/050030 with Publication No. WO2019190331A1 (corresponding to Chinese Patent Application No. 2019800234236 with Publication No. CN111918864A); 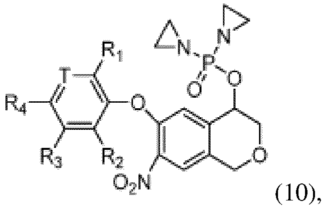
wherein the definitions of R₁, R₂, R₃, R₄, and T are described in the claims of Patent Application PCT/CN2021/118597 with Publication No. WO2022057838A1; 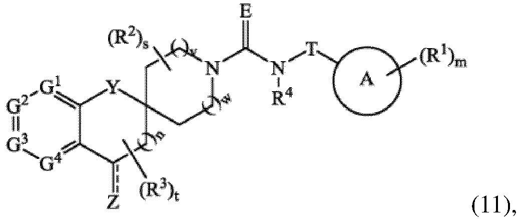
wherein the definitions of R₁, R₂, R₃, R₄, G₁, G₂, G₃, G₄, E, T, Y, Z, m, n, s, t, v, w, and ring A are described in the claims of Patent Application CN202210585771.6 with Publication No. CN115403579A; 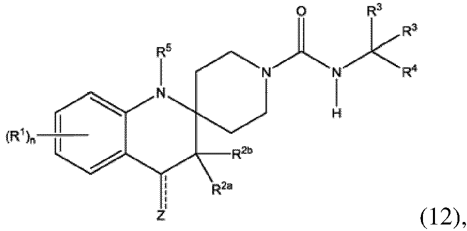
or pharmaceutically acceptable salt thereof,
wherein the definitions of R¹, R^{2a}, R^{2b}, R³, R⁴, R⁵, n and Z are described in the claims of Patent Application PCT/IB2020/057285 with Publication No. WO2021005586A1 (corresponding to Chinese Patent Application No. CN202080053804.1 with Publication No. CN114206870A);
**the gene mutation is any mutation in the homologous DNA double-strand damage repair genes.**

5. The treatment method according to claim 4, wherein the homologous DNA double-strand damage repair genes are selected from the corresponding genes of FANCA, FANCD1, FANCD2, ATM, ATR, CHEK1, CHEK2, CTP, BARD1, BRIP1, PALB2, RAD51D, RAD51C, RAD52, RAD54, RAD55, RAD57, FAM175, NBN, Rad50, MRE11, p53, NBS1, XRS2, XRCC2, XRCC3, XRCC4/XPF, ERCC1, ERCC2/XPD, ERCC3/XPB, ERCC4/XPF, XRCC1, Ku80, MHS6, MGMT, PARP, ERCC5/XPG, CCNH, CDK7, CETN2, DDB1, DDB2, ERCC5/XPG, ERCC6/CSB, ERCC8/CSA, LIG1/DNA ligase I, MMS19, MNAT1, RAD23A, RAD23B, RPA1, RPA2, TFIIH, XAB2, XPA, XPC, MBD4, NEIL1, BAP1, CDK12, EXO1, FAAP20, FAN1, FANCE, FANCM, MDC1, NONO, POLQ, RAD51B, RBBP8, SMC5, USP11, WRN and AP endonuclease, end processing enzyme, DNA polymerase, Flap endonuclease, DNA ligase.

6. The treatment method according to claim 4, wherein,
tumors or cancer tissues of the patient are detected to have any mutation in the homologous DNA double-strand damage repair genes; or
the patient is detected to have any mutation in the homologous DNA double-strand damage repair genes.

7. The treatment method according to any one of claims 1 to 6, wherein the combination of other drugs comprises immunotherapy drugs, preferably, the immunotherapy drugs are selected from immune checkpoint inhibitors, more preferably, the immunotherapy drugs are selected from PD-1 monoclonal antibodies and PD-L1 monoclonal antibodies; or
the combination of other drugs comprises PARP inhibitor, preferably, the PARP inhibitor is Olaparib.

8. The treatment method according to any one of claims 1 to 6, wherein the cancer or tumor is selected from the group consisting of pancreatic cancer, gastric cancer, ovarian cancer, breast cancer, cervical cancer, prostate cancer, liver cancer, non-small cell lung cancer, colon cancer, and rectal cancer.

9. The treatment method according to any one of claims 1 to 6, wherein the patients refer to human patients and mammalian patients other than human.

10. The treatment method according to any one of claims 1 to 6, wherein,
the hypoxia-activated prodrug compound of formula (1) is selected from the compounds of following structures: 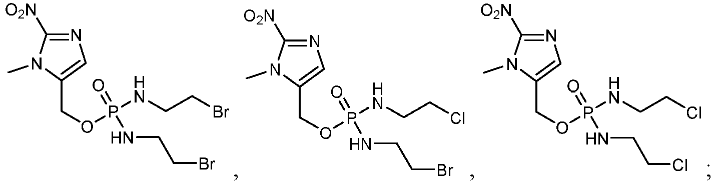
the hypoxia-activated prodrug compound of formula (2) is selected from the compounds of following structures: 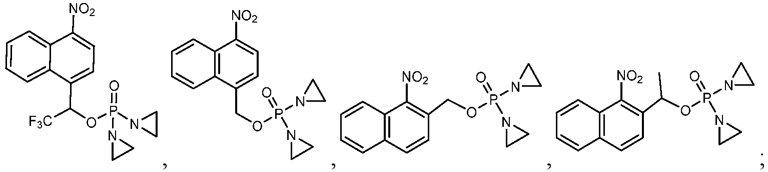
the hypoxia-activated prodrug compound of formula (3) is selected from the compounds of following structures: 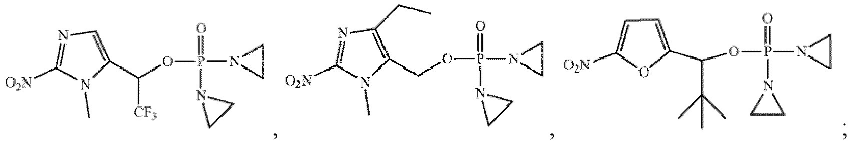
the AKR1C3 enzyme-activated prodrug compound of formula (4) is selected from the compounds of following structures: 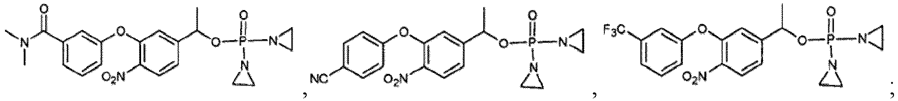
the AKR1C3 enzyme-activated prodrug compound of formula (6) is selected from the compounds of following structures: 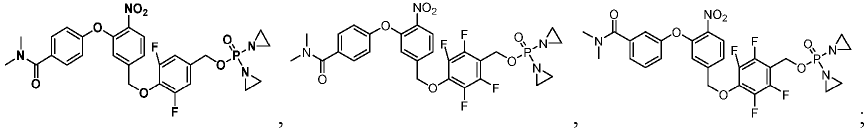
the AKR1C3 enzyme-activated prodrug compound of formula (5) is selected from the compounds of following structures: 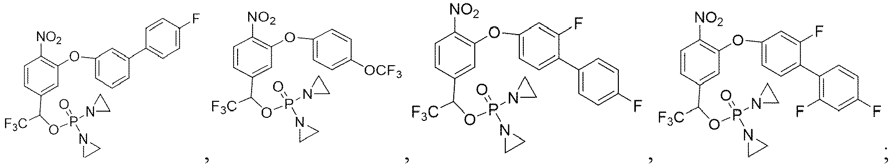
the AKR1C3 enzyme-activated prodrug compound of formula (7) is selected from the compounds of following structures: 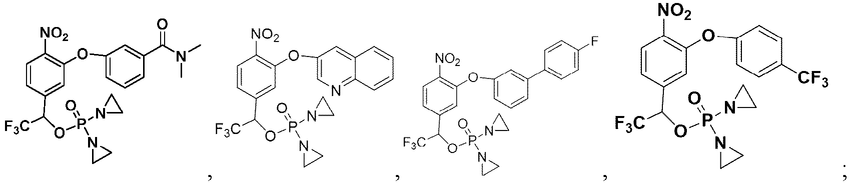
the AKR1C3 enzyme-activated prodrug compound of formula (8) is selected from the compounds of following structures: 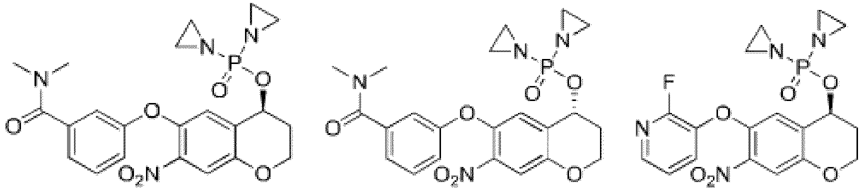
or 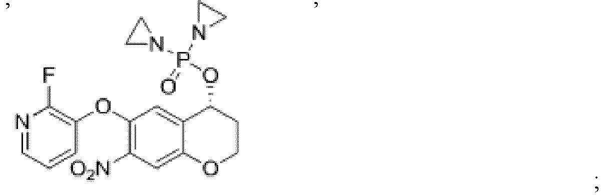
the AKR1C3 enzyme-activated prodrug compound of formula (9) is selected from the compounds of following structures: 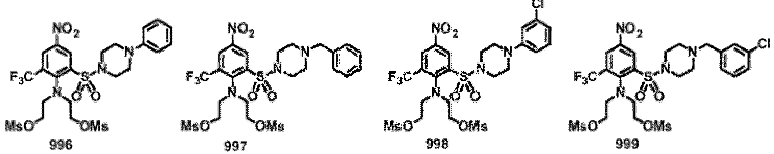 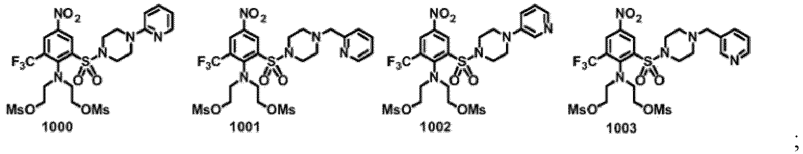
the AKR1C3 enzyme-activated prodrug compound of formula (10) is selected from the compounds of following structures: 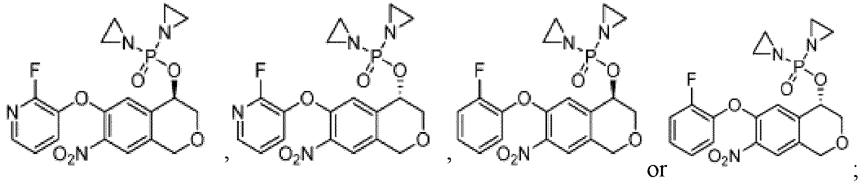
the AKR1C3 enzyme-activated prodrug compound of formula (11) is selected from the compounds of following structures: 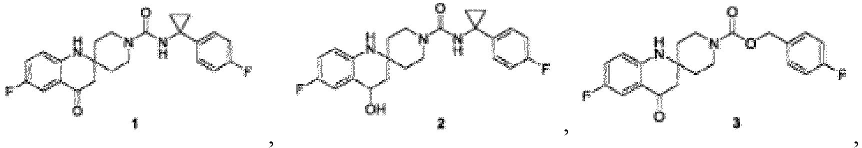 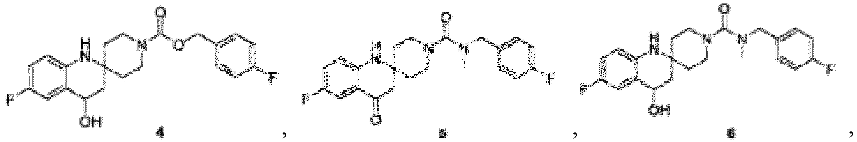
the AKR1C3 enzyme-activated prodrug compound of formula (12) is selected from the compounds of following structures:
